(19) Europäisches Patentamt
European Patent Office
Office européen des brevets

(11) **EP 2 145 607 B1**

(12) **EUROPEAN PATENT SPECIFICATION**

(45) Date of publication and mention
of the grant of the patent:
**29.05.2013 Bulletin 2013/22**

(51) Int Cl.:
***A61F 2/95*** *(2013.01)*

(21) Application number: **09175398.8**

(22) Date of filing: **15.10.2004**

(54) **Delivery system for graft**

Abgabesystem für Graft

Système de mise en place pour prothèse

(84) Designated Contracting States:
**AT BE BG CH CY CZ DE DK EE ES FI FR GB GR
HU IE IT LI LU MC NL PL PT RO SE SI SK TR**

(30) Priority: **16.10.2003 US 686863**

(43) Date of publication of application:
**20.01.2010 Bulletin 2010/03**

(62) Document number(s) of the earlier application(s) in
accordance with Art. 76 EPC:
**04795301.3 / 1 673 003**

(73) Proprietor: **TriVascular, Inc.**
**Santa Rosa,**
**California 95403 (US)**

(72) Inventors:
• **Chobotov, Michael V.**
**Santa Rosa, CA 95403 (US)**
• **Glynn, Brain A.**
**Santa Rosa, CA 95404 (US)**

(74) Representative: **Rögner, Jürgen**
**Schwabe Sandmair Marx**
**Patentanwälte**
**Stuntzstrasse 16**
**81677 München (DE)**

(56) References cited:
EP-A- 1 138 280          WO-A-01/58387
US-A1- 2002 183 827      US-A1- 2002 198 587
US-A1- 2003 135 261      US-B1- 6 290 728

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

BACKGROUND OF THE INVENTION

The present invention relates generally to a system for the treatment of disorders of the vasculature. More specifically, a system for treatment of thoracic or abdominal aortic aneurysm and the like, which is a condition manifested by expansion and weakening of the aorta. Prior methods of treating aneurysms have consisted of invasive surgical methods with graft placement within the affected vessel as a reinforcing member of the artery. However, such a procedure requires a surgical cut down to access the vessel, which in turn can result in a catastrophic rupture of the aneurysm due to the decreased external pressure from the surrounding organs and tissues, which are moved during the procedure to gain access to the vessel. Accordingly, surgical procedures can have a high mortality rate due to the possibility of the rupture discussed above in addition to other factors. Other risk factors for surgical treatment of aortic aneurysms can include poor physical condition of the patient due to blood loss, anuria, and low blood pressure associated with the aortic abdominal aneurysm. An example of a surgical procedure is described in a book entitled Surgical Treatment of Aortic Aneurysms by Cooley published in 1986 by W.B. Saunders Company.

Due to the inherent risks and complexities of surgical intervention, various attempts have been made to develop alternative methods for deployment of grafts within aortic aneurysms. One such method is the non-invasive technique of percutaneous delivery by a catheter-based system. Such a method is described in Lawrence, Jr. et al. in "Percutaneous endovascular graft: experimental evaluation", Radiology (May 1987). Lawrence described therein the use of a Gianturco stent as disclosed in U.S. Patent No. 4,580,568. The stent is used to position a Dacron fabric graft within the vessel. The Dacron graft is compressed within the catheter and then deployed within the vessel to be treated. A similar procedure has also been described by Mirich et al. in "Percutaneously placed endovascular grafts for aortic aneurysms: feasibility study," Radiology (March 1989). Mirich describes therein a self-expanding metallic structure covered by a nylon fabric, with said structure being anchored by barbs at the proximal and distal ends.

One of the primary deficiencies of the existing percutaneous devices and methods has been that the grafts and the delivery systems used to deliver the grafts are relatively large in profile, often up to 0,8 cm (24 French), and stiff in longitudinal bending. The large profile and relatively high bending stiffness of existing delivery systems makes delivery through the vessels of a patient difficult and can pose the risk of dissection or other trauma to the patient's vessels. In particular, the iliac arteries of a patient are often too narrow or irregular for the passage of existing percutaneous devices. Because of this, non-invasive percutaneous graft delivery for treatment of aortic aneurysm is contraindicated for many patients who would otherwise benefit from it.

What is needed is an endovascular graft and delivery system having a small outer diameter relative to existing systems and high flexibility to facilitate percutaneous delivery in patients who require such treatment. What is also needed is a delivery system for an endovascular graft that is simple, reliable and that can accurately and safely deploy an endovascular graft within a patient's body, lumen or vessel.

BRIEF SUMMARY OF THE INVENTION

The invention is directed generally to a delivery system for delivery of an expandable intracorporeal device, specifically, an endovascular graft. Embodiments of the invention are directed to percutaneous non-invasive delivery of endovascular grafts which eliminate the need for a surgical cut-down in order to access the afflicted artery or other intracorporeal conduit of the patient being treated. Such a non-invasive delivery system result in shorter procedure duration, expedited recovery times and lower risk of complication. The flexible low profile properties of some embodiments of the invention also make percutaneous non-invasive procedures for delivery of endovascular grafts available to patient populations that may not otherwise have such treatment available. For example, patients with small anatomies or particularly tortuous vasculature may be contraindicated for procedures that involve the use of delivery systems that do not have the flexible or low profile characteristics of embodiments of the present invention.

In one embodiment, which is not part of the present invention, the delivery system has an elongate shaft with a proximal section and a distal section. The distal section of the elongate shaft includes a portion having an expandable intracorporeal device. An elongate belt support member is disposed adjacent a portion of the expandable intracorporeal device and a belt is secured to the belt support member and circumferentially disposed about the expandable intracorporeal device. The belt member constrains at least a portion of the expandable intracorporeal device. A release member releasably secures the belt in the constraining configuration.

Another embodiment, which is not part of the present invention, is directed to a delivery system that has an elongate shaft with a proximal section and a distal section. The distal section of the elongate shaft has an elongate belt support member disposed adjacent a portion of the expandable intracorporeal device. A belt is secured to the belt support member and is circumferentially disposed about the expandable intracorporeal device. The belt has a configuration which constrains the expandable intracorporeal device and a release member releasably secures the belt in the constraining configuration. The belt may constrain any portion of the expandable intracorporeal device, such as a self-expanding portion of the expandable intracorporeal device. A self-expanding portion of the device may include a self-expanding member such as a tubular stent.

In a particular embodiment which is not part of the present invention, a plurality of belts are secured to various axial positions on the belt support member, are circumferentially disposed about the expandable intracorporeal device and have a configuration which constrains the expandable intracorporeal device. At least one release member releasably secures the belts in the constraining configuration. Each belt can be released by a single separate release member which engages each belt separately, or multiple belts can be released by a single release member. The order in which the belts are released can be determined by the axial position of the belts and the direction of movement of the release member.

Another embodiment which is not part of the present invention, is directed to a delivery system for delivery of a self-expanding endovascular graft with a flexible tubular body portion and at least one self-expanding member secured to an end of the endovascular graft. The delivery system has an elongate shaft having a proximal section and a distal section. The distal section of the elongate shaft has an elongate belt support member disposed within the self-expanding member of the endovascular graft and a belt that is secured to the belt support member adjacent the self-expanding member. The belt is also circumferentially disposed about the self-expanding member and has a configuration that constrains the self-expanding member. A release wire releasably secures ends of the belt in the constraining configuration.

A further embodiment which is not part of the invention, includes a delivery system for delivery of an endovascular graft with a flexible tubular body portion and a plurality of self-expanding members secured to ends of the endovascular graft. The delivery system has an elongate shaft with a proximal section and a distal section. The distal section of the elongate shaft has an elongate guidewire tube disposed within the endovascular graft in a constrained state. A plurality of shape memory thin wire belts are secured to the guidewire tube respectively adjacent the self-expanding members. The belts are circumferentially disposed about the respective self-expanding members and have a configuration that constrains the respective self-expanding members. A first release wire releasably secures ends of the belts disposed about the self-expanding members at the proximal end of the endovascular graft in a constraining configuration. A second release wire releasably secures ends of the belts disposed about the self-expanding members at a distal end of the endovascular graft in the constraining configuration.

It is also disclosed a method for deploying an expandable intracorporeal device within a patient's body. The method includes providing a delivery system for delivery of an expandable intracorporeal device including an elongate shaft having a proximal section and a distal section. The distal section of the elongate shaft has an elongate belt support member disposed adjacent a portion of the expandable intracorporeal device and a belt which is secured to the belt support member. The belt is circumferentially disposed about the expandable intracorporeal device and has a configuration that constrains the expandable intracorporeal device. A release member releasably secures the belt in the constraining configuration.

Next, the distal end of the delivery system is introduced into the patient's body and advanced to a desired site within the patient's body. The release member is then activated, releasing the belt from the constraining configuration. Optionally, the delivery system may also have an outer protective sheath disposed about the endovascular graft in a constrained state, the belt in its constraining configuration and at least a portion of the release wire disposed at the belt. The method of deployment of an expandable intracorporeal device also includes retraction of the outer protective sheath from the endovascular graft prior to activation of the release member.

It is also disclosed a method for delivery of bifurcated intracorporeal device, an elongate shaft has a proximal section and a distal section. The distal section of the shaft has an elongate primary belt support member and at least one primary belt disposed on the primary belt support member. The primary belt support member is configured to be circumferentially disposed about a bifurcated intracorporeal device and at least partially constrain the device. A primary release member is configured to engage and releasably secure the primary belt in a constraining configuration. At least one elongate secondary belt support member is disposed adjacent the elongate primary belt support member. At least one secondary belt is disposed on the secondary belt support member. This at least one secondary belt is configured to be circumferentially disposed about a bifurcated intracorporeal device and at least partially constrain the device. A secondary release member is configured to engage and releasably secure the secondary belt in a constraining configuration.

In a method, which is not part of the invention, for deploying a bifurcated intracorporeal device within a patient's body, a delivery system for delivery and deployment of a bifurcated intracorporeal device is provided. The delivery system includes an elongate shaft having a proximal section and a distal section. The bifurcated intracorporeal device is disposed on the distal section of the elongate shaft. The distal section of the elongate shaft also includes an elongate primary belt support member and at least one primary belt secured to the primary belt support member. The primary belt is configured to be circumferentially disposed about a bifurcated intracorporeal device and at least partially constrain the device. A primary release member engages and releasably secures the primary belt in the constraining configuration. The distal section of the elongate shaft also includes at least one elongate secondary belt support member disposed adjacent the elongate primary belt support member. At least one secondary belt is secured to the secondary belt support member and is configured to be circumferentially disposed about a bifurcated intracorporeal device to at least partially constrain the device. A secondary release member engages and releasably secures the secondary belt in a constraining configuration.

The distal end of the delivery system is introduced into the patient's body and advanced to a desired site within the patient's body. The release members are then activated to release the belts from the constraining configuration and the device is deployed. Thereafter, the delivery system can be removed from the patient's body. The secondary belt support member may be detached and removed from the delivery system prior to withdrawal of the delivery system from the patient. The secondary belt support member may be displaced laterally towards the primary belt support member so as to be substantially parallel to the primary belt support member and enable withdrawal of the delivery system through an ipsilateral side of the bifurcated intracorporeal device.

BRIEF DESCRIPTION OF THE DRAWINGS

FIG. 1 is an elevational view in partial longitudinal section illustrating an embodiment, which is not part of the invention, of a delivery system for an expandable intracorporeal device.

FIG. 2 is a transverse cross sectional view of the delivery system of FIG. 1 taken along lines 2-2 of FIG. 1.

FIG. 3 is a transverse cross sectional view of the delivery system of FIG. 1 taken along lines 3-3 of FIG. 1.

FIG. 4 is a transverse cross sectional view of the delivery system of FIG. 1 taken along lines 4-4 of FIG. 1.

FIG. 5 is a transverse cross sectional view of the delivery system of FIG. 1 taken along lines 5-5 of FIG. 1.

FIG. 6A is an enlarged elevational view in partial section of the delivery system in FIG. 1.

FIG. 6B is an enlarged elevational view in partial section of the delivery system of FIG. 1 with portions of the graft and self-expanding members cut away for clarity of view of the belt bushings.

FIG. 7A is a perspective view showing release belt configurations..

FIG. 7B is a perspective view showing an alternative embodiment, which is not part of the invention, of release belts.

FIG. 7C is an end view showing an alternative embodiment, which is not part of the invention, of release belts.

FIG. 7D is a perspective view of the embodiment of FIG. 7C, which is not part of the invention.

FIG. 7E is an enlarged view of a particular coupling configuration between end loops of release belts.

FIG. 7F is a perspective view, partially cut away, of a particular embodiment, which is not part of the invention, of an end loop of a release belt.

FIG. 7G is a perspective view of an alternative embodiment, which is not part of the invention, of a release belt.

FIG. 7H is a perspective view of an alternative embodiment, which is not part of the invention, of a release belt.

FIG. 7I is a perspective view of an alternative embodiment, which is not part of the invention, of a branched release wire.

FIG. 7J is an end view showing an alternative embodiment, which is not part of the invention, of a release belt.

FIG. 7K is a transverse cross sectional view showing the alternative embodiment, which is not part of the invention, of the release belt configuration of FIG. 7J constraining a self-expanding member.

FIG. 7L is a detail of the connection formed where a release wire is used with the alternative release belt embodiment, which is not part of the invention, of FIGS. 7J-7K.

FIGS. 7M-7N are schematic views of a portion of a self-expanding member belted by two belts and secured by a release wire in various locations relative to the self-expanding member crowns or apices.

FIG. 8 is an elevational view in partial section of the proximal adapter shown in FIG. 1.

FIG. 9 is a diagrammatic view of a patient's body illustrating the patient's heart, aorta, iliac arteries, femoral arteries, and a delivery system disposed within the femoral artery and aorta.

FIG. 10 is a diagrammatic view of a delivery system disposed within an artery of a patient with an expandable intracorporeal device being deployed within the artery.

FIG. 11 is a diagrammatic view of a delivery system disposed within an artery of a patient with an expandable intracorporeal device being deployed within the artery.

FIG. 12 is an enlarged diagrammatic view of a delivery system disposed within an artery of a patient with an expandable intracorporeal device being deployed within the artery.

FIG. 13 is an elevational view in partial section of a connection between an inflation tube and an inflation port of an endovascular graft.

FIG. 14 is an elevational view in partial longitudinal section of a delivery system for an expandable intracorporeal device having features of the invention.

FIG. 15 is a transverse cross sectional view of the delivery system of FIG. 14 taken along lines 15-15 in FIG. 14.

FIG. 16 is an enlarged elevational view in partial section of the delivery system shown in FIG. 14.

FIG. 17 is an elevational view in partial section of the proximal adapter of the delivery system shown in FIG. 14.

FIG. 18 is an elevational view in partial section of an alternative embodiment of the proximal adapter of the delivery system shown in FIG. 14 with a nested handle configuration.

FIG. 19 is an elevational view of a bifurcated stent graft suitable for delivery and deployment by embodiments of the invention.

FIG. 20 is a transverse cross sectional view of the stent graft of FIG. 19 taken along lines 20-20 in FIG. 19.

FIG. 21 is a transverse cross sectional view of the stent graft of FIG. 19 taken along lines 21-21 of FIG. 19.

FIG. 22 is a transverse cross sectional view of the stent graft of FIG. 19 taken along lines 22-22 of FIG. 19.

FIG. 23 is an elevational view in partial section of an embodiment, which is not part of the invention, of a delivery system.

FIG. 24 is a transverse cross sectional view of the delivery system of FIG. 23 taken along lines 24-24 of FIG. 23.

FIG. 25 is a transverse cross sectional view of the delivery system of FIG. 23 taken along lines 25-25 of FIG. 23.

FIG. 26 is an elevational view in partial section showing an enlarged view of a distal portion of the delivery system of FIG. 23.

FIG. 27 is a transverse cross sectional view of the delivery system of FIG. 26 taken along lines 27-27 of FIG. 26.

FIG. 28 is a transverse cross sectional view of the delivery system of FIG. 26 taken along lines 28-28 of FIG. 26.

FIG. 28A is a transverse cross sectional view of an alternative embodiment, which is not part of the invention, of a secondary belt support member of a delivery system similar in function to that shown in FIG. 28.

FIG. 28B is an elevational view of the alternative embodiment, which is not part of the invention, of the secondary belt support member of FIG. 28A.

FIG. 29 is a transverse cross sectional view of the delivery system of FIG. 26 taken along lines 29-29 of FIG. 26.

FIG. 30 is a transverse cross sectional view of the delivery system of FIG. 26 taken along lines 30-30 in FIG. 26.

FIG. 31 is an elevational view in partial section of the proximal adapter of the delivery system of FIG. 23.

FIG. 31A is an elevational view in partial section of the proximal adapter of the delivery system of FIG. 23, showing an optional ripcord and flexible fill catheter.

FIG. 31B is a simpler cross sectional schematic view of a bent or angled contralateral leg inflatable channel having a bead or lumen patency member disposed in a channel lumen taken along line 31B-31B in FIG. 19.

FIG. 32 is a perspective view of the belt support member assembly at a distal portion of the delivery system of FIG. 23.

FIG. 33 illustrates a portion of the internal vasculature of a patient, including the aorta, iliac and femoral arteries branching therefrom.

FIG. 34 is a magnified view of the abdominal aorta area of the patient shown in FIG. 33 and shows a guidewire positioned in the aorta from the right iliac artery.

FIGS. 35-37 illustrate the magnified view of the abdominal aorta of the patient shown in FIG. 33 and depict a deployment sequence of the bifurcated endovascular stent graft of FIG. 19 with the delivery system of FIG. 23.

FIG. 37A is a perspective view of a marker disposed on the delivery system distal section in the vicinity of the nosepiece.

FIG. 37B is a perspective view of an alternative embodiment of a marker for use in the delivery system of the present invention.

FIGS. 38-52 continue to illustrate a deployment sequence of the bifurcated endovascular stent graft of FIG. 19.

FIGS. 53-57 illustrate a number of alternative catheter distal shaft arrangements in which a well is provided to facilitate the orderly and tangle-free withdrawal of the release strand from the delivery catheter.

FIGS. 58-60 illustrate a further alternative belt support member and contralateral leg delivery system configurations and operation.

FIGS. 61-63B illustrate optional ipsilateral leg sleeve embodiments for protecting the bifurcated graft ipsilateral leg from damage by other graft and delivery system components.

DETAILED DESCRIPTION OF THE INVENTION

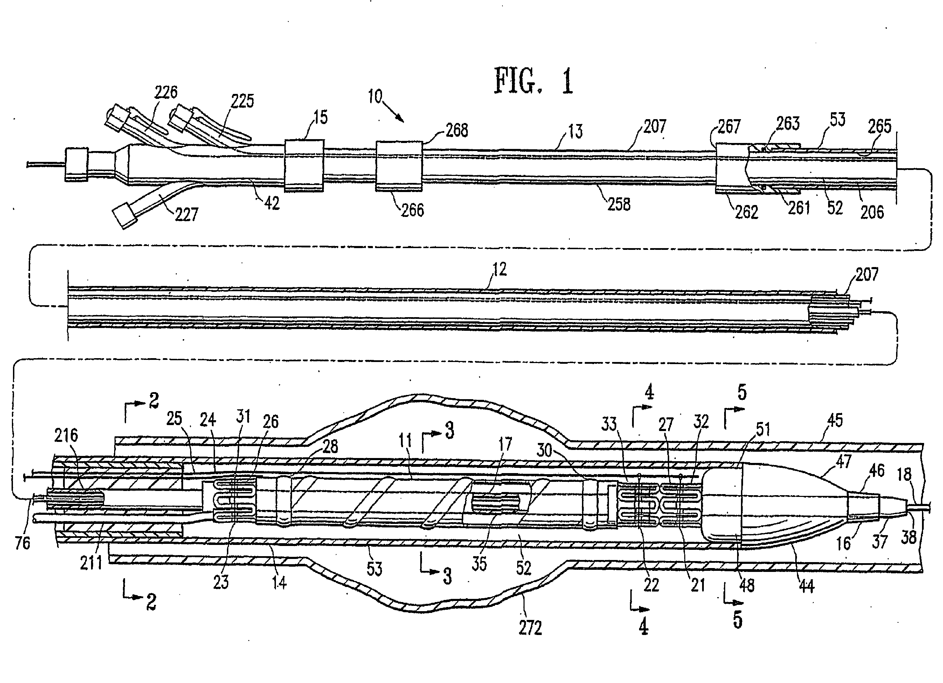

FIGS. 1-8 and 10 illustrate an embodiment which is not part of the invention, of delivery system 10 for delivering a variety of expandable intracorporeal devices; specifically, an expandable endovascular graft 11. One such expandable endovascular graft 11 useful for delivery and deployment at a desired site within a patient is disclosed in co-pending U.S. Patent Application Ser. No. 09/133,978, filed August 14, 1998, by M. Chobotov.

Delivery system 10 in FIG. 1 has an elongate shaft 12 with a proximal section 13, a distal section 14, a proximal end 15 and a distal end 16. The distal section 14 has an elongate belt support member in the form of a guidewire tube 17 disposed adjacent a portion of the expandable endovascular graft 11. A guidewire 18 is disposed within guidewire tube 17. A plurality of belts 21, 22, and 23 are secured to the guidewire tube 17 and are circumferentially disposed about portions of the endovascular graft 11. FIG. 1 shows the belts in a configuration that constrains the endovascular graft 11. First and second release members 24 and 25 releasably secure belts 21, 22, and 23 in a constraining configuration as shown.

The endovascular graft 11 has a proximal end 26, a distal end 27, a proximal inflatable cuff 28, a distal inflatable cuff 30, a proximal self-expanding member 31, a first distal self-expanding member 32 and a second distal self-expanding member 33. As defined herein, the proximal end of the elongate shaft is the end 15 proximal to an operator of the delivery system 10 during use. The distal end of the elongate shaft is the end 16 that enters and extends into the patient's body. The proximal and distal directions for the delivery system 10 and endovascular graft 11 loaded within the delivery system 10 as used herein are the same. This convention is used throughout the specification for the purposes of clarity, although other conventions are commonly used. For example, another useful convention defines the proximal end of an endovascular graft as that end of the graft that is proximal to the source of blood flow going into the graft. Such a convention is used in the previously discussed co-pending patent application, Ser. No. 09/133,978, although that convention is not adopted herein.

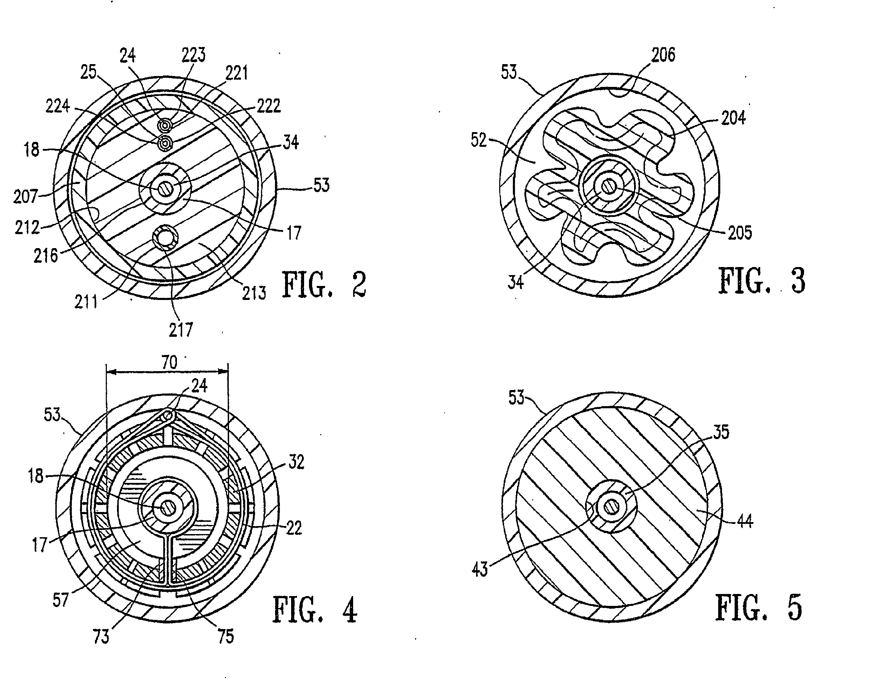

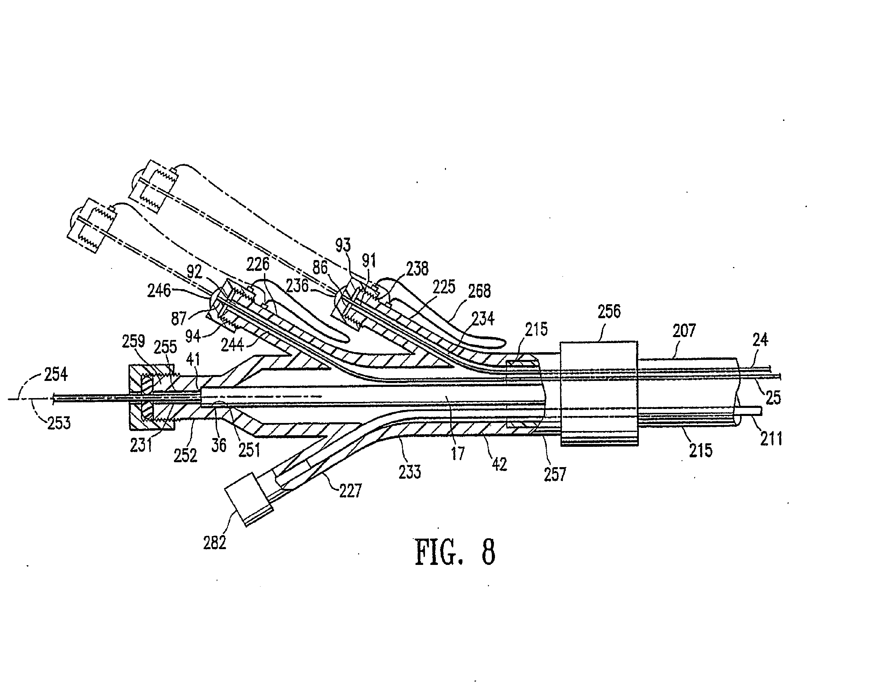

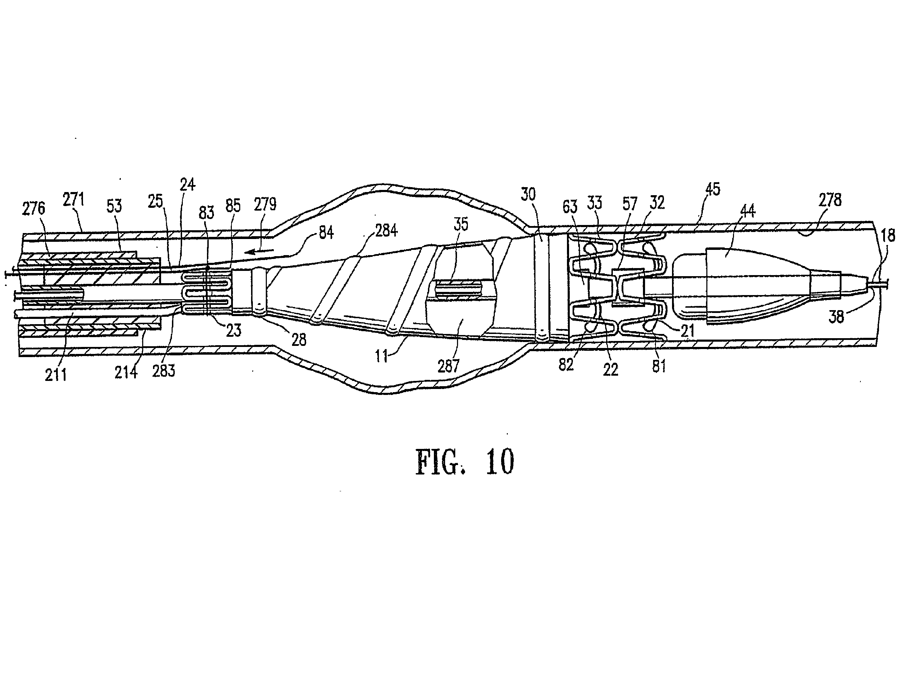

The guidewire tube 17 has an inner lumen 34, as shown in FIG. 2, a distal section 35, a proximal end 36, as shown in FIG. 8, and a distal end 37. The inner lumen 34 of the guidewire tube 17 terminates at the distal end 37 with a distal guidewire tube port 38, as shown in FIG. 10. As seen in FIG. 8, the proximal end 36 of guidewire tube 17 terminates in a port 41 disposed in the proximal adapter 42. The port 41 is typically a tapered fitting such as a Luer lock fitting which facilitates the attachment of a hemostasis valve (not shown). The guidewire tube 17 is a hollow tubular member that normally has an annular cross section, although oval cross-sectional profiles and others are also suitable.

A portion of the distal section 35 of the guidewire tube 17, shown in FIG. 1, is disposed within an inner lumen 43 of a distal nose piece 44, as shown in FIG. 5. Distal nose piece 44 is configured in a streamlined bullet shape for easy passage within a patient lumen or vessel such as aorta 45. Guidewire tube 17 may be bonded to the inner lumen 43 of the nose piece 44, or it may be molded into the nose piece 44 during manufacture. Referring to FIG. 1, the nose piece 44 has a distal portion 46, an intermediate portion 47 and a proximal shoulder portion 48 configured to slidingly engage the distal portion 51 of an inner lumen 52 of an outer tubular member 53.

Referring to FIGS. 1, 6A, 6B and 7A, on the distal section 35 of guidewire tube 17, proximal to the proximal shoulder portion 48 of nose piece 44, a first distal belt 21 is secured to the guidewire tube 17. The first distal belt may be secured to the guidewire tube 17 with any suitable adhesive such as cyanoacrylate, epoxy or the like. Both free ends 55 and 56 of the first distal belt 21 are secured to the guidewire tube 17. The guidewire tube 17 may be made from a variety of suitable materials including polyethylene, teflon, polyimide and the like.

Referring to FIGS. 2-5, the inner lumen 34 of the guidewire tube 17 has an inside diameter that can accommodate a guidewire suitable for guiding a device such as delivery system 10. The inner lumen 34 of the guidewire tube 17 may have an inside diameter of about 0,04 cm to about 0,11 cm (0.015 inch to about 0.045 inch); specifically, about 0,05 cm to about 0,1 cm (0.020 inch to about 0.040 inch). The outer diameter of the guidewire tube 17 may range from about 0,05 cm to 0,15 cm (0.020 inch to about 0.060 inch); specifically, about 0,06 cm to about 0,11 cm (0.025 inch to about 0.045 inch).

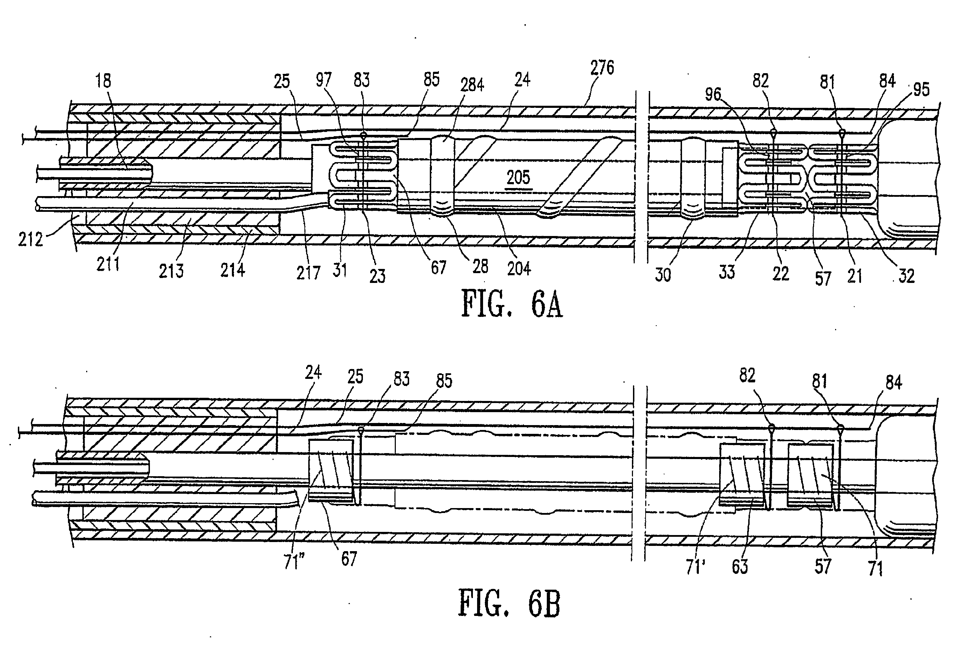

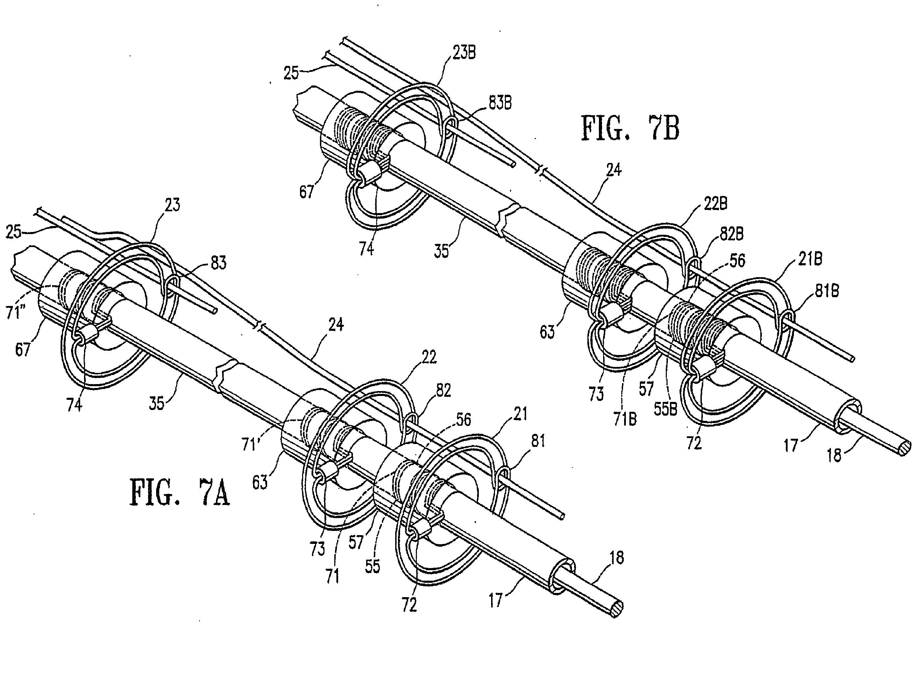

Referring again to FIGS. 6A, 6B and 7A, an optional first distal belt bushing 57 is disposed about the guidewire tube 17 so as to cover the portions of the free ends 55 and 56 of the first distal belt 21 that are secured to the distal section 35 of the guidewire tube 17. This bushing 57 may also serve to control the constrained configuration of the belted self-expanding members, and may include geometric features to engage or support the belted members. A similar configuration is present at a second distal belt 22 which has free ends secured to the guidewire tube 17 proximal to the first distal belt 21. A second distal belt bushing 63 is disposed about the guidewire tube 17 so as to cover the portions of the free ends of the second distal belt 22 that are secured to the guidewire tube 17. A proximal belt 23 has free ends secured to the guidewire tube 17 proximal to the second distal belt 22 and has an optional proximal belt bushing 67, as shown in FIG. 6, configured similarly to the first and second distal belt bushings 57 and 63.

The belts 21, 22 and 23 can be made from any high strength, resilient material that can accommodate the tensile requirements of the belt members and remain flexible after being set in a constraining configuration. Typically, belts 21, 22 and 23 are made from solid ribbon or wire of a shape memory alloy such as nickel titanium or the like, although other metallic or polymeric materials are possible. Belts 21, 22 and 23 may also be made of braided metal filaments or braided or solid filaments of high strength synthetic fibers such as Dacron®, Spectra or the like. An outside transverse cross section of the belts 21, 22 and 23 may range from about 0,005 cm to about 0,03 cm (0.002 to about 0.012 inch), specifically, about 0,01 cm to about 0,02 cm (0.004 to about 0.007 inch). The cross sections of belts 21, 22 and 23 may generally take on any shape, including rectangular (in the case of a ribbon), circular, elliptical, square, etc.

In general, we have found that a ratio of a cross sectional area of the belts to a cross sectional area of the release members, 24 and 25, of about 1:2 is useful to balance the relative strength and stiffness requirements. Other ratios, however, may also be used depending on the desired performance characteristics.

The inner diameters of belt bushings 57, 63 and 67 are sized to have a close fit over the guidewire tube 17 and secured portion 71, as shown in FIG. 7A, of the free ends of the belts 21, 22 and 23 that are secured to the guidewire tube 17. Typically, the inner diameter of the belt bushings 57, 63 and 67 range from about 0,06 cm to about 0,16 cm (0.025 inch to about 0.065 inch); specifically, about 0,07 cm to about 0,13 cm (0.030 inch to about 0.050 inch). In addition, the outer diameter of belt bushing 57 may be sized to approximate an inner diameter 70, as shown in FIG. 4, of the respective first distal self-expanding member 32 of the endovascular graft 11 when the member 32 is in a fully constrained state. The other belt bushings 63 and 67 may be similarly configured with respect to the second distal self-expanding member 33 and the proximal self-expanding member 31.

Such an arrangement keeps the self-expanding members 31, 32 and 33 properly situated when in a constrained state and prevents the various portions of the self-expanding members 31, 32 and 33 from overlapping or otherwise entangling portions thereof while in a constrained state. The outer diameter of the belt bushings 57, 63 and 67 may range from about 0,1 cm to about 0,5 cm (0.040 inch to about 0.200 inch); specifically, about 0,15 cm to about 0,23 cm (0.060 inch to about 0.090 inch). The material of the belt bushings 57, 63 and 67 may be any suitable polymer, metal, alloy or the like that is bondable. Generally, the belt bushings 57, 63 and 67 are made from a polymer such as polyurethane, silicone rubber or PVC plastic.

As shown in FIG. 7A, belts 21, 22 and 23 extend radially from the guidewire tube 17 through optional standoff tubes 72, 73 and 74. Standoff tubes 72, 73 and 74 are disposed about belts 21-23 adjacent the guidewire tube 17 and act to prevent separation of belts 21-23 in a circumferential direction as tension is applied to the belts. Standoff tubes 72 - 74 also prevent belts 21-23 from applying other undesirable forces on portions of the endovascular graft 11 that are constrained by the belts. Specifically, the standoff tubes 72 -74 prevent the belts 21-23 from spreading the self-expanding members 31 - 33, or portions thereof, at those locations where the belts 21-23 extend radially through the self-expanding members.

The standoff tubes 72 - 74 typically have a length substantially equal to a single wall thickness of the self-expanding members 31, 32 and 33. The length of the standoff tubes 72 - 74 may range from about 0,03 cm to about 0,08 cm (0.010 inch to about 0.030 inch). An inner diameter of an inner lumen 75 of the standoff tubes, as shown in FIG. 4, may range from about 0,1 cm to about 0,06 cm (0.004 to about 0.024 inch), with a wall thickness of the standoff tubes being about 0,005 cm to about 0,015 cm (0.002 inch to about 0.006 inch). Typically, the standoff tubes 72 - 74 are made from a high strength metal or alloy such as stainless steel, although they may be polymeric as well.

Belts 21-23 exit the outer apertures of standoff tubes 72 - 74 and extend circumferentially about the respective portions of the expandable intracorporeal device 11. The term "circumferential extension" as used with regard to extension of the belts 21-23 is meant to encompass any extension of a belt in a circumferential direction. The belts may extend circumferentially a full 360 degrees, or any portion thereof. For example, belts or belt segments may extend partially about an endovascular device, and may be combined with other belts or belt segments that also partially extend circumferentially about an endovascular device. Typically, a plane formed by each of the belts 21-23 when in a constraining configuration is generally perpendicular to a longitudinal axis 76, shown in FIG. 1, of the distal section 14 of shaft 12. As shown in FIGS. 6A and 6B, loop ends 81, 82 and 83 of the belts 21, 22 and 23, respectively, are releasably locked together by one or more release members. For example, in the embodiment, which is not part of the invention, shown in FIG. 1, a release member in the form of a first release wire 24 is shown disposed within end loops 81 of the first distal belt 21 and end loops 82 of the second distal belt 22 so as to secure the first and second distal belts 21 and 22 in a constraining configuration about the endovascular graft 11. Another release member in the form of a second release wire 25 is shown disposed within end loops 83 of the proximal belt 23 so as to secure the proximal belt 23 in a constraining configuration about the endovascular graft 11.

A single release wire may also be used to perform the function of each of the first and second release wires, 24 and 25, so that first distal belt 21, second distal belt 22, and proximal belt 23 may be releasably secured by a single release wire. A highly controlled, sequential belt deployment scheme may be realized with the use of a single release wire.

It is disclosed that any number of release wires and belts may be needed to effectively secure and deploy graft 11 in combination .

In some embodiments of the invention, which is not part of the invention, when constrained, the end loops of any single belt touch each other or are spaced closely together such that the belt as a whole forms a substantially circular constraint lying substantially in a plane. Release wire 24 and 25 may be made from suitable high strength materials such as a metal or alloy (e.g., stainless steel) which can accommodate the torque force applied to the release wire by the belt end loops 83 when the belts 23 are under tension from the outward radial force of the constrained portions of the endovascular graft 11, i.e., the self-expanding members 32 and 33. Release wire 24 and 25 may alternatively comprise a composite structure in which an outer portion of release wire 24 and 25 has a lower coefficient of friction than that of the bare wire material to facilitate ease of wire retraction through retention belt end loops during deployment. For instance, a nitinol or other release wire may be coated with, coaxially joined to or coextruded with a metallic or polymeric tubing or coating, or the wire may be sputter coated or impregnated with graphite or other material to render its surface more lubricious. Polyamide tubing is a useful material for this purpose and may be affixed to the nitinol wire by an adhesive such as cyanoacrylate. The polyamide or other polymeric coating or tubing may be refined by doping its outer surface with lubricious materials such as PTFE, etc. to further ease release wire retraction.

The release wires 24 and 25 may generally have an outer diameter ranging from about 0,015 cm to about 0,036 cm (0.006 to about 0.014 inch). Distal end portions 84 and 85 of release wires 24 and 25, respectively, may terminate at any appropriate site distal of the end loops 81-83 of belts 21-23. As shown in FIG. 8, the proximal ends 86 and 87 of the release wires 24 and 25 extend through the elongate shaft 12 of the delivery system 10 through proximal ports 91 and 92 on the proximal adapter 42, respectively, and terminate at respective release wire handles 93 and 94 which are releasably secured to the proximal adapter 42.

FIG. 7B illustrates an alternative embodiment, which is not part of the invention, of the belts 21-23 of FIG. 7A. In FIG. 7A, belts 21-23 are shown as each consisting of a single strand of wire formed into the end loops 81-83, respectively, with the end loops in an overlapping configuration. Free ends 55 and 56 of belt 81 are shown secured to the distal section 35 of the guidewire tube 17. In contrast, FIG. 7B, wherein like elements with regard to FIG. 7A are shown with like reference numerals, shows belts 21B, 22B and 23B formed of two strands of wire, with each strand formed into a single loop which overlaps a loop of the other strand to form end loops 81B, 82B and 83B. The free ends of the belts 21B-23B may be secured in a similar manner to those of free ends 55 and 56 of FIG. 7A.

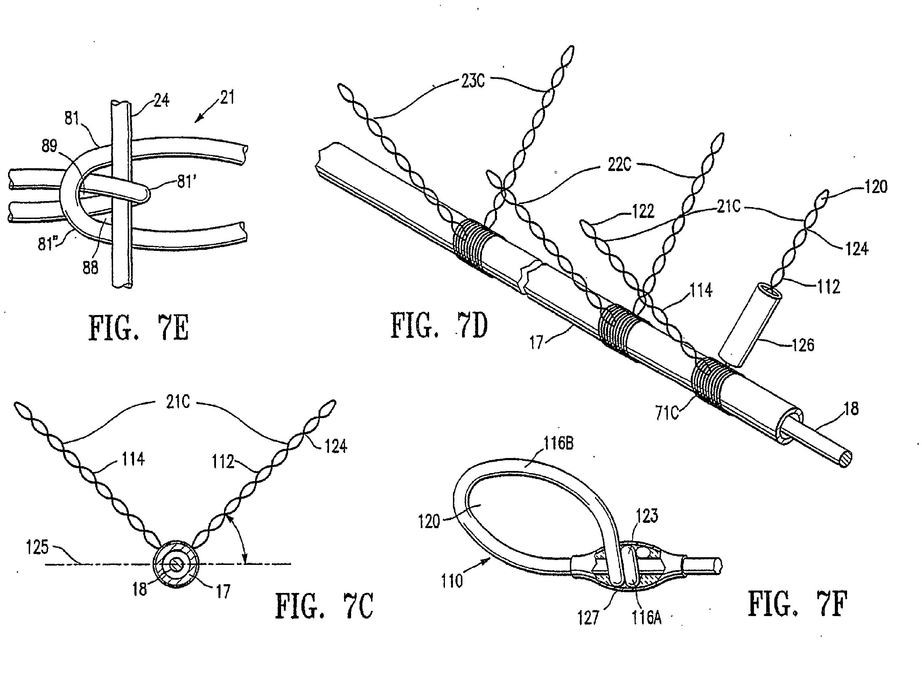

Turning now to FIGS. 7C and 7D, alternative embodiments, which are not part of the invention, for portions of the delivery system of the present invention are shown. FIGS. 7C and 7D illustrate alternative belts 21C, 22C and 23C disposed on guidewire tube 17. Single or multiple belts 21C - 23C may be deployed at various locations along guidewire tube 17 as desired. In addition, the members comprising belts 21C-23C are shown as a single line. However, belts 21C-23C may be of a single- or multiple strand or filament design with various cross-sectional shapes as previously described. A single solid ribbon or wire is particularly useful.

Belts 21C-23C shown in FIGS. 7C and 7D are a single strand filament wrapped around guidewire tube 17 and fixed thereon via any number of suitable techniques, such as gluing with adhesive, mechanical fixation, etc. Especially useful is fixing the belt with an ultraviolet-curable adhesive.

Alternatively, belts 21C-23C may comprise two strand filaments each wrapped around guidewire tube 17 so that, for instance, belt 21C is a two-filament component.

Belt 21C includes belt arms 112 and 114, each of which, in the embodiments, which are not part of the invention, shown, is a loop of filament twisted upon itself to form a helix. Any number of twists may be imparted to arms 112 and 114 to provide a relatively loose or relatively tight helix as desired. Typically the number of twists (with a single twist being defined as a single overlap of wire segment) in each belt arm 112 and 114 numbers from zero to about 50 or more; specifically, about two to about 10. The choice of material used for belt 21C is an important factor in determining the optimum number of twists for each belt arm. Belt arms 112 and 114 may be formed into other configurations (e.g., braid, double helix, etc.) as well.

Disposed within the end loops of the belt arms 112 and 114 are distal apertures or openings 120, 122, respectively. During assembly of the delivery system, a release wire (such as wire 24) is passed through each aperture 120, 122 after the belt arms are wrapped around the graft self-expanding member, preferably in a circumferential groove as further described below. The release wire may also be disposed through any aperture created along the length of belt arms 112, 114 by each helix twist, although the distal-most apertures 120, 122 are preferred.

The wire optionally may be welded, glued, or otherwise fixed to itself at discrete points or along all or any portion of belt arms 112, 114, save their corresponding apertures 120 and 122. For instance, the belt arm wire may be glued or welded to itself at the overlap or twist points, such as points 124.

FIG. 7D shows an optional belt arm sleeve 126 that may be used to enclose a portion of one or both belt arms 112, 114, or any of the other belt embodiments, which are not part of the invention, contemplated herein. Belt 112 is shown in FIG. 7D being constrained or covered over a length thereof by a flexible sleeve or coating 126 (or alternatively, a coil wrapping or by fixing the loop to itself by adhesives, welding, soldering, brazing, etc.). Sleeve or coating 126 may optionally be shrink-wrapped, crimped, or otherwise configured to constrain or cover belt arm 112 therein. These fixation and sleeve features help to minimize the potential of belt arm untwisting and tend to close or block some or all of the helix apertures along the length except those through which the release wire are intended to pass. They can also provide greater structural and operational stability to the catheter system as a whole.

Belt arm sleeve 126 can be configured to have a transverse dimension that is sized to fit a twisted belt arm with fixed nodal points such as the belt arm 112 shown in FIG. 7D. In order to accommodate such a twisted belt arm 112, the inner diameter and outer diameter would be large relative to a transverse dimension of the wire material that forms the belt ann 112. However, the belt arm sleeve 126 can also be only slightly larger in transverse dimension that the wire that forms the belt arm. For example, embodiments, which are not part of the invention, of belt arms that do not have twisted wires may have a sleeve 126 that fits closely or tightly over two strands of wire forming a belt arm. The sleeve 126 can cover substantially the entire length of such an untwisted belt arm from at least the guidewire tube to just proximal of the distal loop, such as distal loop 120. The distal loop should remain exposed for engagement by a release wire. In such an embodiment, the sleeve covered portion of the belt arm may also be wrapped around and secured to the guidewire tube just as the unsleeved belt portion of the belt arm 112 shown in FIG. 7D is shown at 71C. This type of low profile belt arm sleeve may also be used to cover twisted belt arm embodiments, which is not part of the invention, although a slightly larger diameter sleeve would be required.

It may be desirable to impart a particular free resting angle to the belt arms 112, 114 to improve the reliability of the system and further reduce the possibility of the arms 112 and 114 interfering with other components of the prosthesis or delivery system. The FIG. 7C view shows belt arms 112, 114 symmetrically disposed at an angle α as measured from a horizontal plane 125. This angle α may range from zero to 180 degrees. For example, one or both belt arm 112, 114 may lie along plane 125 or they may rest in the configuration shown (α = 45 degrees). Any known techniques may be used to impart a desired resting configuration to the system, such as, for example, cold working or shape-setting by way of an athermal phase transformation (in the case of shape memory alloys).

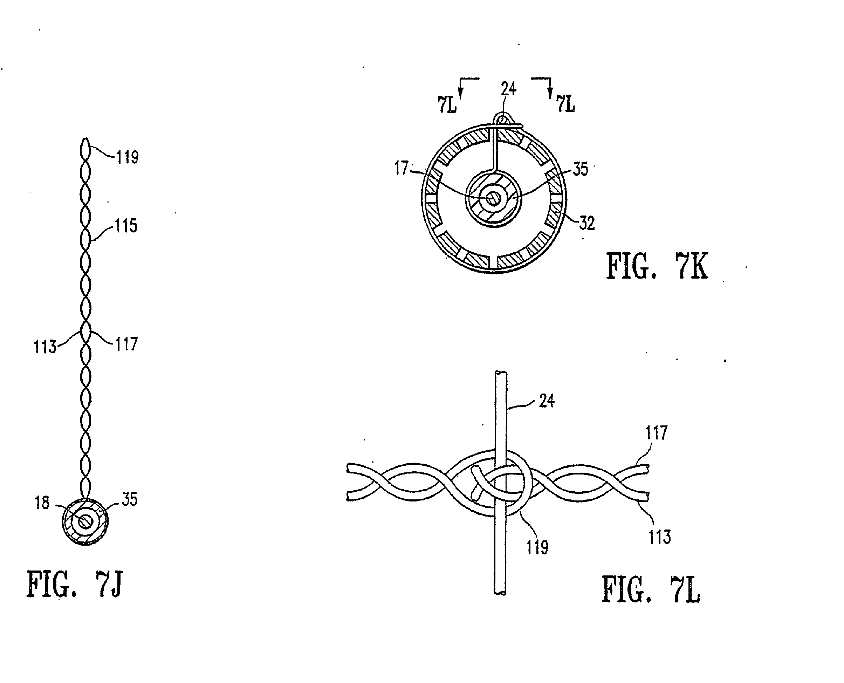

FIG. 7J shows a single belt example of the version shown in FIGS. 7C and 7D. Here, a single belt arm 113 is shown disposed about the distal end 35 of guidewire tube 17. Belt arm 113 is significantly longer than either belt arm 112 or 114 of the FIGS. 7C-7D embodiment, which is not part of the invention, so that it may extend at least around the circumference of any one of self-expanding members 31, 32, or 33. The distal portion 115 of belt arm 113 meets a more proximal portion 117 where one or both strands (when the belt arm 113 is a twisted variety) extends through an end loop 119 in the belt arm 115 distal portion. As discussed with other embodiments, which are not part of the invention, a release member such as release wire 24 may be inserted through end loop 119 and the intersecting portion of the belt arm proximal portion 117 to releasably secure belt arm 113 in a constraining configuration about the endovascular graft 11. FIG. 7K depicts a simplified schematic cross-sectional view of belt arm 113 (shown here untwisted) held in place by a release wire 24 about an exemplary self-expanding member 32. FIG. 7L is a detail of the connection formed where release wire 24 intersects the distal and proximal portions, 115 and 117, respectively, of belt arm 113.

All of the features discussed herein with respect to the FIGS. 7C-7D embodiment, which is not part of the invention, may be employed in the embodiment of FIGS. 7J-7K as well.

This helix configuration shown in the embodiments of FIGS. 7C-7D and 7J-7L, which are not part of the invention, is a particularly reliable configuration. It reduces the possibility that a portion of belt 21C becomes entangled with a self-expanding member (such as members 31, 32 and 33) or otherwise interferes with the safe and effective deployment of the prosthesis.

FIG. 7E depicts a particularly useful arrangement for configuring the belt end loops 81-83 with release wires 24-25 during assembly of delivery system 10. In this example, first and second end loops 81' and 81" of belt 21 are shown connected via release wire 24. To achieve the configuration of FIG. 7E, first end loop 81' is passed through aperture 88 disposed in second end loop 81". A portion of aperture 89 disposed in first end loop 81' should extend through the plane created by second end loop 81" as shown in FIG 7E.

Next, release wire 24 is passed through the portion of aperture 89 that extends beyond this plane so that wire 24 "locks" the two looped ends 81' and 81" together as shown. We have found that this is a stable configuration that lends itself well to a reliable and safe deployment protocol.

Other techniques for assembling wire 24 and first and second end loops 81' and 81" may be used; the method described above is merely exemplary. Wire 24 may simply pass through loop ends as configured and as shown at reference numerals 81, 82 and 83 in FIG. 7A, and 81B, 82B and 83B of FIG. 7B as well.

In the embodiment, which is not part of the invention, of FIG. 7F, belt 110 is a member in the shape of a wire formed into an end loop 116B having an aperture 120 for receiving a release wire. This arrangement may be used on one or both ends of belt 110 or, alone if belt 110 is in the form of a single belt arm as discussed above. Connection 123 is shown in FIG. 7F as a simple wrapping of the distal end 116A of the wire comprising belt 110. Connection 123 need not be limited to such a tapered or cylindrical sleeve or coating, however. Other methods to form end loop 116B are contemplated, including, for example, the use of adhesives, welding, brazing, soldering, crimping, etc. An optional protective sleeve or coating 127 (shown in sectional view in FIG. 7F) covers or is part of connection 123 and serves to protect the patient as well as components of the delivery system and prosthesis from damage.

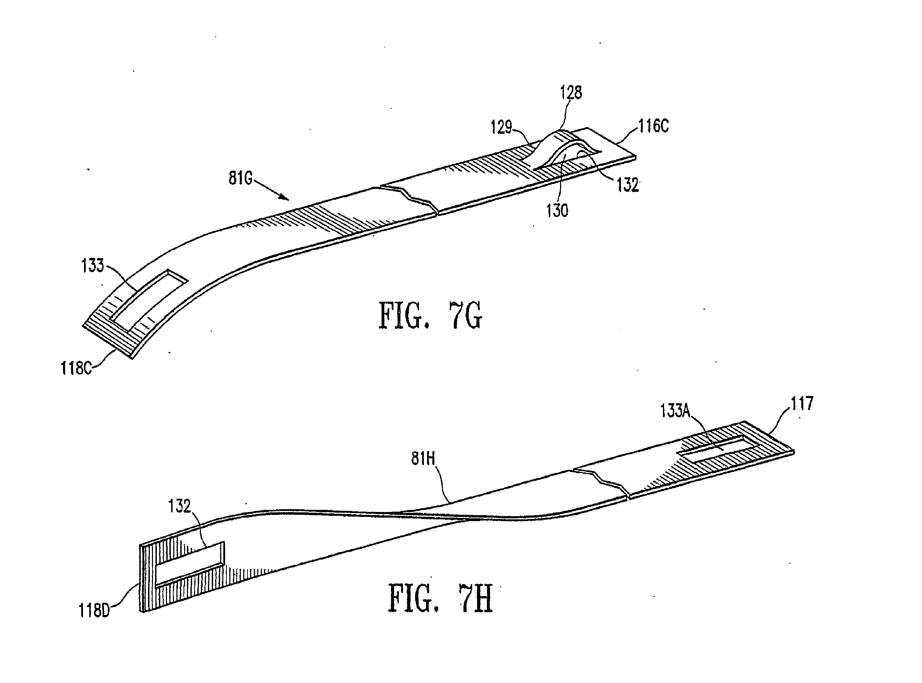

Turning now to FIGS. 7G and 7H, two alternative embodiments, which are not part of the invention, of a ribbon-like belt 81G and 81H are shown. In FIG. 7G, a section 128 of material has been partially displaced from belt 81G distal end 116C and worked into a loop-like member 129 such that two generally orthogonal apertures 130, 132 are formed in belt distal end 116C. A set of hinges or other protective mechanism or material may be used on each end of this member 128 so that further tearing or peeling of this member may be prevented. Section 128 may be formed integrally from the belt distal end 116C as shown in FIG. 7G or maybe a separate component that is attached to the belt distal end by any suitable means.

Second belt distal end 118C in FIG. 7G is shown as having an aperture 133 disposed therein. In use, a half-twist is imparted to the ribbon-like belt 81G as the second distal end 118C is brought through aperture 130 such that apertures 132 and 133 are at least partially aligned. A release wire (such as wire 24) is then brought through apertures 132 and 133 to releasably join ends 116C and 118C.

FIG. 7H shows yet another embodiment, which is not part of the invention, of a belt 81H where a simple rectangular aperture 133A is disposed in the distal end 117 of belt 81 H through which another belt end and release wire may be disposed as taught herein. As with the embodiment of FIG. 7G, which is not part of the invention, a half-twist is imparted to the belt 81 H in use so that the second distal end 118D is brought through aperture 133. A release wire may then be threaded through apertures 132 and 133 to releasably join ends 117 and 118D. In this embodiment, which is not part of the invention, aperture 132 should be large enough to accommodate both second distal end 118D and a release wire.

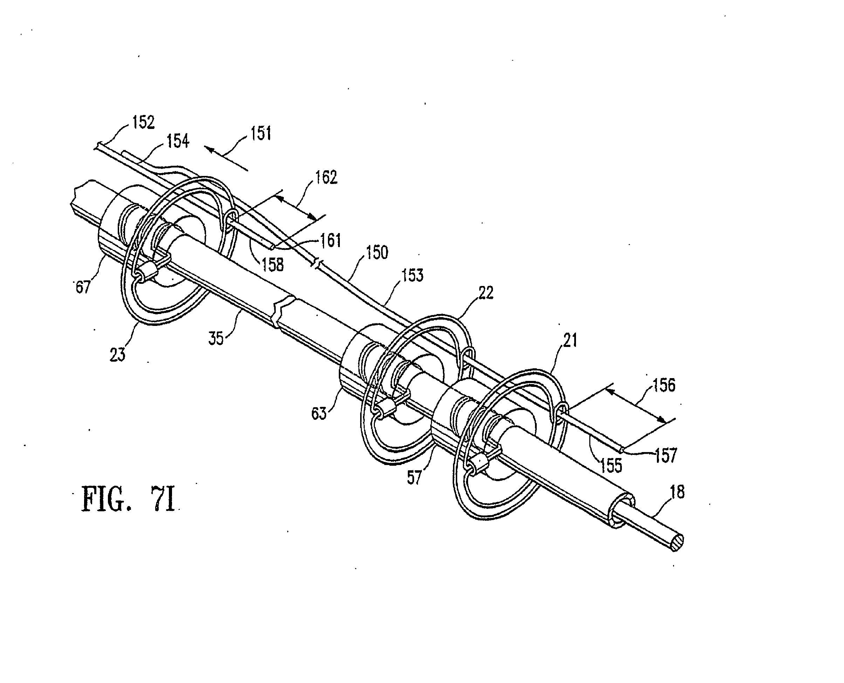

FIG. 7I shows a perspective view of a belt assembly similar to that shown in FIG. 7A, wherein like elements are shown with like reference numerals. An alternative embodiment, which is not part of the invention, of a release wire consisting of a branched release wire 150 is illustrated in FIG. 7I. The branched release wire 150 engages belts 21-23 and is configured to release belts 21-23 at different times with a proximal withdrawal movement of the branched release wire 150, the direction of which is indicated by arrow 151. Branched release wire 150 has a main portion 152 and a branch portion 153. Branch portion 153 is secured to main portion 152 by a solder joint 154. The joint 154 could also be made by any other suitable means, such as welding, bonding with an epoxy, mechanically binding the joint, or the like. The embodiment of the branched release wire shown in FIG. 7I consists of wire which is generally round in cross section. The wire of the branched release wire can have the same or similar material and mechanical properties to the wire of the release wires 24 and 25 discussed above. Branch portion 153 engages first distal belt 21 and second distal belt 22. A distal segment 155 has a length L indicated by arrow 156 which extends distally from first distal belt 21 to the distal end 157 of branch portion 153.

Main portion 152 of the branched release wire 150 engages the proximal belt 23 and has a distal segment 158 that extends distally from the proximal belt 23 to a distal end 161 of the main portion. The length L' of the distal segment 158 of the main portion 152 is indicated by arrow 162. Length L of distal segment 155 is greater than length L' of distal segment 158. In this way, as the branched release wire is withdrawn proximally, proximal belt 23 is released first, first distal belt 21 is released second and second distal belt is released last. Such a branched release wire allows a wide variety of belt release timing with a single continuous withdrawal or movement of a proximal end (not shown) of the branched release wire 150. The proximal end of the branched release wire may be terminated and secured to a release wire handle or the like, as discussed herein with regard to other embodiments of release wires. The ability to deploy multiple release wires in a desired timing sequence with a single branched release wire 150 gives the designer of the delivery system great flexibility and control over the deployment sequence while making the deployment of the belts simple and reliable for the operator of the delivery system. Although the branched release wire 150 has been shown with only a single branch, any number of branches or desired configuration could be used to achieve the deployment sequence required for a given embodiment of a delivery system. For example, a separate branch could be used for each belt in a multiple belt system, with varying distal segment length used to control the sequence of deployment. Also, multiple branched release wires, or the like, could be used in a single delivery system to achieve the desired results.

A number of embodiments for the belt and belt arm components are described herein. In general, however, we contemplate any belt or belt arm configuration in which the belt may be used to releasably hold or restrain an implant member in conjunction with a release member. The particular examples disclosed herein are not meant to be limiting, and other variations not explicitly disclosed herein, such as those in which multiple apertures (which may have varying shapes and sizes) are disposed along the belt length, those in which the belt or belt arm distal ends comprises a separate material or element that is affixed to the belt or belt arm. Furthermore, various embodiments, which are not part of the invention, of the ends of the belts or belt arms taught herein may exist in any combination in a single delivery system.

Turning now to FIG. 6A, belts 21-23 lie within circumferential grooves or channels 95, 96 and 97, respectively, formed into the respective self-expanding members 31, 32 and 33. Grooves 95-97 prevent axial displacement of the belts 21-23 prior to activation or release of the releasable members 24 and 25, i.e., proximal retraction of the first and second release wires. Although grooves 95-97 are illustrated in the embodiment, which is not part of the invention, shown, other alternatives are possible to achieve the same or similar function of the grooves. For example, abutments extending slightly from the self-expanding members 31-33 on either side of the belts 21-23 in their constraining configuration could prevent axial movement of the belts. A detachable adhesive or the like could also be used.

As shown in FIG. 10, the release of end loops 81-83 occurs when the distal end portions 84 and 85 of the release wires 24 and 25, respectively, pass from within the overlapped end loops 81-83. If the end loops 81-83 move axially in response to movement of the release wires 24 and 25 due to frictional forces imposed on the end loops 81-83 by the release wires, the point at which the distal ends of the release wires 84 and 85 pass from within the end loops 81-83 would vary depending on the amount of movement of the end loops 81-83.

If the end loops 81-83 were to be axially displaced from their normal position relative to the distal ends of the release wires prior to deployment, the timing of the release of the belts 21-23 could be adversely affected. Thus, the prevention of axial displacement of the belts 21-23 during proximal retraction of the release wires 24 and 25 facilitates accurate release of the belts by keeping the overlap joint of the belt looped end portions in a constant axial position during such retraction.

In addition, it may be desirable to keep belts 21-23 positioned at or near the general center of a given constrained self-expanding members 31-33 so that the self-expanding member 31-33 is substantially uniformly and evenly constrained over its axial length. If belts 21-23 constrain the self-expanding members 31-33 at a non-centered axial position on the member, an end of the member opposite that of the non-centered position may be less constrained and may interfere with axial movement of the outer tubular member 53 (and consequently deployment of the endovascular graft 11).

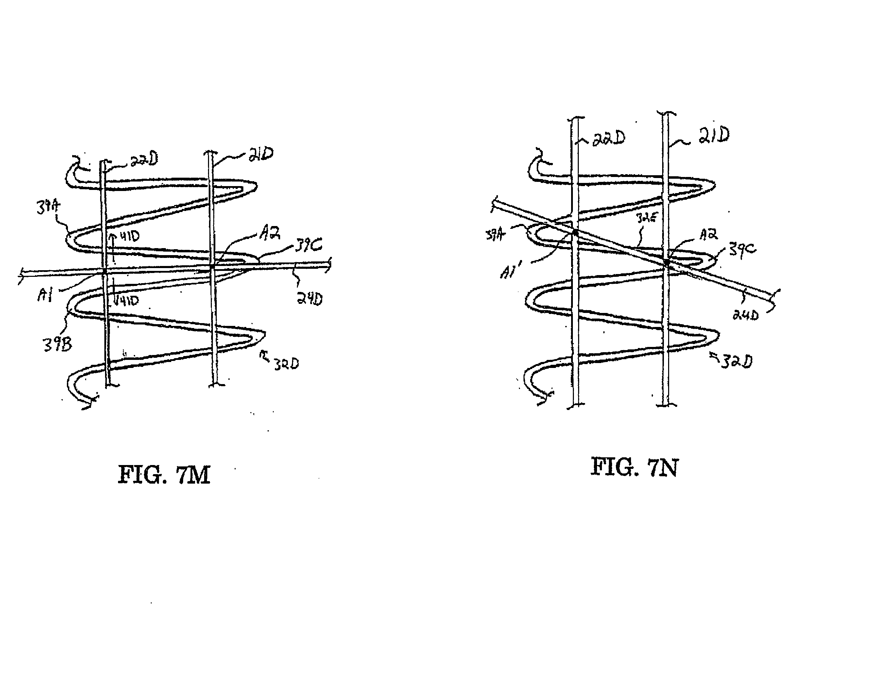

The number and location of the belts on the guidewire tube relative to the crowns or ( apices of the associated self-expanding member can also play a role in packing efficiency of the grafts 11, 401 in the delivery systems 10, 400. FIGS. 7M-7N schematically illustrate optional configurations that enhance such packing efficiency.

FIGS. 7M-7N schematically shows, in simplified format, two belts 21D and 22D disposed in conjunction with a release wire or member 24D and constraining a single self-expanding member 32D. Any self-expanding members discussed in conjunction with the present disclosure, such as proximal self-expanding members 31 or first or second distal self-expanding members 32, 33 of stent-graft 11 or proximal self-expanding members 407, 408 or first or second distal self-expanding members 422, 411, of bifurcated stent-graft 401 may be used in the optional configurations of FIGS. 7M-7N.

FIG. 7M depicts an example in which the end loops (not shown) of belts 21D and 22D are joined by a single release wire 24D at points A1 and A2. Note that point A1 lies between two adjacent self-expanding member crowns or apices 39A and 39B, while at point A2 the end loops (not shown) of belt 21D join release wire 24D at or within the vicinity of a single self-expanding member crown or apex 39C.

When the release wire 24D is belted at points A1 and A2, forces imposed by the belt have a tendency to push the crowns or apices 39A and 39B of self-expanding member 32D away from one another as shown by arrows 41D in FIG. 7M. The crowns or apices, however, are more resistant to this belt force at point A2 in the vicinity of or within crown 39C, as compared to point A1, between crowns 39A and 39B. This difference can result in an asymmetric packing of the self-expanding member 32D when belted prior to deployment, which in turn can result in lower reliability and increased difficulty in deploying grafts 11, 401, due, e.g., to variability in unsheathing forces and the forces required to withdraw the release wire 24D as described herein.

FIG. 7N shows an alternative configuration in which the end loops (not shown) of belts 21D and 22D are joined again by a single release wire 24D, except that in this configuration points A1' and A2 both are within a crown 39A and 39C, respectively, of the self-expanding member 32D, points at which the self-expanding member can best resist the forces imposed by the belt that tend to pull the crowns or apices apart as described above. As a result, there is a more uniform belted packing of the self-expanding members during assembly of the delivery systems 10, 400, resulting in a more reliable deployment of graft 11, 401.

When the self-expanding member 32D takes the form of a self-expanding stent that utilizes one or more integral barbs disposed on selected struts (as discussed in co-pending U.S. Patent Application Serial No. 10/327,711), such barbs may be tucked or hidden when the stent is belted underneath an adjacent stent strut (that may have a barb tuck pad integral to the strut) to ensure the barb is not exposed until the release wire is retracted, freeing the belts and the stent to expand into place. However, the radial profile of the stent struts when the stent is belted as discussed herein tends to bulge outward (out of the plane of the drawing sheet in FIG. 7N) in the region of the barb between the belts, not unlike the shape of a football. This asymmetric radial profile may lead to lower reliability and increased difficulty in deploying grafts 11, 401 as discussed above.

To minimize the potential for this inefficiency, it is useful to select points A1' and A2 to ensure that the release wire such as wire 24D in FIG. 7N pass directly over or near stent strut, such as strut 32E, where a tucked barb is disposed (note that for ease of illustration, in the schematic illustration of FIG. 7N, adjacent stent struts are shown spaced apart and without barbs). The presence of the release wire such as wire 24D tends to counteract the radial bulging forces presented by the tucked barb, resulting in a more cylindrical and more uniform radial stent profile.

Note also that it is disclosed to use two or more belts per self-expanding member such as illustrated in FIGS. 7M and 7N.

Tubular body member 205 of the endovascular graft 11 is disposed between and secured to the second distal self-expanding member 33 and the proximal self-expanding member 31. The tubular body member comprised of flexible material 204, is shown constrained in an idealized view in FIGS. 1, 3 and 6, for clarity. In practice, tubular body member 205 while constrained is tightly compressed with minimal air space between layers of flexible material 204 so as to form a tightly packed configuration as shown in FIG. 3. Tubular body member 205 is optionally radially constrained by an inside surface 206 of the inner lumen 52 of outer tubular member 53.

An inner tubular member 207 is slidably disposed within the inner lumen 52 of outer tubular member 53. Release wires 24 and 25, guidewire tube 17 and an inflation tube 211 are disposed within an inner lumen 212 of the inner tubular member 207. Inner lumen 212 is optionally sealed with a sealing compound, depicted in FIGS. 1, 2 and 6 by reference numeral 213 at distal end 214. The sealing compound 213 prevents leakage of fluids such as blood, etc., from a proximal end 215, shown in FIG. 8, of the inner tubular member 207. Sealing compound 213 fills the space within the inner lumen 212 of the inner tubular member 207 between an outer surface 216 of the guidewire tube 17, the outer surface 217 of the inflation tube 211 and outer surfaces 221 and 222 of a tubular guide 223 for the first release wire 24 and a tubular guide 224 for the second release wire 25. The sealing compound 213 can be any suitable material, including epoxies, silicone sealer, ultraviolet cured polymers, or the like.

In FIG. 2, the tubular guides 223 and 224 for the first release wire 24 and the second release wire 25 allow axial movement of the release wires with respect to the sealing compound 213 and inner tubular member 207. The inside diameter of the inner lumens of the tubular guides 223 and 224 are sized to fit closely with an outer diameter or transverse dimension of the release wires 24 and 25. Alternatively, tubular guides 223 and 224 may be replaced by a single tubular guide that houses one or more release wires, such as wires 24 and 25.

Turning to FIG. 8, the inner tubular member 207 terminates proximally with the proximal adapter 42 having a plurality of side arms 225, 226 and 227 and a proximal exit port 231 for the inner lumen 34 of the guidewire tube 17. First release wire side arm 225 branches from a proximal adapter body portion 233 and has an inner lumen 234 and proximal end 86 of the first release wire 24. A proximal extremity 236 of the first release wire 24 is anchored to the first release wire proximal handle 93 which is threaded onto the proximal end 238 of the first release wire side arm 225. The proximal extremity 236 of first release wire 24 is configured as an expanded bushing or other abutment that captures the handle 93 and translates proximal axial movement of the handle 93 to the first release wire 24 but allows relative rotational movement between the handle 93 and the proximal end 86 of the first release wire 24.

A similar configuration exists for the proximal end 87 of the second release wire 25. There, a second release wire side arm 226 branches from the proximal adapter body portion 233 and has an inner lumen 244 that houses the proximal end 87 of the second release wire 25 which is free to slide in an axial orientation within the lumen 244. A proximal extremity 246 of the second release wire 25 is configured as an expanded bushing or other abutment that captures the second release wire handle and translates axial proximal movement of the second release wire handle 94 to the second release wire 25, but allows relative rotational movement between the proximal end 87 of the second release wire 25 and the second release wire handle 94.

The first release wire handle 93 and second release wire handle 94 may optionally be color coded by making each, or at least two, release wire handles a color that is distinctly different from the other. For example, the first release wire handle 93 could be made green in color with the second release wire handle 94 being red in color. This configuration allows the operator to quickly distinguish between the two release wire handles and facilitates deployment of the belts in the desired order.

In another embodiment, which is not part of the invention, instead of color coding of the release wire handles 93 and 94, the spatial location of the handles can be configured to convey the proper order of deployment of the release wires to the operator of the delivery system. For example, if three release wire handles are required for a particular embodiment, which is not part of the invention, the corresponding three side arms can be positioned along one side of the proximal adapter. In this configuration, the release wire handle that needs to be deployed first can extend from the distal-most side arm. The release wire handle that needs to be deployed second can extend from the middle side arm. The release wire handle that is to be deployed last can extend from the proximal-most side arm. For such a configuration, the operator is merely instructed to start deployment of the release wires at the distal-most release wire handle and work backward in a proximal direction to each adjacent release wire handle until all are deployed. Of course, an opposite or any other suitable configuration could be adopted. The configuration should adopt some type of spatially linear deployment order, either from distal to proximal or proximal to distal, in order to make reliable deployment of the release wires in the proper order easy to understand and repeat for the operator of the delivery system. Other types of release order indicators such as those discussed above could also be used, such as numbering each release wire handle or side arm with a number that indicates the order in which that handle is to be deployed.

The proximal end 36 of the guidewire tube 17 terminates and is secured to an inner lumen 251 of the proximal end 259 of the proximal adapter 42. Inner lumen 251 typically has a longitudinal axis 253 that is aligned with a longitudinal axis 254 of the proximal section 13 elongate shaft 12 so as to allow a guidewire to exit the proximal end 15 of the elongate shaft 12 without undergoing bending which could create frictional resistance to axial movement of the guidewire. A proximal port 255 of the proximal adapter 42 may be directly fitted with a hemostasis valve, or it may be fitted with a Luer lock fitting which can accept a hemostasis valve or the like (not shown).

The proximal adapter 42 may be secured to the proximal end 215 of the inner tubular member 207 by adhesive bonding or other suitable method. A strain relief member 256 is secured to the distal end 257 of the proximal adapter 42 and the inner tubular member 207 to prevent kinking or distortion of the inner tubular member 207 at the joint.

As seen in FIG. 1, the proximal end 261 of the outer tubular member 53 is secured to a proximal fitting 262 that slides over an outer surface 258 of the inner tubular member 207. A seal 263 located in proximal fitting 262 provides a fluid seal for the lumen 265 formed between the outer surface 258 of the inner tubular member 207 and the inner surface 206 of the inner lumen 52 of the outer tubular member 53. The fit between the outer surface 258 of the inner tubular member 207 and the inner surface 206 of the outer tubular member 53 is typically close, but still allows for easy relative axial movement between outer tubular member 53 and inner tubular member 207. A stop 266 is disposed and secured to the outer surface 258 of the inner tubular member 207 distal of the proximal adapter 42 to limit the amount of proximal axial movement of the outer tubular member 53 relative to the inner tubular member 207.

When the outer tubular member 53 is positioned on the proximal shoulder 48 of the distal nose piece 44 prior to deployment of endovascular graft 11, the distance between a proximal extremity 267 of proximal fitting 262 and a distal extremity 268 of stop 266 is approximately equal to or slightly greater than an axial length of the endovascular graft 11 in a constrained state. This configuration allows the outer tubular member 53 to be proximally retracted to fully expose the endovascular graft 11 in a constrained state prior to deployment of the graft. This distance may be greater, but should not be less than the length of the endovascular graft 11 in a constrained state in order to completely free the constrained graft 11 for radial expansion and deployment.

Retraction limiters may alternatively be used to prevent excessive axial movement of the release wires 24 and 25 in a proximal direction during deployment. Particularly in embodiments of the invention, which is not part of the invention, where single release wires are used to constrain and deploy multiple belts such as with first release wire 24, retraction limiters may be used to allow enough axial movement of the release wire 24 to deploy a first belt 21, but prevent deployment of a second more proximally located belt 22. For example, as shown in FIG. 8, a retraction limiter in the form of a filament 268 could be disposed between the proximal adapter 42 and the handle 93 of the first release wire 24 such that proximal retraction of the first release wire 24 sufficient for deployment of the first distal belt 21 could be achieved, but not so much as to allow deployment of the second distal belt 22. In order to deploy the second distal belt 22, the filament 268 would have to be severed or otherwise released. This type of configuration can allow more control over deployment of the endovascular graft 11 and allow deployment in stages which are sequentially controlled to prevent inadvertent deployment of a portion of the graft 11 in an undesirable location within the patient's vessels.

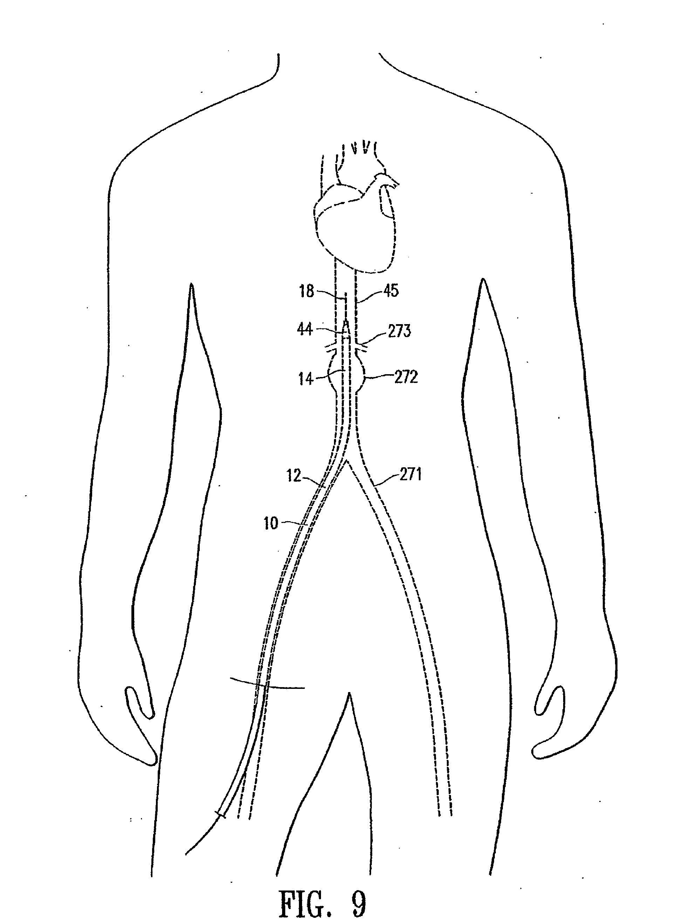

In use, the delivery system 10 is advanced into a patient's arterial system 271 percutaneously as shown in FIG. 9 and positioned so that the endovascular graft 11 spans an aneurysm 272 in the patient's aorta 45 as illustrated in FIGS. 1 and 9-12. It is generally desirable to have the tubular body portion 205 of the graft 11 positioned below the renal arteries 273 in order to prevent significant occlusion of the renal arteries. The procedure typically begins with the placement of guidewire 18 into the patient's target vessel 45 across the target location, e.g., the aneurysm 272. Common percutaneous techniques known in the art may be used for the initial placement of the guidewire 18. For example, as shown in FIG. 9, percutaneous access to the aorta may be had through the femoral or iliac artery, although other access sites may be used. The delivery system 10 may then be advanced over the guidewire 18 to a desired position within the patient's vessel 45. Alternatively, delivery system 10 and guidewire 18 could be advanced together into the patient's vasculature 272 with the guidewire 18 extending distally from the distal port 38 of the guidewire tube 17. In addition, it may be desirable in some cases to advance the delivery system 10 to a desired location within the patient without the use of a guidewire 18.

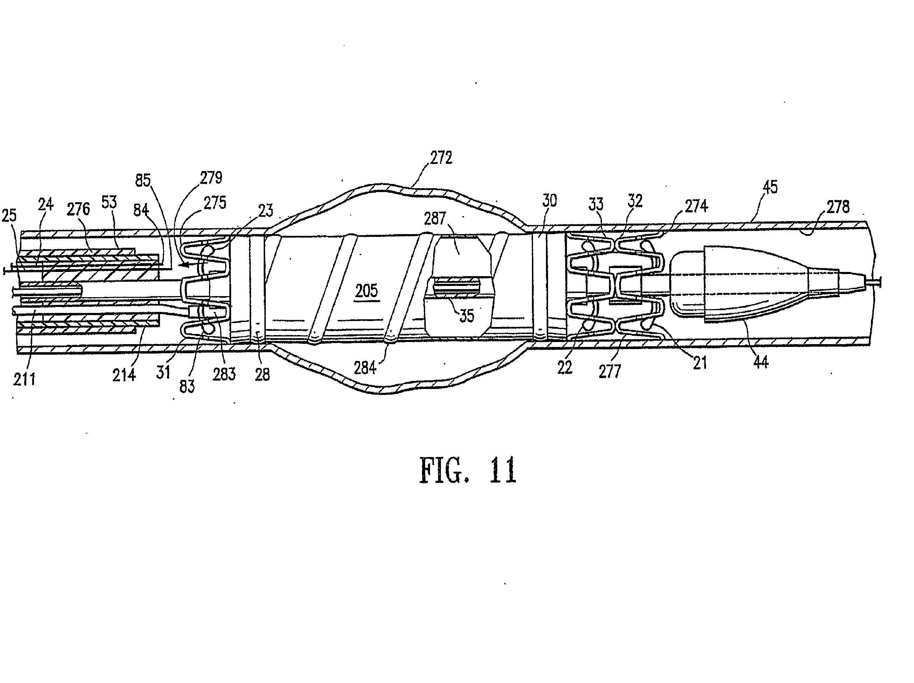

Generally, the position of the delivery system 10 is determined using fluoroscopic imaging or the like. As such, it may be desirable to have one or more radiopaque markers (not shown) secured to the delivery system at various locations. For example, markers may be placed longitudinally coextensive with the respective distal and proximal extremities 274 and 275, as shown in FIG. 11. In this way, it can be readily determined whether the graft 11 is spanning the aneurysm 272 of the patient's artery. Imaging markers, such as radiopaque markers, may also be secured to desirable positions on the endovascular graft 11 itself. Other types of imaging and marking systems may be used such as computed tomography (CT), magnetic resonance imaging (MRI) and nuclear magnetic resonance (NMR) imaging systems and markers.

Once the distal section 14 of the delivery system 10 is properly positioned within the patient's artery 45, the operator moves the proximal end 261 of outer tubular member 53 in a proximal direction relative to inner tubular member 207. The relative axial movement is carried out by grasping the proximal end 215 of the inner tubular member 207 or proximal adapter 42, and grasping the proximal end 261 of the outer tubular member 53, and moving the respective proximal ends towards each other. This retracts the distal section 276 of the outer tubular member 53 from the constrained endovascular graft 11 and frees the graft for outward radial expansion and deployment. However, in this deployment scheme, note that the operator is free to reinsert graft 11 back into the outer tubular member 53 if necessary, as the release bands have not yet released the graft.

Once the distal section 276 of the outer tubular member 53 has been retracted, handle 93 of the first release wire 24 may then be unscrewed or otherwise freed from the proximal adapter 42 and retracted in a proximal direction indicated by arrow 279 in FIG. 10 until the distal end 84 of the first release wire 24 passes from within the end loops 81 of the first distal belt 21. When this occurs, the looped ends 81 of the first distal belt 21 are released and the first distal belt 21 ceases to radially constrain the first distal self-expanding member 32 which thereafter self-expands in a radial direction into an inner surface 278 of the patient's aorta 45 as shown in FIG. 10.

If the operator of the delivery system 10 is not satisfied with the position, particularly the axial position, of the endovascular graft 11 after deployment of the first distal self-expanding member 32, it may then be possible to re-position the endovascular graft 11 by manipulating the proximal end 15 of the elongate shaft 15. Movement of the elongate shaft 12 can move the endovascular graft 11, even though physical contact between the expanded member 32 and the vessel inner surface 278 generates some static frictional forces that resist such movement. It has been found that the endovascular graft 11 can be safely moved within a blood vessel 45 even in the state of partial deployment discussed above, if necessary.

Once the operator is satisfied with the position of the graft 11, the first release wire 24 may then be further proximally retracted so as to deploy the second distal belt 22 in a manner similar to the deployment of the first distal belt 21. The deployment of the second distal belt 22 occurs when the distal end 84 of the first release wire 24 passes from within end loops 82 of the second distal belt 22 which are held in a radially constraining configuration by the first release wire 24. Upon release of the second distal belt 22, the second distal self-expanding member 33 expands in a radial direction such that it may engage inner surface 278 of the patient's aorta 45. The amount of outward radial force exerted by the serf-expanding members 32 and 33 on the inside surface 278 of the patient's aorta 45, which may vary between members 32 and 33, is dependent upon a number of parameters such as the thickness of the material which comprises the self-expanding members 32 and 33, the nominal diameter which the self-expanding members 32 and 33 would assume in a free unconstrained state with no inward radial force applied, material properties of the members and other factors as well.

Once the distal members 32 and 33 are deployed, the handle 94 for the second release wire 25 can be disengaged and axially retracted in a proximal direction from the proximal adapter 42 until the distal end 85 of the second release wire 25 passes from within the end loops 83 of the proximal belt 23. Once the proximal belt 23 is released, the proximal self expanding member 31 is deployed and expands in an outward radial direction, such that it may engage or be in apposition with the inner surface 278 of the patient's aorta 45 as shown in FIG. 11. Thereafter, the endovascular graft 11 may be inflated with an inflation material (not shown) introduced into the proximal injection port 282 in the proximal adapter 42, through the inflation tube 211, and into the inflation port 283 of the endovascular graft 11. Inflation material may be injected or introduced into the inflation port 283 until the proximal and distal inflatable cuffs 28 and 30 and inflatable channels 284 of the graft 11 have been filled to a sufficient level to meet sealing and other structural requirements necessary for the tubular body to meet clinical performance criteria.

Before or during the deployment process, and preferably prior to or simultaneous with the step of inflating the endovascular graft 11, it may be beneficial to optionally treat vessel 45 in which the graft 11 is deployed so to obtain a better seal between the graft 11 and the vessel inner surface 278, thus improving the clinical result and helping to ensure a long term cure.

One approach to this treatment is to administer a vasodilator, or spasmolytic, to the patient prior to deploying graft 11. This has the effect of reducing the tone of the smooth muscle tissue in the patient's arteries; specifically, the smooth muscle tissue in the wall of vessel 45 into which graft 11 is to be deployed. Such tone reduction in turn induces the dilation of vessel 45, reducing the patient's blood pressure. Any number of appropriate vasoactive antagonists, including the direct acting organic nitrates (e.g., nitroglycerin, isosorbide dinitrate, nitroprusside), calcium channel blocking agents (e.g., nifedipine), angiotensin-converting enzyme inhibitors (e.g., captopril), alpha-adrenergic blockers (e.g., phenoxybenzamine, phentolamine, prasozin), beta-adrenergic blockers (e.g., esmolol) and other drugs may be used as appropriate. Particularly useful are those vasodilators that can be administered intravenously and that do not have unacceptable contraindications such as aoritic aneurysm dissection, tachycardia, arrhythmia, etc.

The degree of vasodilatation and hypotensive effect will depend in part on the particular vessel in which graft 11 is to be placed and the amount of smooth muscle cell content. Generally, the smaller the vessel, the larger percentage of smooth muscle cell present and thus the larger effect the vasodilator will have in dilating the vessel. Other factors that will effect the degree of vasodilatation is the health of the patient; in particular, the condition of the vessel 11 into which graft 11 is to be placed.

In practice, once the vasodilator has been administered to the patient, graft 11 may be deployed and filled with inflation material so that graft 11 reaches a larger diameter than would otherwise be possible if such a vasodilator was not used. This allows the inflation material to expand the diameter of graft 11, for a given inflation pressure, beyond that which would be achievable if the vessel 45 were in a non-dilated state (and nominal diameter). Alternatively, a larger diameter graft 11 may be chosen for deployment. We anticipate that an increased vessel diameter of between two and twenty percent during vasodilatation may be optimal for achieving an improved seal.

The vessel 45 in which graft 11 is to be placed may optionally be monitored pre-and/or post-dilation but before deployment of graft 11 (via computed tomography, magnetic resonance, intravenous ultrasound, angiography, blood pressure, etc.) so to measure the degree of vasodilatation or simply to confirm that the vasodilator has acted on the vessel 45 prior to deploying graft 11.

Once the vasodilator wears off, preferably after between about five and thirty minutes from the time the drug is administered, the vessel 45 surrounding graft 11 returns to its normal diameter. The resultant graft-vessel configuration now contains an enhanced seal between graft 11 and vessel inner surface 278 and provides for reduced luminal intrusion by graft 11, presenting an improved barrier against leakage and perigraft blood flow compared to that obtainable without the sue of vasodilators or the like.

Such vasodilating techniques may be used with all of the examples of the present invention, including the tubular graft 11 as well as a bifurcated graft version of the expandable intracorporeal device of the present invention as is discussed in detail below.

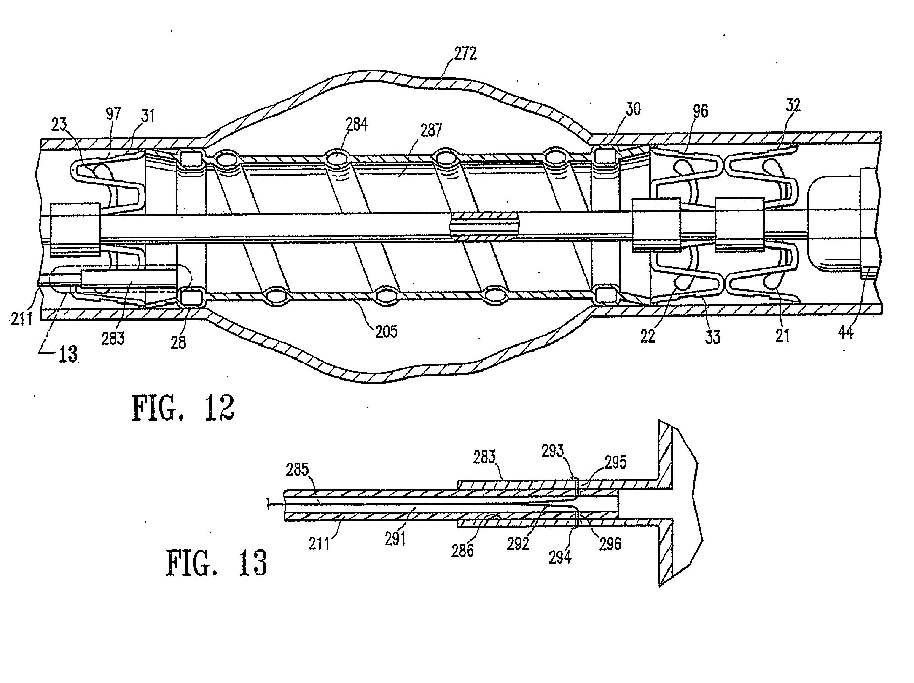

Once graft 11 is fully deployed, a restraining or retention device, such as retention wire 285 that binds the distal end 286 of the inflation tube 111 to the inflation port 283, as shown in FIGS. 12 and 13, is activated. The retention wire 185 is activated by pulling the proximal end of the wire in a proximal direction so as to disengage the distal ends 293 and 294 from the holes 295 and 296. This eliminates the shear pin function of the distal ends 293 and 294 and allows the distal end 286 of the inflation tube 211 to be disengaged from the inflation port 283. The release wires 24 and 25 may then be fully retracted from the elongate shaft 12 in a proximal direction and the delivery system 10 retracted in a proximal direction from the deployed endovascular graft 11. The unconstrained distal belts 21-23 slip through the openings in the expanded members 31, 32 and 33 as the delivery system 10 is retracted and are withdrawn through the inner passageway 287 of the deployed graft 11. The distal nosepiece 44 is also withdrawn through the inner passageway 287 of the deployed graft 11 as the delivery system 10 is withdrawn as shown in FIG. 10-12.

FIG. 13 illustrates the junction between the distal end 286 of inflation tube 211 and inflation port 283. Typically, retention wire 285 extends from the inflation port 283 proximally to the proximal end 15 of delivery system 10. In this way, an operator can disengage the distal end 286 of the inflation tube 211 from the inflation port 283 by pulling on the proximal end 283 of retention wire 285 from a proximal end 15 of delivery system 10. The retention wire 285 can be a small diameter wire made from a material such as a polymer, stainless steel, nickel titanium, or other alloy or metal; in a particular embodiment of the invention, retention wire 285 may be a spring formed of a variety of suitable spring materials. Alternatively retention wire 285 may have a braided or stranded configuration.

FIG. 13 shows a single retention filament or wire 285 disposed within the lumen 291 of the inflation tube 211. The distal end 292 of retention wire 285 may have one or more loops 293 and 294, respectively, disposed within one or more side holes disposed in the inflation port 283 of the distal end 286 of the inflation tube 211. A number of side hole configurations may be utilized. The disclosure of FIG. 13 has two sets of opposed side hole locations 295 and 296. The distal loops 293 and 294 of the retention wire 285 act to interlock the side holes 295 and 296 by creating a removable shear pin element which prevents relative axial movement between the distal end 286 of the inflation tube 211 and the inflation port 283. Alternate examples may include multiple retention filaments or wires disposed within the lumen 291 of the inflation tube 211. An external sleeve (not shown) may be added over this assembly to further secure the interface and prevent leakage of inflation material through side holes 295 and 296. This sleeve is attached to inflation tube 211 and is received with it.

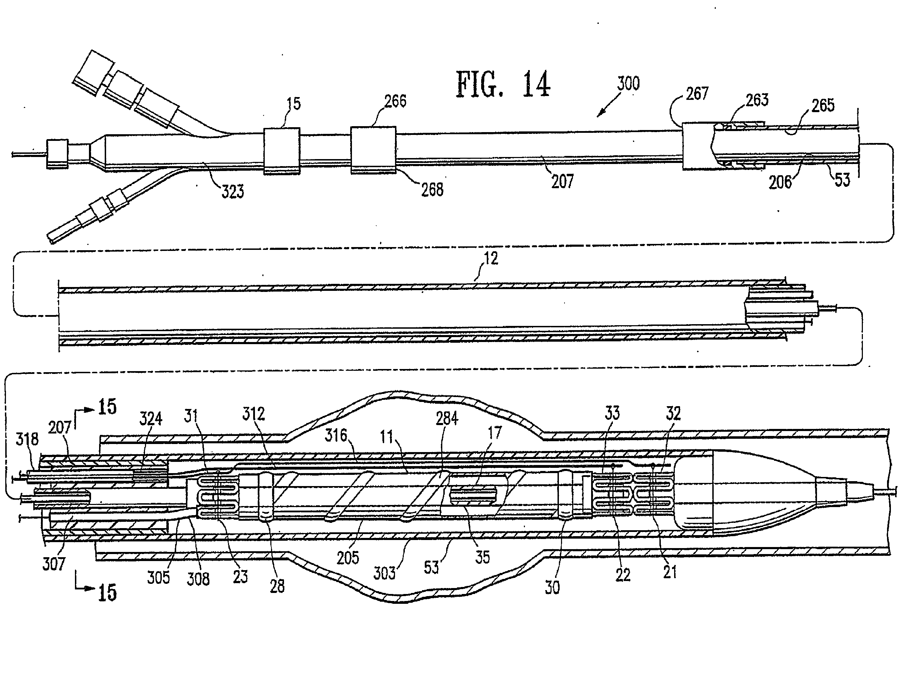

FIGS. 14-17 illustrate an embodiment of the delivery system shown in FIG. 1. In FIGS. 14-17, like elements with respect to the example of FIG. 1 will be shown with like reference numerals where appropriate. The delivery system 300 has an outer tubular member 53 and inner tubular member 207 at a distal section 303 of the delivery system 300. An endovascular graft 11 is disposed within the outer tubular member in the distal section 303. An inflation tube 305, similar to that of the example shown in FIG. 1 is coupled to an inflation port 283 of the endovascular graft 11. However, the inflation tube 305, having a proximal end 307 and a distal end 308, does not extend the majority of the length of the delivery system 300. Instead, the proximal end 307 of the inflation tube 305 terminates at a proximal end 311 of the potted section 213 as shown in FIGS. 14 -16.

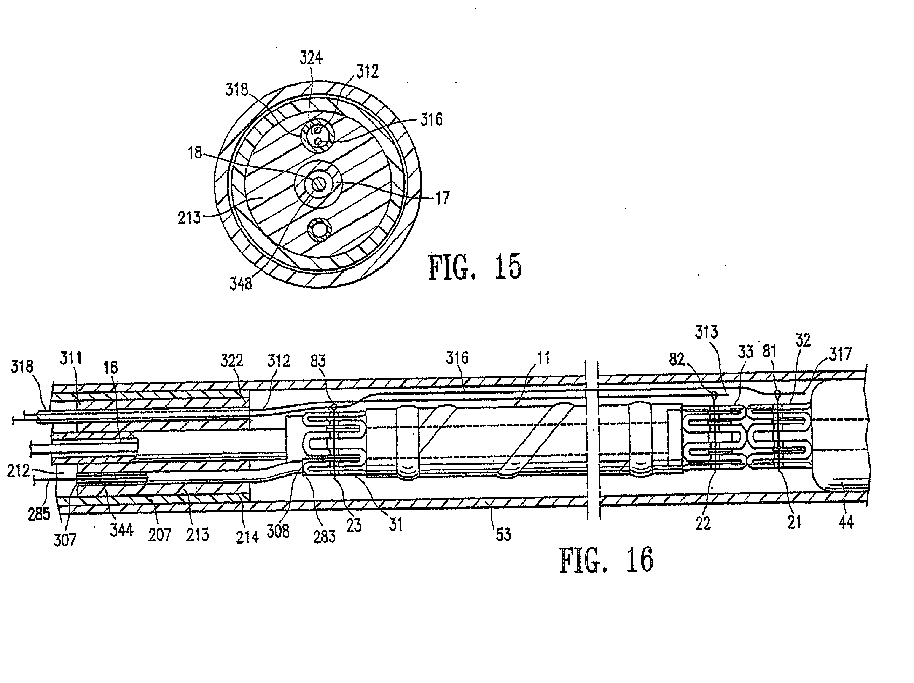

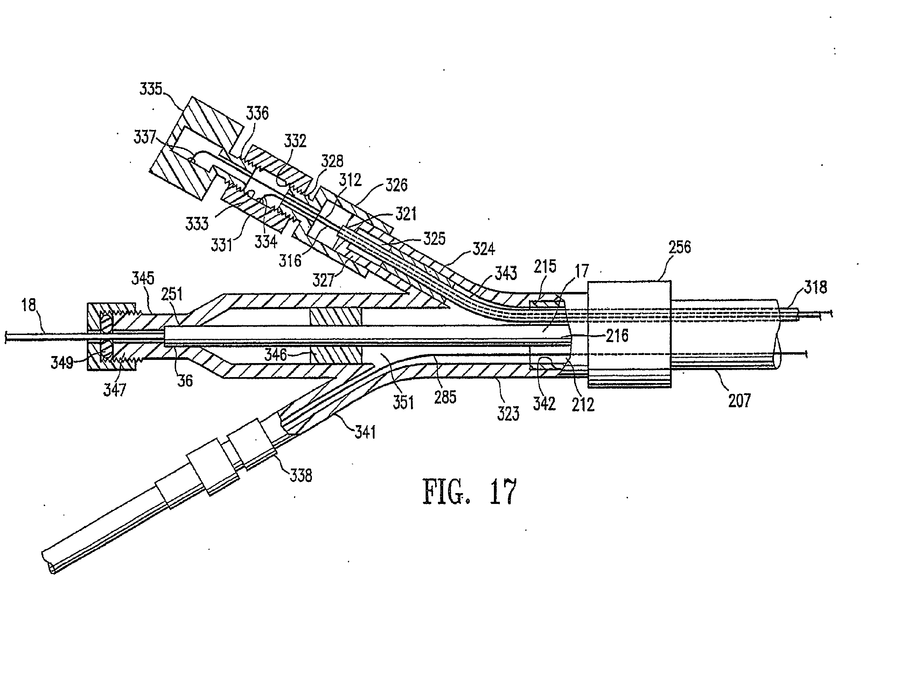

Referring to FIG. 14 and 16, first release wire 312 having distal end 313 engages end loops 82 of second distal belt 22. The second distal belt 22 is disposed about and constrains the second distal self-expanding member 33. A second release wire 316 having a distal end 317 engages the end loops 81 of the first distal belt 21 and the end loops 83 of the proximal belt 23. The first distal belt 21 is disposed about and constrains the first distal self-expanding member 32. The proximal belt 23 is disposed about and constrains the proximal self-expanding member 31. A release wire tube 318, having a proximal end 321, as shown in FIG. 17, and a distal end 322, shown in FIG. 16, extends from the potted section 213 of the distal section 303 of the delivery system 300 to the proximal adapter 323 shown in FIG. 17. The release wire tube 318 has a lumen 324, as shown in FIG. 15, which contains the first release wire 312 and the second release wire 316.

The proximal adapter 323 has a first side arm 324 with an inner lumen 325 that secures the proximal end 321 of the release wire tube 318. A threaded end cap 326 is secured to a proximal end 327 of the first side arm 324 and has a threaded portion 328. A second release wire handle 331, having a distal threaded portion 332 and a proximal threaded portion 333, is threaded onto the threaded end cap 326. A proximal end 334 of the second release wire 316 is secured to the second release wire handle 331. A first release wire handle 335 has a threaded portion 336 that is releasably threaded onto the proximal threaded portion 333 of the second release wire handle 331. A proximal end 337 of the first release wire 312 is secured to the first release wire handle 335.

Once the outer tubular member 53 has been proximally retracted, belts 21-23 can be released. This configuration allows the operator of the delivery system 300 to first disengage and proximally retract the first release wire handle 335 so as to first release the second distal self-expanding member 33 without releasing or otherwise disturbing the constrained state of the first distal self-expanding member 32 or the proximal self-expanding member 31. Once the second distal self-expanding member 33 has been deployed or released, the endovascular graft 11 may be axially moved or repositioned to allow the operator to adjust the position of the graft 11 for final deployment.

This is advantageous, particularly in the treatment of abdominal aortic aneurysms, because it allows the physician to accurately place graft 11 into position. In many cases, it is desirable for the physician to place the graft 11 such that the distal end of the tubular body portion 205 of the graft is just below the renal arteries 273, shown in FIG. 9, to prevent occlusion of the renal arteries by the tubular body portion 205. If a self-expanding member, such as self-expanding member 32 is radiopaque and the delivery procedure is performed using fluoroscopic imaging, adjustment of the position of the graft after release of self-expanding member is readily achievable. Because self-expanding member 32 is immediately adjacent the distal end of the tubular body portion 205 of the graft 11, the ability to visualize and reposition the self-expanding member 32 is particularly useful in order to position the distal end of the tubular body portion 205 just below the renal arteries without occluding the renal arteries, if such positioning is indicated for the patient being treated.

Thereafter, the second release wire handle 331 may be unscrewed or otherwise released from the end cap 326 and proximally, retracted so as to first release the first distal belt end loops 81 and then the proximal belt end loops 83. Of course, the position of the graft 11 may still be adjustable even with both distal self-expanding members 32 and 33 deployed, depending on the particular configuration of the graft 11 and the self-expanding members 32 and 33. The release of the belts 21, 22 and 23 is the same or similar to that of the belts of the example of FIG. 1 and occurs when the distal end of the release wires 313 and 317 which lock the end loops 81-83 together is proximally retracted past the end loops 81-83 of the belts 21-23 which are constrained.

Once the self-expanding members 31-33 of the endovascular graft 11 have been deployed or released, and the graft 11 is in a desired location, the graft 11 can then be inflated by injection of an inflation material (not shown) into the injection port 338 on a second side arm 341 of the proximal adapter 323. The inflation material is introduced or injected directly into an inner lumen 212 of the inner tubular member 207, as shown in FIG. 17, and travels distally between an inside surface 342 of the inner tubular member 207, outside surface 343 of the release wire tube 318 and outside surface 216 of the guidewire tube 17. This allows the inflation material, which can be highly viscous, to flow through the cross sectional area between the inside surface 342 of the inner tubular member 207 and the outside surfaces 216 and 343 of the release wire tube 318 and guidewire tube 17. This cross sectional area is large relative to the cross sectional area of the inner lumen of the inflation tube 211 of the example of FIG. 1. This results in more rapid flow of inflation material to the inflatable cuffs 28 and 30 and channels 284 of the endovascular graft 11 and decreases inflation time.

Once the inflation material, which is travelling distally in the delivery system 300 during inflation, reaches the potted portion 213 of the distal section 303 of the delivery system, it then enters and flows through a lumen 344, as shown in FIG. 16, at the proximal end 307 of the inflation tube 305 and into the inflation port 283 of the graft 11. Upon inflation of the graft 11 with an inflation material, a release device, such as retention wire 285 can be retracted or otherwise activated so as to de-couple the inflation tube 305 from the inflation port 283 of the endovascular graft 11.

A proximal end 36 of the guidewire tube 17 is secured within a central arm 345 of the proximal adapter 323 that has a potted section 346. A seal 349 is disposed on a proximal end 347 of the central arm 345 for sealing around the guidewire 18 and preventing a backflow of blood around the guidewire. A hemostasis adapter (not shown) can be coupled to the proximal end 347 of the central arm 345 in order to introduce fluids through the guidewire tube lumen 348, as shown in FIG. 15, around an outside surface of the guidewire 18. The potted section 346 of the central arm 345 prevents any fluids injected through the hemostatis adapter from passing into the inflation material lumen 351 within the proximal adapter 323 or the inner tubular member 207.

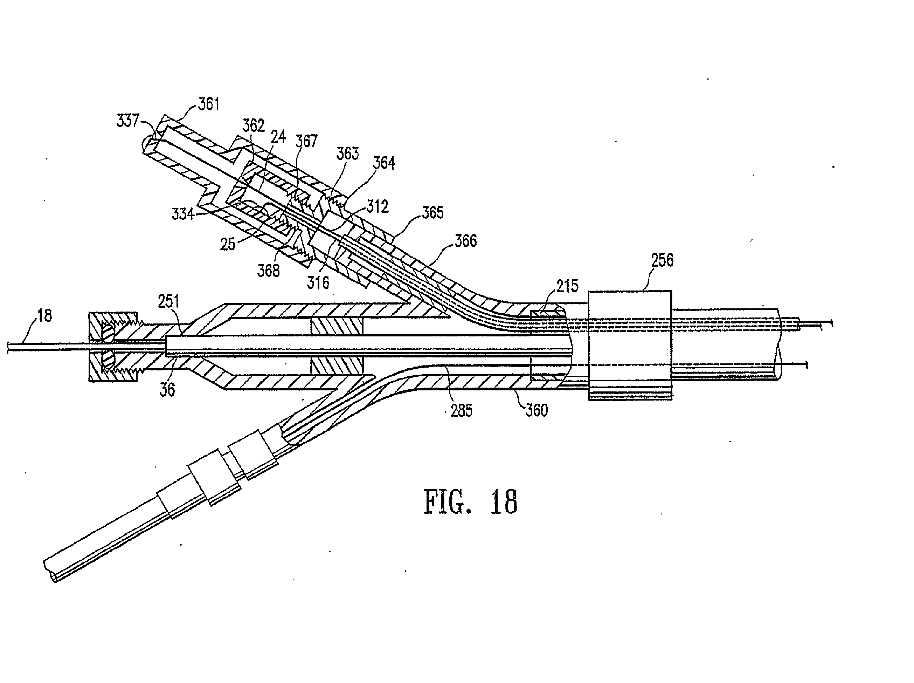

FIG. 18 illustrates an alternative embodiment to the proximal adapters 42 and 323 used in the example of FIG. 1 and in the embodiment of the invention of FIG. 14. In this embodiment, the proximal adapter 360 has a first release wire handle 361 and a second release wire handle 362 which are in a nested configuration. The proximal end 334 of the second release wire 316 is secured to the second release wire handle 362. The proximal end 337 of the first release wire 312 is secured to the first release wire handle 361. This configuration prevents the operator from inadvertently deploying or activating the second release wire 316 prior to deployment or activation of the first release wire 312 which could result in an undesirable endovascular graft deployment sequence.

In use, the operator first unscrews or otherwise detaches a threaded portion 363 of the first release wire handle 361 from an outer threaded portion 364 of a first side arm end cap 365 of a first side arm 366. The first release wire handle 361 is then proximally retracted which releases the end loops 82 of the second distal belt 22 as discussed above with regard to the embodiment of the invention shown in FIG. 14.

Once the first release wire handle 361 is removed from the first side arm end cap 365, the second release wire handle 362 is exposed and accessible to the operator of the delivery system. A threaded portion 367 of the second release wire handle 362 can then be unscrewed or otherwise detached from an inner threaded portion 368 of the first side arm end cap 365. The second release wire handle 362 can then be retracted proximally so as to sequentially deploy the first distal belt 21 and self-expanding member 32 and proximal belt 23 and proximal self-expanding member 31, respectively. The other functions and features of the proximal adapter 360 can be the same or similar to those of the proximal adapters 42 and 323 shown in FIG. 1 and FIG. 17 and discussed above.

Optionally, this embodiment may comprise reverse or oppositely threaded portions, 363 and 367 respectively, of the first and second release wire handles 361 and 362. Thus, for instance, a counter-clockwise motion may be required to unthread threaded portion 363 of the first release wire handle 361 from the outer threaded portion 364, while a clockwise motion is in contrast required to unthread threaded portion 367 of the second release wire handle 367 from the inner threaded portion 368. This feature serves as a check on the overzealous operator who might otherwise prematurely unscrew or detach the threaded portion 367 of the second release wire handle 362 by unscrewing in the same direction as required to release the threaded portion 363 of the first release wire handle 361.

In another aspect of the invention, a delivery system 400 for delivery and deployment of a bifurcated intracorporeal device, specifically, an embodiment of the invention directed to delivery and deployment of a bifurcated endovascular graft or stent is contemplated. As with all the delivery systems disclosed herein, the delivery system 400 for a bifurcated device is configured for delivery and deployment a wide variety of intracorporeal devices. Although the focus of the specific embodiments are directed to systems for delivery of endovascular grafts or stent grafts, embodiments of the delivery systems disclosed herein can are also suitable for delivery of intravascular filters, stents, including coronary stents, other types of shunts for intracorporeal channels, aneurysm or vessel occluding devices and the like.

The structure, materials and dimensions of the delivery system 400 for bifurcated devices can be the same or similar to the structure, materials and dimensions of the delivery systems discussed above. In addition, the structure, materials and dimensions of bifurcated grafts contemplated herein can have structure, materials and dimensions similar to those of grafts having a primarily tubular shape discussed above.

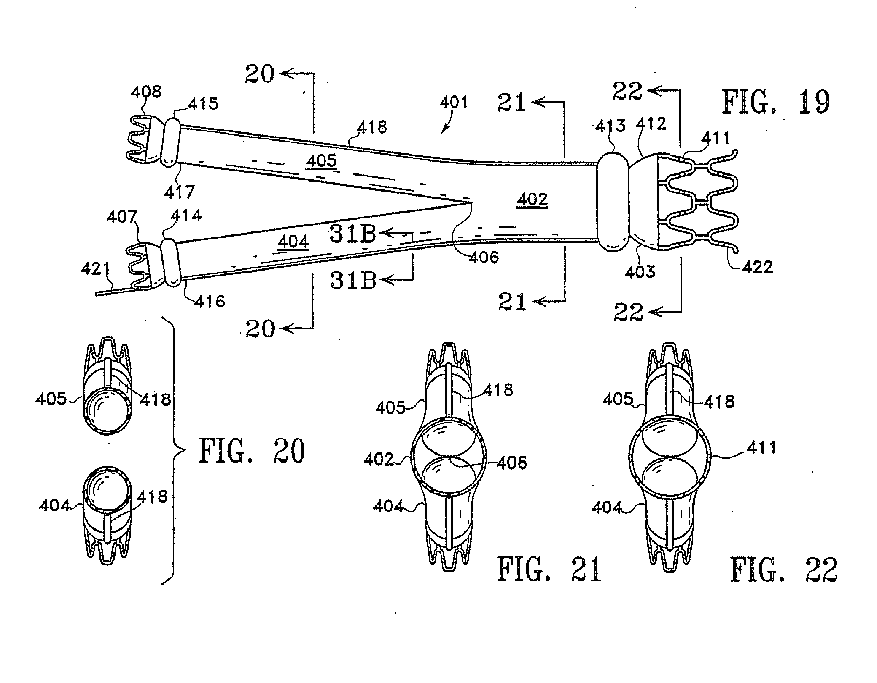

FIGS. 19-22 illustrate an embodiment of an expandable intracorporeal device in the form of a bifurcated stent-graft 401. This embodiment includes a main body portion 402 at a distal end 403 of the graft 401 that has a generally tubular cross-sectional profile when the graft takes on an expanded or deployed configuration. An ipsilateral leg 404 and contralateral leg 405 (short leg), both having a substantially tubular configuration when expanded or deployed, branch from the main body portion 402 at bifurcation 406 and extend in a proximal direction from the bifurcation 406. The ipsilateral leg 404 terminates proximally with a proximal self-expanding member 407 and the contralateral leg 405 terminates proximally with a proximal self-expanding member 408.

The main body portion 402 of the graft may have a transverse dimension when in an expanded or deployed state ranging from about 10 mm to about 40 mm, specifically from about 15 mm to about 30 mm. The legs 404 and 405 of the graft 401 may have a transverse dimension when in an expanded or deployed state ranging from about 5 mm to about 16 mm, specifically from about 8 mm to about 14 mm. The main body portion 402 of the graft 401 may have a length ranging from about 2 cm to about 12 cm, specifically from about 4 cm to about 8 cm.

A second distal self-expanding member 411 is disposed at a distal end 412 of the main body portion 402 of the graft 401 as with the graft embodiments previously discussed. Also, as with other endovascular graft embodiments discussed herein, the graft 401 may have inflatable channels and inflatable cuffs that serve, among other functions, to provide support for the graft 401 and the inflatable channels and cuffs can have configurations which are the same or similar to those inflatable channels and cuffs of other graft embodiments discussed herein, as well as other configurations. A distal inflatable cuff 413 is disposed at the distal end 412 of the main body portion 402. Proximal inflatable cuffs 414 and 415 are disposed on a proximal end 416 of the ipsilateral leg 404 and a proximal end 417 of the contralateral leg 405 respectively. Inflatable channels 418 are fluid tight conduits which connect the inflatable cuffs 413, 414 and 415. The inflatable channels 418 and inflatable cuffs 413 and 414 are inflatable through an inflation port 421 that may be disposed at or near the proximal end 416 of the ipsilateral leg 404. The inflation port 421 may also be disposed at or near the proximal end 417 of the contralateral leg 405, or it may be disposed on other portions of the device as necessary. Generally, the structure and the materials used in the graft 401 (both the graft portion and the self-expanding members) can be similar to the structure and materials of the other graft embodiments discussed above. In one particular embodiment, the main body portion and legs of the graft are made of expanded polytetrafluoroethylene (ePTFE) and the self-expanding members are made of nickel titanium, stainless steel or the like.

A first distal self-expanding member 422 is secured to the second distal self-expanding member 411 as shown in FIG. 19. This configuration is similar to that of endovascular graft 11 illustrate in FIGS. 1-6B, 10-12 and 14-16 above. Graft 11 has first and second distal self-expanding members 32 and 33 that may be deployed in any desired sequence. In a particular embodiment having first and second distal self-expanding members, it may be desirable to first deploy the second distal self-expanding member 33 prior to deploying the first distal self-expanding member 32. As discussed above, deploying the second distal self-expanding member 33 first may allow the operator to accurately adjust the axial position of the graft in the body lumen or vessel to within one to several millimeters before deploying the first distal self-expanding member 32. Using this technique, deployment of the second distal self-expanding member 33 alone provides sufficient resistance to axial displacement of the graft 11 for the graft position to be maintained in normal blood flow, but still allows deliberate axial displacement by the operator to achieve a desired axial position. This may be particularly important if tissue-penetrating members are included on the distal-most or first distal self-expanding member 32. If such tissue penetrating members are used on the first distal self-expanding member 32, axial movement may be difficult or even impossible once this member 32 is deployed without risking damage to the body lumen or vessel. As such, accurate axial placement of the graft 11 prior to deployment of the first distal self-expanding member 32 can be critical.

In addition, although not shown in the figures, this graft embodiment 401 may include two or more proximal self-expanding members disposed on one or both of the ipsilateral leg 404 and/or contralateral leg 405. These self-expanding members may have a configuration similar to that of the first and second distal self-expanding members 411 and 422

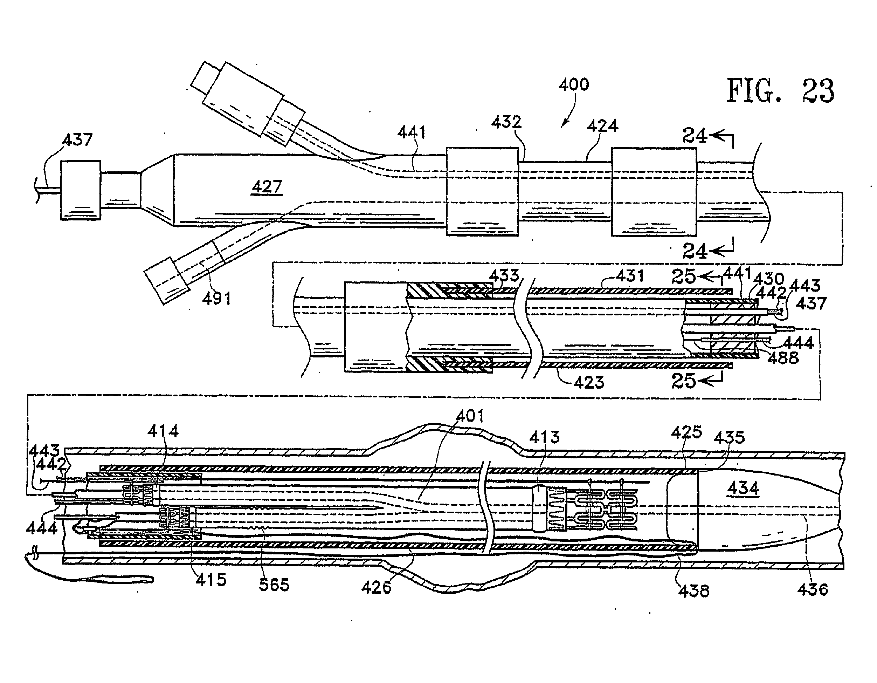

FIGS. 23-32 illustrate an embodiment, which is not part of the invention, of a delivery system 400 having features of the invention. FIG. 23 shows delivery system 400 in partial section having an elongate shaft 423 with a proximal end 424, a distal end 425 and a distal section 426. A proximal adapter 427 is disposed at the proximal end 424 of the elongate shaft 423 and houses the controls that enable the operator to manipulate elements at the distal section 426 of delivery system 400 to release and deploy the graft 401, including inflating the graft channels 418 and cuffs 413, 414 and 415. The elongate shaft 423 has an inner tubular member 430 and an outer tubular member 431 disposed about the inner tubular member 430. The outer tubular member 431 is generally configured to slide in an axial direction over the inner tubular member 430. A proximal end 432 of the inner tubular member 430 is secured to or disposed on the proximal adapter 427. The inner and outer tubular members 430 and 431 may be made of polymeric materials, e.g., polyimides, polyester elastomers (HYTREL®), or polyether block amides (PEBAX®), and other thermoplastics and polymers. The outside diameter of the outer tubular member 431 may range from about 0,254 cm to about 1,016 cm (0.1 inch to about 0.4 inch); specifically from about 0,381 cm to about 0,508 cm (0.15 inch to about 0.20 inch). The wall thickness of the outer tubular member 431 may range from about 0,005 cm to about 0,038 cm (0.002 inch to about 0.015 inch), specifically from about 0,01 cm to about 0,02 cm (0.004 inch to about 0.008 inch). The proximal adapter 427 is generally fabricated from a polymeric material such as polyethylene, acetal resins (DELRIN®), etc., but can also be made from any other suitable material.

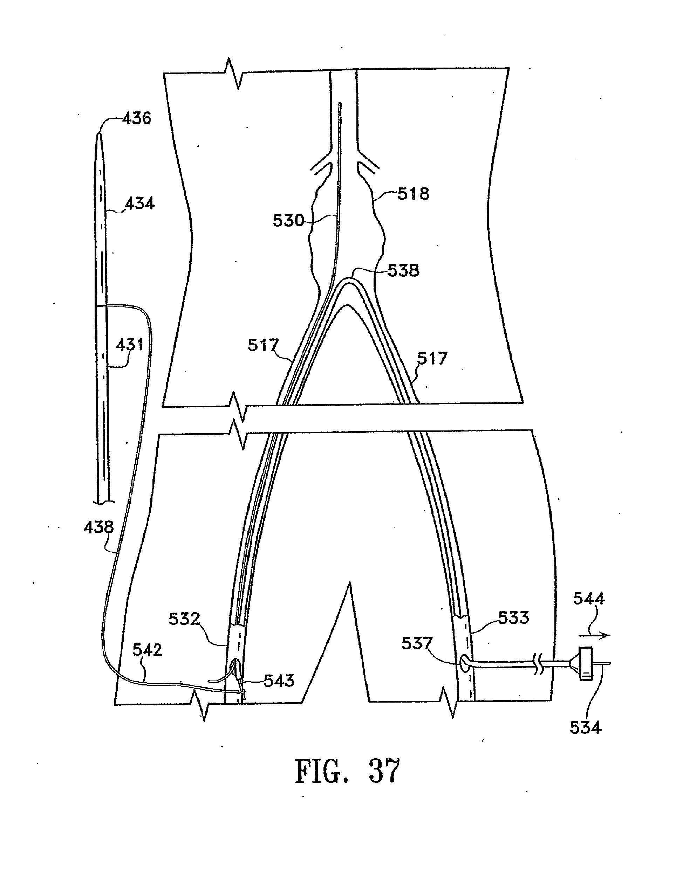

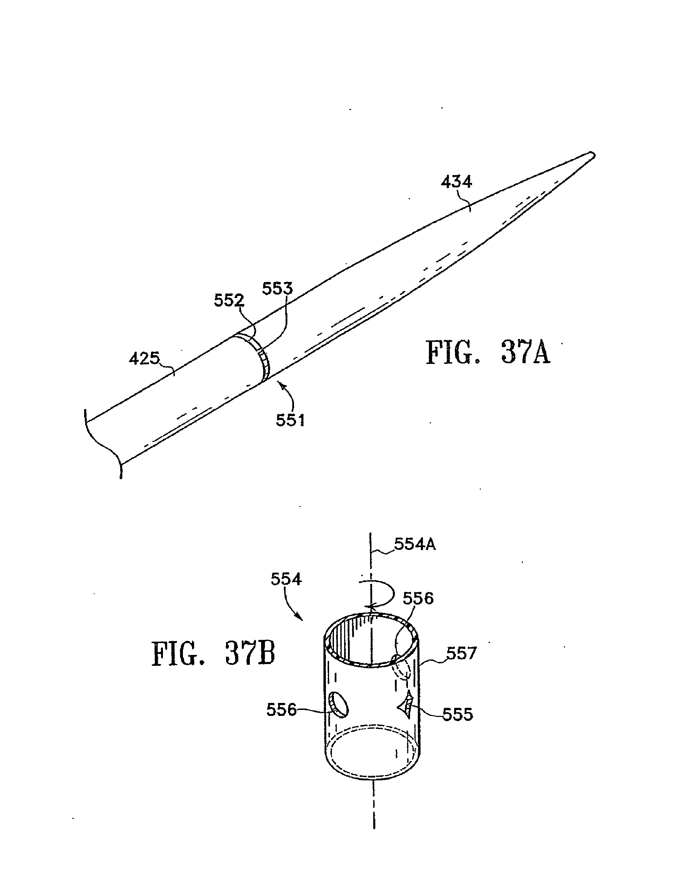

Bifurcated stent graft 401 is shown in FIGS. 23-28 disposed within the distal section 426 of the elongate shaft 423 in a constrained configuration. The outer tubular member 431 is disposed about the graft 401 in the constrained state but can be retracted proximally so as to expose the constrained graft 401 by proximally retracting a proximal end 433 of the outer tubular member 431. As illustrated more fully in FIG. 37, a distal nosepiece 434 may be disposed on a distal end 435 of the outer tubular member 431 and forms a smooth tapered transition from a guidewire tube 436 to the outer tubular member 431. This transition helps to facilitate the tracking of the outer tubular member 431 over a guidewire 437. In order to form this smooth transition, the nosepiece 434 may have a length to major diameter ratio ranging from about 3:1 to about 10:1 (the "major diameter" being defined as the largest diameter of the nosepiece). The outer tubular member 431 is not typically permanently secured to the nosepiece 434 and may be retractable from the nosepiece 434 during the deployment sequence. A secondary release cable 438 extends from an opening in the distal section of the elongate shaft. Nosepiece 434 may be grooved to receive secondary release cable 438 if desired.

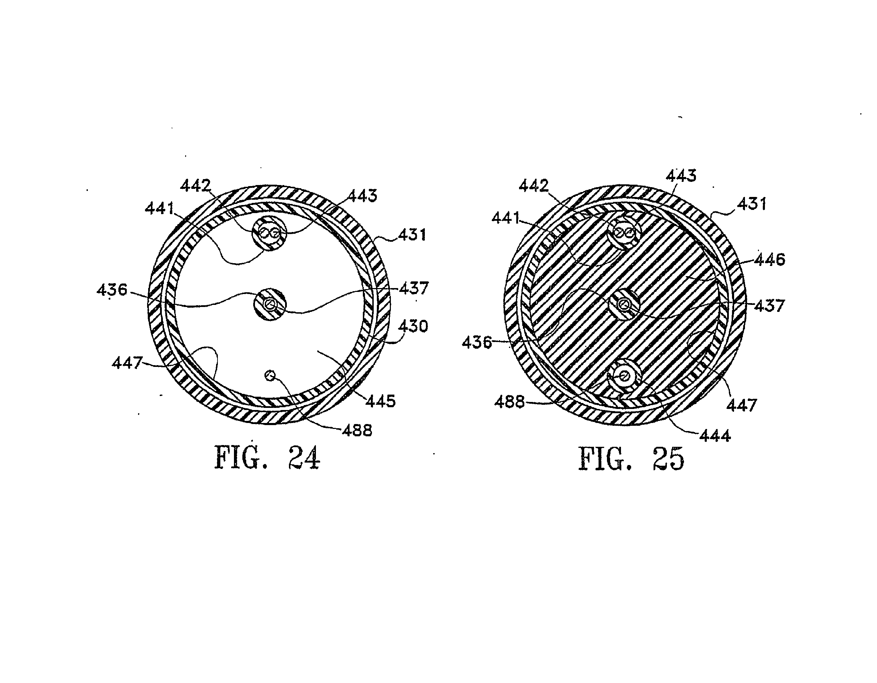

FIG. 24 shows the inner tubular member 430 disposed within the outer tubular member 431 and the guidewire tube 436 disposed within the inner tubular member 430. The guidewire tube 436 may be made from polymeric materials such as polyimide, polyethylene, polyetheretherketones (PEEK™), or other suitable polymers, and may have an outside diameter ranging from about 0,05 cm to about 0,2 cm (0.02 inch to about 0.08 inch), specifically about 0,09 cm to about 0,14 cm (0.035 inch to about 0.055 inch). The guidewire tube 436 wall thickness may range from about 0,005 cm to about 0,06 cm (0.002 inch to about 0.025 inch), specifically from about 0,01 cm to about 0,02 cm (0.004 inch to about 0.010 inch).

A release member tube in the form of a release wire tube 441 is disposed about a distal primary release member in the form of a distal primary release wire 442. The release wire tube 441 is also disposed about a proximal primary release member in the form of a proximal primary release wire 443. Both the release member tube 441 and an inflation tube 444 are disposed within an inner lumen 445 of the inner tubular member 430. The outside diameter of the release wire tube 441 may range from about 0,025 cm to about 0,027 cm (0.01 inch to about 0.05 inch), specifically about 0,038 cm to about 0,06 cm (0.015 inch to about 0.025 inch). The wall thickness of the release wire tube 441 may range from about 0,0025 cm to about 0,015 cm (0.001 inch to about 0.006 inch), specifically from about 0,05 cm to about 0,01 cm (0.002 inch to about 0.004 inch).

The outside diameter of the inflation tube 444 may range from about 0,05 cm to about 0,25 cm (0.02 inch to about 0.10 inch); specifically from about 0,1 cm to about 0,2 cm (0.04 inch to about 0.08 inch). The inflation tube 444 wall thickness may range from about 0,005 cm to 0,06 cm (0.002 inch to about 0.025 inch); specifically from about 0,007 cm to 0,025 cm (0.003 inch to about 0.010 inch).

In FIG. 25, a potted portion 446 is disposed between an inner surface 447 of a distal end 448 of the inner tubular member 430, the release wire tube 441, the guidewire tube 436 and the inflation tube 444. The potted portion 446 seals the inner lumen 445 of the inner tubular member 430 from bodily fluids that are exposed to the constrained graft 401 and potted portion 446 once the outer tubular member 431 is proximally retracted. The potted portion 446 may be made from adhesives, thermoforming plastics, epoxy, metals, or any other suitable potting material. Alternatively, a molded or machined plug may be bonded or affixed to the distal end of the inner tubular member, with lumens to accommodate the passage of tubes 441, 436 and 444.

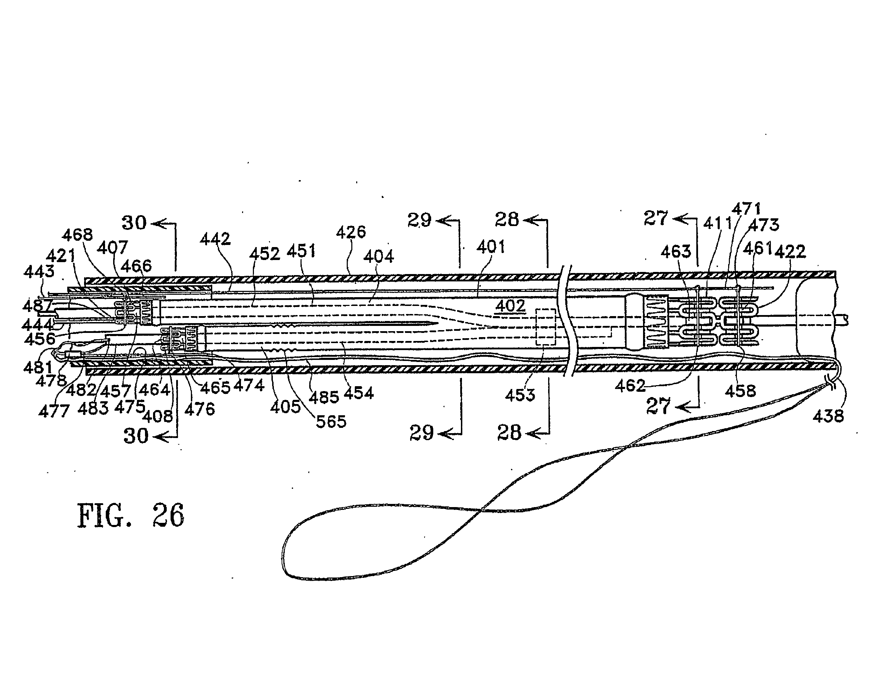

A more detailed view of the distal section 426 of the elongate shaft 423 is shown in partial section in FIGS. 26-30. A distal section 451 of the guidewire tube 436 serves as a primary belt support member 452 and is disposed within the main body portion 402 and ipsilateral leg 404 of the graft 401. Alternatively, the primary belt support member 452 may be disposed adjacent the graft main body portion 402 and ipsilateral leg 404. A secondary belt support member housing 453 is secured to the primary belt support member 452. An additional length of guidewire tube or other elongate member serving as a secondary belt support member 454 is slidably disposed within an appropriately configured lumen 455 of the housing 453. The secondary belt support member 454 is shown in FIG. 26 disposed within the graft main body portion 402 and contralateral leg 405; however, the secondary belt support member 454 may also be disposed adjacent the contralateral leg 405, regardless of whether the primary belt support member 452 is disposed adjacent or within the main body portion 402 and ipsilateral leg 404.

The secondary belt support member housing lumen 455 and secondary support member 454 cross sections may be keyed, singly or in combination, to allow relative sliding motion without relative rotation motion and therefore limit any twisting of the secondary support member 454 and the contralateral leg 405. The secondary belt support member 454 may be made from alloys such as nickel titanium, stainless steel, or polymeric materials such as polyimide and can have an outside transverse dimension ranging from about 0,025 cm to 0,15 cm (0.01 inch to about 0.06 inch).

A proximal primary belt 456 is shown in FIG 26 disposed about and radially constraining the proximal self-expanding member 407 of the ipsilateral leg 404. This proximal self-expanding member 407 in turn is disposed about a bushing 457 that is shown as cylindrical in form, but which may have other configurations as well. The bushing 457 is secured to the primary belt support member 452 adjacent the proximal self-expanding member 407 of the ipsilateral leg 404.

A first distal primary belt 458 is disposed about and radially constraining the first distal self-expanding member 422, which itself is disposed about a cylindrical bushing 461.

A second distal primary belt 462 is disposed about and radially constraining the second distal self-expanding member 411 and the second distal self-expanding member 411 is disposed about a cylindrical bushing 463.

A secondary belt 464 is shown disposed about and radially constraining the proximal self-expanding member 408 of the contralateral leg 405. This proximal self-expanding member 408 is disposed about a bushing 465 that is cylindrical in shape.

As with the other embodiments or examples disclosed herein of the present invention, the belts 456, 458, 462 and 464 are typically made from nickel titanium, an alloy that is capable of exhibiting a unique combination of high strain without elastic deformation, high strength and biocompatability. However, any other suitable materials may be used including other metallic alloys such as stainless steel, high strength fibers such as carbon, KEVLAR®, polytetrafluoroethylene (PTFE), polyimide, or the like. The outer transverse dimension or diameter of the belts 456, 458, 462 and 464 can be from about 0,005 cm to about 0,03 cm (0.002 inch to about 0.012 inch); specifically about 0,010 cm to about 0,01 cm (0.004 inch to about 0.007 inch).

A distal portion 466 of the proximal primary release wire 443 is disposed within end loops 468 of the proximal primary belt 456 so as to releasably secure the proximal self-expanding member 407 of the ipsilateral leg 404 in a constrained state. The proximal primary belt 456 may be disposed about the self-expanding member 407 in a hoop-like configuration. The proximal self-expanding member 407 exerts outward radial pressure on the releasably secured belt 456. The primary proximal release wire 443 is axially moveable within the end loops 468 of the proximal primary belt 456 to allow for release of the belt by proximal retraction of the primary proximal release wire 443 in the same manner as described above with respect to other embodiments or examples disclosed herein of the present invention.

Likewise, a distal portion 471 of the distal primary release wire 442 is disposed within end loops 472 of the second distal primary belt 462 that radially constrains the second distal self-expanding member 411. The second distal primary belt 462 is formed in a hoop configuration about the second distal self-expanding member 411 and the second distal self-expanding member 411 exerts outward radial force on the second distal primary belt 462. The distal primary release wire 442 is axially moveable within the end loops 472 of the second distal primary belt 462 to allow for release of the radial constraint as discussed above with respect to the proximal primary release wire 443 and as discussed above for other embodiments or examples disclosed herein of the present invention. The distal portion 471 of the distal primary release wire 442 is also disposed within end loops 473 of the first distal primary belt 458 and radially constrains the first distal self-expanding member 422 in a similar fashion.

Although the distal primary release wire 442 and proximal primary release wire 443 are shown as two separate components, the release wires 442 and 443 could be combined into a shingle release member, such as the branched release wire 150 shown in FIG. 7I above. A branched release wire is capable of releasing multiple belts in a desired sequence by proper configuration of the lengths of the various branches of the wire. The relative amount of the release wire extending beyond the looped ends of the belt as indicated by reference numeral 156 in FIG. 7I controls the timing of the release of the belts. Alternatively, a single release wire may engage both distal and proximal primary belts 456, 458 and 462. As this single release wire 150 is moved proximally, the first distal primary belt 458 is first released, followed by the release of the second distal primary belt 462 and then release of the proximal primary belt 456.

A distal portion 474 of a secondary release member in the form of a secondary release wire 475 is disposed within end loops 476 of a secondary belt 464 that radially constrains the proximal self-expanding member 408 of the contralateral leg 405. The proximal self-expanding member 408 of the contralateral leg 405 exerts outward radial force on the secondary belt 464 when the self-expanding member 408 is in a constrained configuration. The secondary release wire 475 is axially moveable within the end loops 476 of the secondary belt 464.

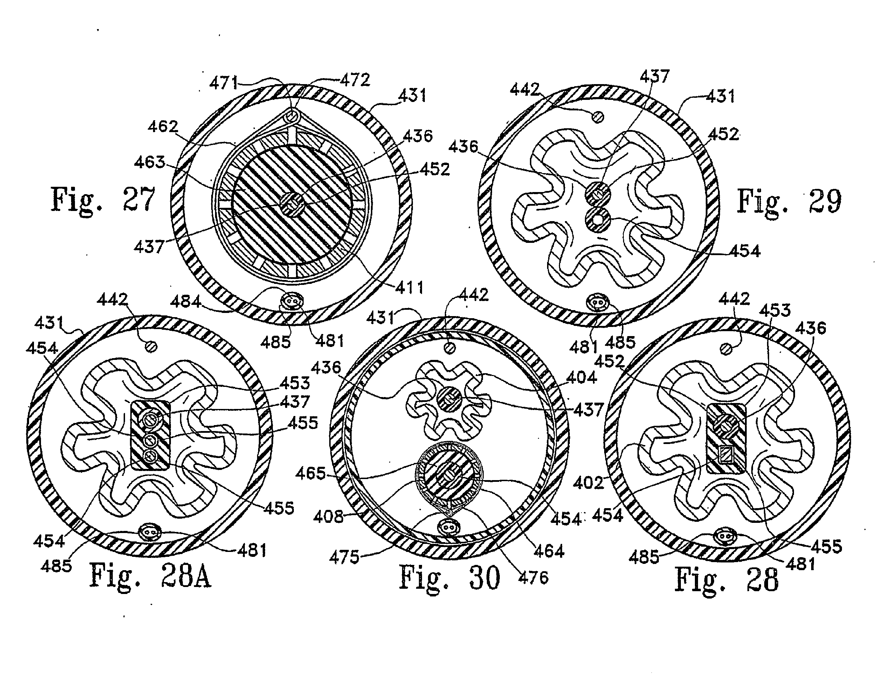

A proximal end 477 of the secondary release wire 475 is secured to an actuator hub 478. A release strand 481 is secured to the actuator hub 478 and is attached to the secondary belt support member 454, and is shown by way of example in the embodiment of FIG. 26 as being looped through a hole 482 in the proximal end 483 of the secondary belt support member 454. Both portions of the release strand 481 that are looped through the proximal end 483 of the secondary belt support member 454 pass into an inner lumen 484 of a release strand tube 485 as seen in FIG. 27. The release strand tube 485 passes through an aperture 486 in the distal end 435 of the outer tubular member 431. Release strand 481 may comprise any filamentary thread or wire, metallic, polymeric, or otherwise, suitable for manipulation as will be herein described. It also may be braided or twisted if desired. The release strand 481 may be made of a filamentary thread of ePTFE.

As discussed above with respect to other embodiments or examples described herein, the release wires 442, 443 and 475 are generally made from a biocompatible high strength alloy such as stainless steel, but can also be made from any other suitable materials. Examples include other metallic alloys such as nickel titanium, non-metallic fibers such as carbon, polymeric materials, composites thereof, and the like. As discussed above, the diameter and stiffness of the release wires 442, 443 and 475 can be important with respect to the diameter and stiffness of the belts 456, 458, 462 and 464.

The configuration of the end loops 468, 472, 473 and 476 of the belts 456, 458, 462 and 464 may vary to suit the particular example of the delivery system 400 and device to be delivered. For example, FIGS. 7C-7H illustrate a variety of belt and end loop configurations that may be suitable for delivery systems for bifurcated devices. Referring to FIG. 7C, belts 112 and 114 are shown having a twisted configuration that has a tendency to reduce snagging or entanglement of the belts 112 and 114 after deployment and release of the belts from a constrained configuration. In addition, FIG. 7C illustrates an angle $\alpha$ that belts 112 and 114 make with respect to line 125. In one example, belts 112 and 114 would be substantially parallel to each other when in an unconstrained state such that this angle is approximately ninety degrees. It may also be desirable to use belts that have end loops that have different cross sectional areas (or transverse dimensions). For example, FIG. 7E shows end loops 81' and 81" constrained by release wire 24. We have found that, depending on the transverse dimension and material of loop 81' disposed within loop 8 1 ", elastic deformation of loop 81' can hinder the release process when release wire 24 is proximally retracted. Therefore, it may be desirable to make loop 81' from a material that is substantially smaller in cross sectional area or transverse dimension that that of loop 81 ". In a particular example, loop 81' is made from nickel titanium wire having a diameter of about 0,007 cm to about 0,012 cm (0.003 to about 0.005 inch), and loop 81" is made from the same material having a diameter ranging from about 0,012 cm to about 0,018 cm (0.005 to about 0.007 inch).

Inflation port 421 extends proximally from the proximal end 416 of the ipsilateral leg 404 of the graft 401. The inflation port 421 is coupled to a distal end 487 of the inflation tube 444 by a retention mechanism, such as a retention wire 488, the operation of which can be the same or similar to like embodiments of retention wire 285 discussed above. Typically, the retention wire 488 extends from the inflation port 421 proximally to the proximal adapter 427 of delivery system 400. The distal end 487 of the inflation tube 444 can be disengaged from the inflation port 421 by pulling on a proximal end 491 of retention wire 488, as shown in FIGS. 23, 26 and 31. The retention wire 488 may be a small diameter wire made from a material such as a polymer, stainless steel, nickel titanium, other alloy or metal, or composite; in a particular embodiment of the invention, retention wire 488 may be a spring formed of a variety of suitable spring materials. Alternatively, the retention wire 488 may have a braided or stranded configuration.

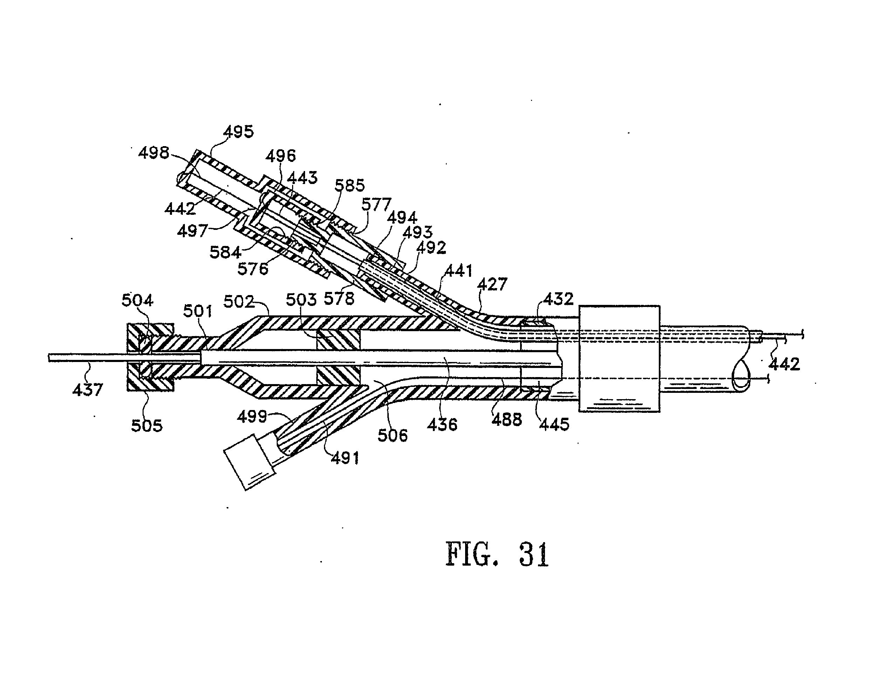

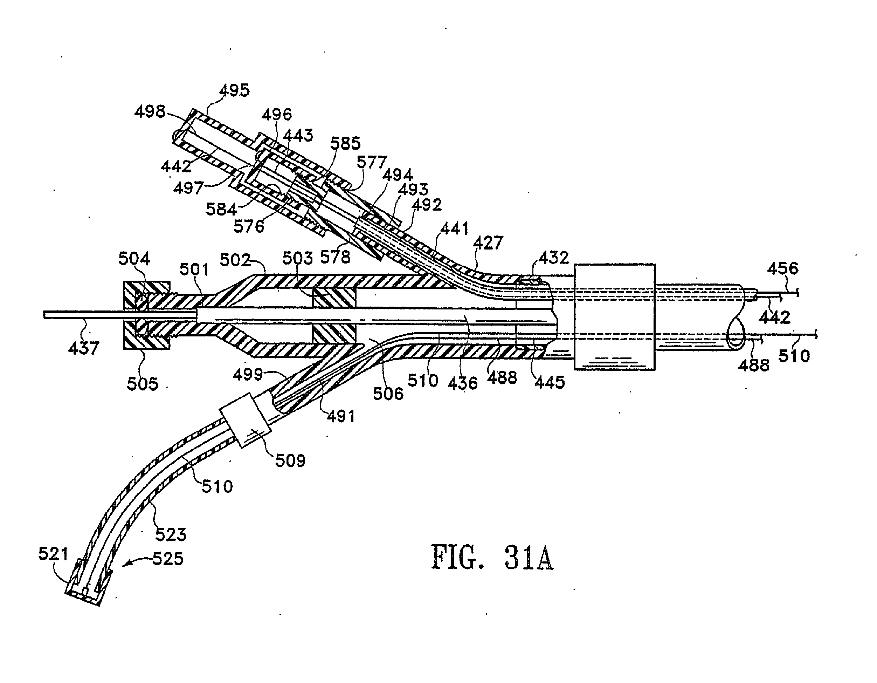

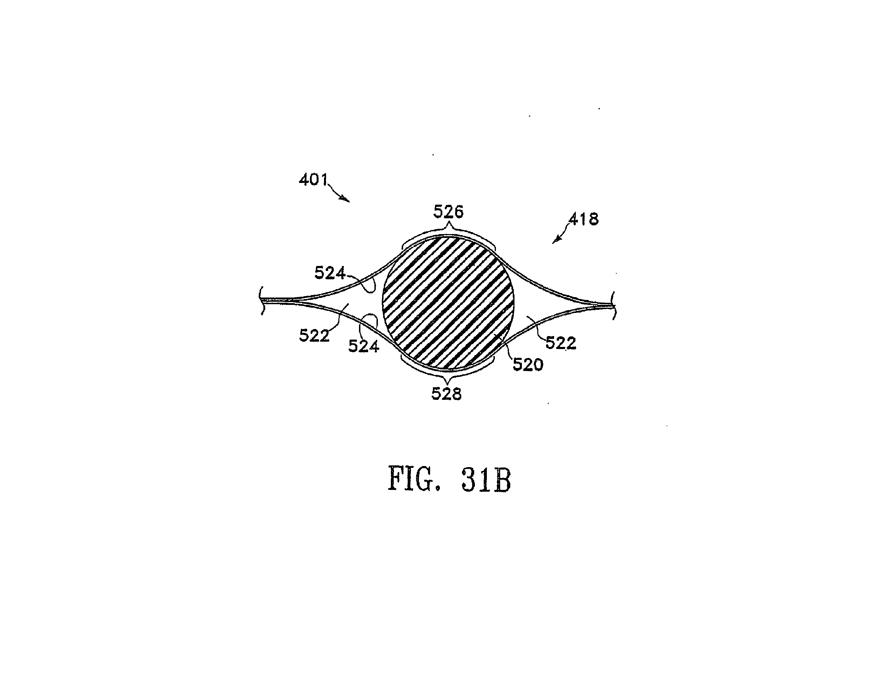

FIG. 31 illustrates proximal adapter 427 which is suitable for use with embodiments and examples disclosed herein of the present invention. The proximal adapter 427 houses the proximal termination of the primary release wires 442 and 443, guidewire tube 436, retention wire 488 and release wire tube 441. The proximal adapter 427 has a first side arm 492 with an inner lumen 493 that secures the proximal end 494 of the release wire tube 441 and second side arm 499 having an inner lumen in fluid communication with inflation material lumen 506 that houses proximal end 491 of retention wire 488. The proximal adapter 427 has a distal primary release wire handle 495 and a proximal primary release wire handle 496 that are disposed in a nested configuration on the first side arm 492. A proximal end 497 of the proximal primary release wire 443 is secured to the proximal primary release-wire handle 496. A proximal end 498 of the distal primary release wire 442 is secured to the distal primary release wire handle 495. This configuration prevents the operator from inadvertently deploying or activating the proximal primary release wire 443 prior to deployment or activation of the distal primary release wire 442 which could result in an undesirable graft 401 deployment sequence.

A proximal end 501 of the guidewire tube 436 is secured within a central arm 502 of the proximal adapter 427 that has a potted section 503. A seal 504 may be disposed on a proximal end 505 of the central arm 502 for sealing around the guidewire lumen and preventing a backflow of fluid. The potted section 503 of the central arm 502 prevents any injected fluids from passing into the inflation material lumen 506 within the proximal adapter 427 or the inner tubular member 430. The other functions and features of the proximal adapter 427 may be the same or similar to those of the proximal adapters 42 and 323 shown in FIG. 1 and FIG. 17 and discussed above.

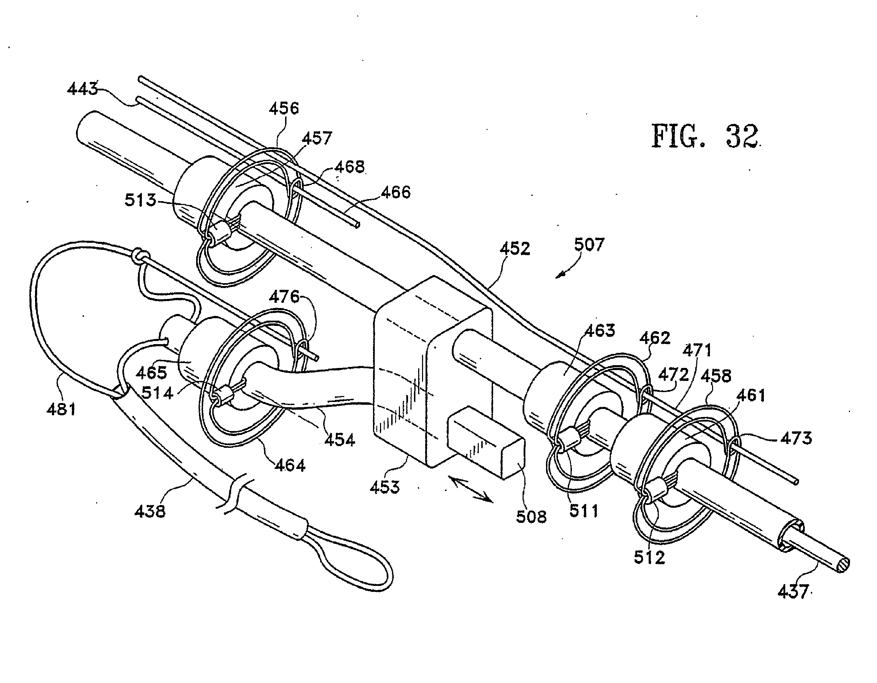

FIG. 32 illustrates a belt support member assembly 507 of the delivery system 400. The distal end 508 of the secondary belt support member 454 is slidingly disposed within the secondary belt support member housing 453 that is secured to the primary belt support member 452. The second distal primary belt 462 is secured to the primary belt support member 452 (which in this example is the guidewire tube 436) and extends radially therefrom through an optional second distal primary standoff tube 511. Similar optional first distal primary standoff tube 512, proximal primary standoff tube 513 and optional secondary standoff tube 514 are disposed on the first distal primary belt 458, proximal primary belt 456 and secondary belt 464, respectively.

In general, the various features and components (including, e.g., details of various embodiments of the release wires, the self-expanding members, belts, inflation port and tube, guidewire tube, standoff tubes, proximal adapter and its associated components, the materials and dimensions for each of the various components, etc.) as discussed herein with respect to those examples of FIGS. 1-18 may be used in the bifurcated examples of the present invention as discussed herein and as illustrated in FIGS. 19-32.

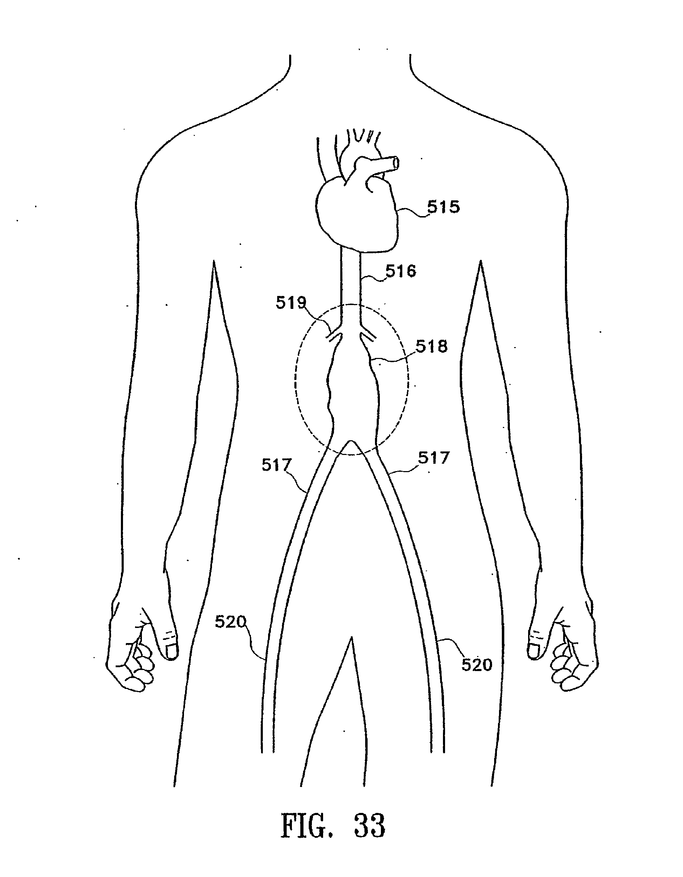

In use, the delivery system 400 for delivery of a bifurcated intracorporeal device, specifically, a bifurcated graft 401, can be operated in a similar fashion to the delivery systems discussed above. FIG. 33 illustrates generally the anatomy of a patient's heart 515, aorta 516 and iliac arteries 517. The aorta extends from the heart 515 and descends into the abdomen of the patient's body. An aneurysm 518 is disposed in the aorta 516 just below the renal arteries 519. The aorta 516 branches into the right and left iliac arteries 517 below the aneurysm, which then become the femoral arteries 520.

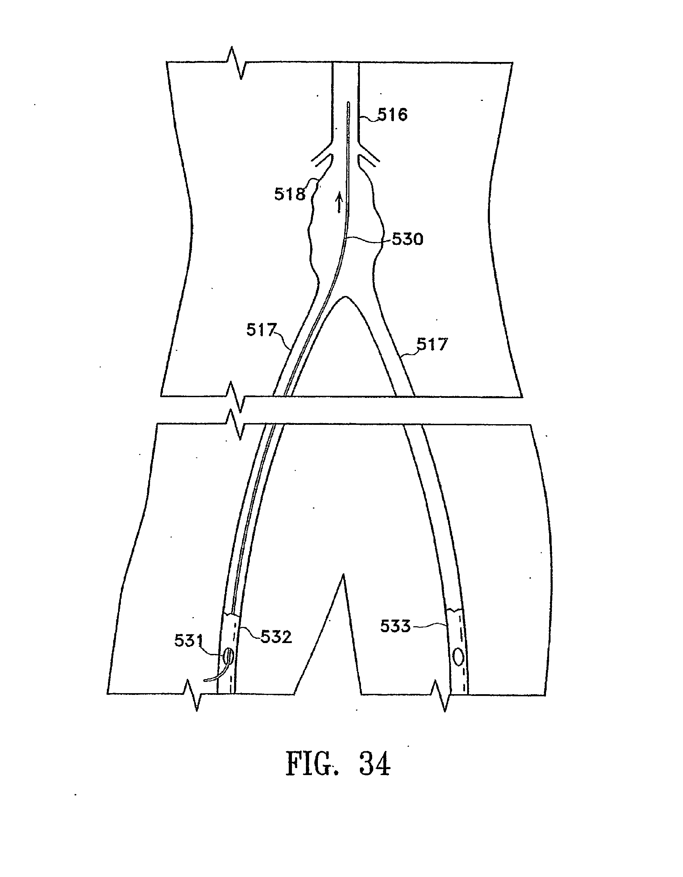

One disclosed delivery procedure begins with delivery of a first guidewire 530 into an access hole 531 in a femoral artery, the right femoral artery 532 for the procedure depicted in FIG. 34, and advanced distally through the iliac artery 517 and into the patient's aorta 516. Access into the femoral artery 532 is generally accomplished with a standard sheath and trocar kit, although sheathless access may also be employed. It should be noted that although the procedure described herein and illustrated in FIGS. 34-52 is initiated in the right femoral artery 532, the same procedure could be carried out beginning in the left femoral artery 533 with the orientation reversed. A vasodilator may optionally be administered to the patient at this point as previously discussed. If desired, a vasodilator may also be administered later in the procedure, but preferably prior to or simultaneous with the step of introducing inflation material into the graft 401.

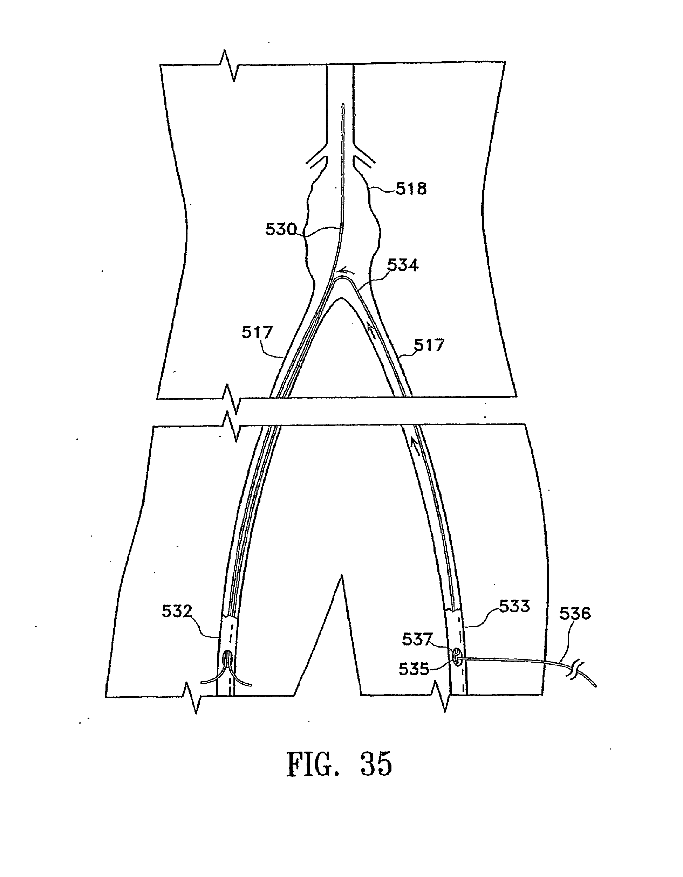

With the first guidewire 530 positioned across the aneurysm 518, a second guidewire 534 is then introduced into the ipsilateral or right femoral artery 532 and guided into the iliacs 517 and then back down into the contralateral or left femoral artery 533 as shown in FIG. 35. A distal end 535 of the second guidewire 534 may then be captured with a snare 536 or similar device inserted through an access hole 537 in the left femoral artery 533. The distal end 535 of the second guidewire 534 may then be pulled out of the left femoral artery 533 through the same left femoral artery access hole 537, providing a continuous length of wire passing through each iliac artery 517 via the left and right femoral artery access holes 537 and 531 as shown in FIG. 35.

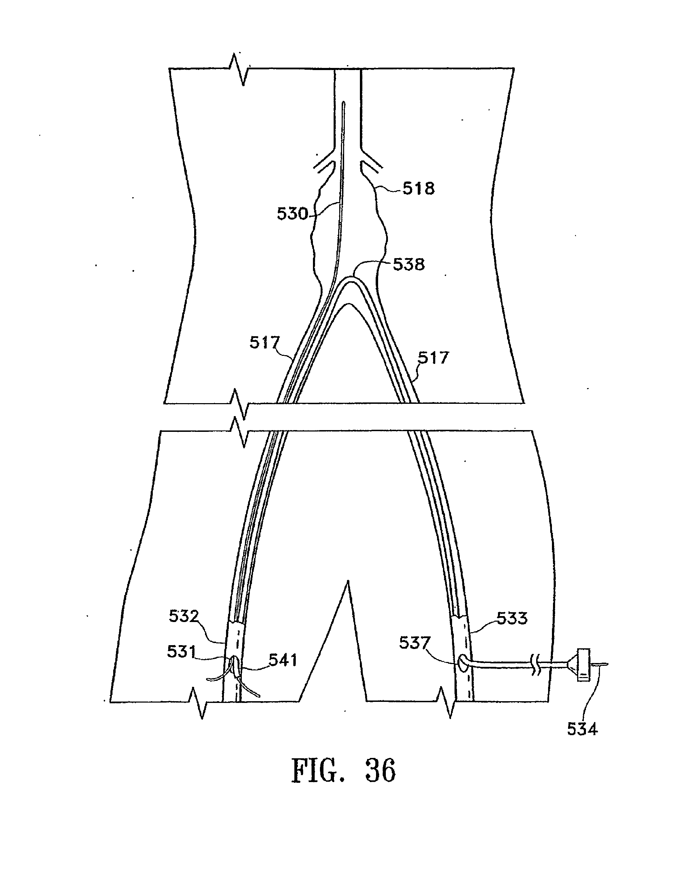

Once the second guidewire 534 exits the access hole 537 in the left femoral artery 533, a tubular catheter 538 may be advanced over the second guidewire 534 through the left femoral artery access hole 537 so as to extend out of the body from the access hole 531 in the right femoral artery 532 as shown in FIG. 36. This provides a continuous conduit between the right and left iliac arteries 517. With a distal end 541 of the tubular catheter 538 extending from the access hole 531 in the right femoral artery 532, a distal end 542 of the secondary release cable 438 may then be affixed to a proximal end 543 of the second guidewire 534 as shown in FIG. 37. For purposes of simplicity, the secondary release cable 438 is shown in, e.g., FIGS. 37-40 in schematic form as a single strand. However, it is understood that the term "secondary release cable" encompasses a single or multiple-component feature of the present invention that may be used to assist in the deployment of the graft. For instance, in the embodiment depicted herein, the secondary release cable 438 represents the combination of the release strand 481 and release strand tube 441 discussed above in conjunction with, e.g., FIG. 26.

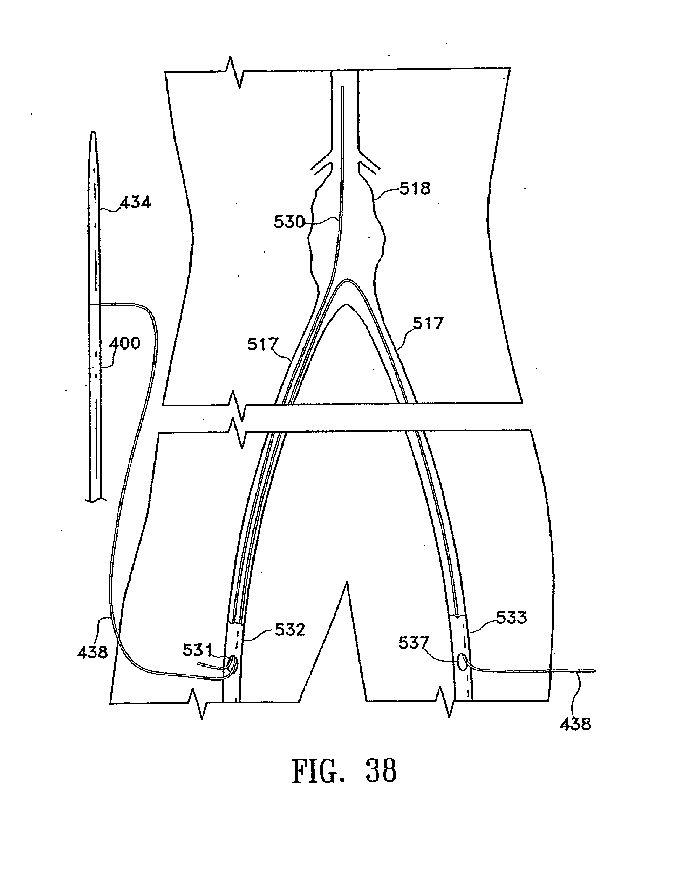

The second guidewire 534 is then pulled out of the tubular catheter 538 from the left femoral artery access hole 537, in the direction indicated by the arrow 544 in FIG. 37, so that the secondary release cable 438 then extends through the tubular catheter 538 from the right iliac artery to the left iliac artery. The tubular catheter 538 may then be withdrawn, leaving the secondary release cable 438 extending through the left and right iliac arteries 517 from the access hole 531 in the right femoral artery 532 to the access hole 537 in the left femoral artery 533 as shown in FIG. 38. The first guidewire 530 remains in position across the aneurysm 518.

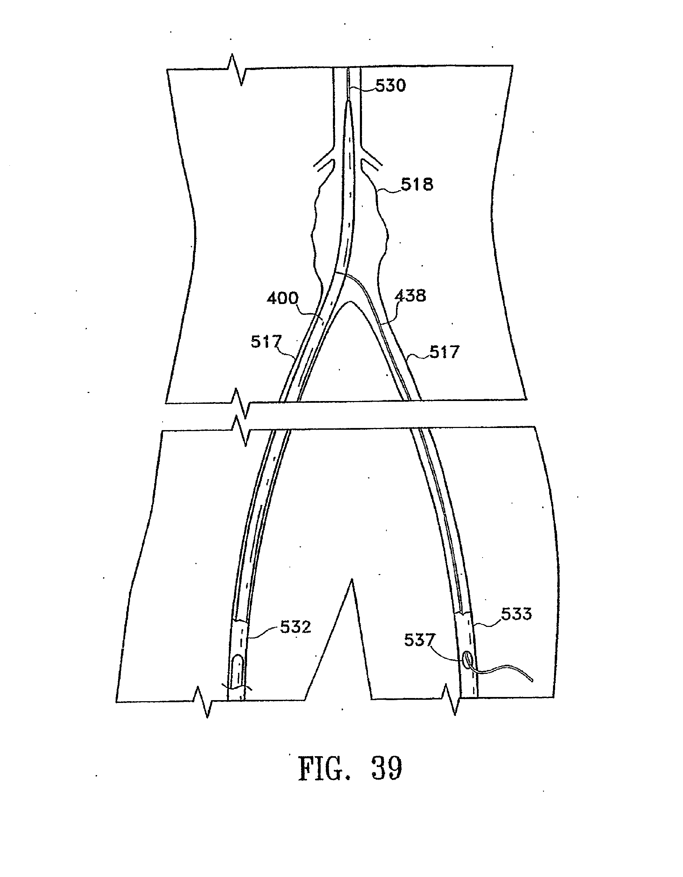

The delivery system 400 is then advanced into the patient's right femoral artery 532 through the access hole 531 over the first guidewire 530 as shown in FIG. 39. It may be desirable to apply tension to the secondary release cable 438 as the delivery system 400 is advanced to the vicinity of the aneurysm 518 so as to remove slack in the cable 438 and prevent tangling of the cable 438 or the like. Tension on the secondary release cable 438 may also help to prevent twisting of the delivery system 400 during insertion.

FIGS. 37A-B show an optional marker band that may disposed adjacent nosepiece 434 or generally in the vicinity of the distal end of the delivery system 425. Such a marker band 551 may also be integral with the delivery system 400; for example, it may be incorporated as part of the distal nosepiece 434. A useful marker 551 can be one that does not add to the profile of the delivery system 400 as shown in FIG. 37A (i.e., one that does not give the delivery system 400 a higher diameter). The examples of FIGS. 37A-B are useful in the present example, although they may be used in the examples discussed above. Such a marker may be used to aid the operator in introducing the delivery system 400 without twisting.

For example, the marker example 551 of FIG. 37A comprises a marker body 552 in the form of a simple discontinuous ring made of an appropriate radiopaque material (e.g., platinum, gold, etc.) visible under fluoroscopy, etc. The cross section of the ring may be asymmetric so that under fluoroscopy the cross section may be seen in the vicinity of the discontinuity 553. The operator will be able to tell if the delivery system 400 is twisted by how the ring 552 is presented under fluoroscopy. Alternatively, ring 552 may be continuous but have a notch or similar cutout to serve the same purpose.

The example 554 of FIG. 37B is an example of such a marker. Here, both a notch 555 and two circular holes 556 have been cut out of the marker body 557 for easier determination of its orientation when disposed on the notch or other part of the delivery system 400. For instance, in an orientation where the two circular holes 556 are aligned with respect to the fluoroscope field of view, the user will see a single circular hole to the left of a triangular or vee-shape cutout 555 on the side of the marker 554. As the angular orientation of the device 400 (and thus the marker 554) about the longitudinal axis changes, the appearance of the two circular holes 556 and side notch 555 will change. If the device is twisted clockwise ninety degrees from this orientation along its central longitudinal axis 554A, for instance, the circles 556 will largely disappear from view and the side notch 555 will generally appear in the front of the field of view as a symmetric diamond. Comparing these views will allow the user to know that the entire delivery system 400 has twisted about ninety degrees. Keeping the same orientation, then, will be made easier with such a marker 554.

For each of the examples of FIGS. 37A-B, variations in the shape, number, orientation, pattern and location of the notch 553 and 555, holes 556 or other discontinuity, as well as various marker body dimensions cross sectional shape, etc., maybe realized, as long as the marker 551 and 554 is configured so that the angular orientation of the delivery system 400 may readily be determined by the user under fluoroscopy or similar imaging technique.

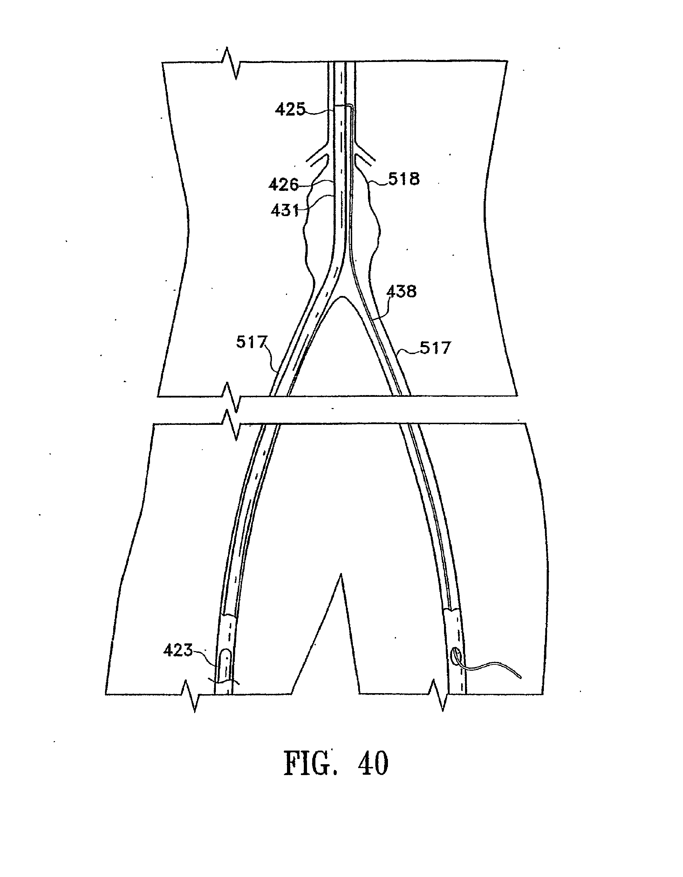

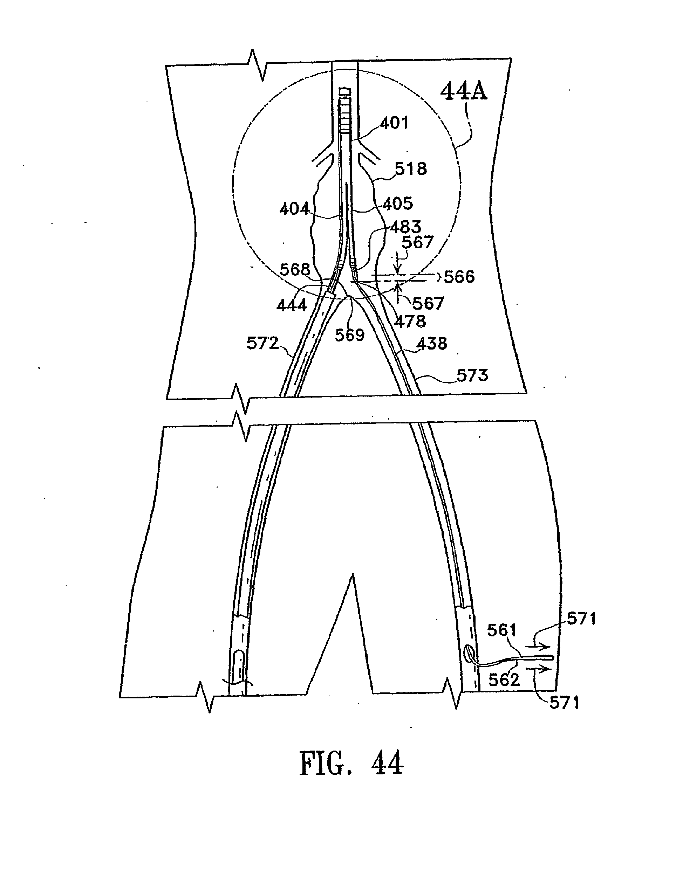

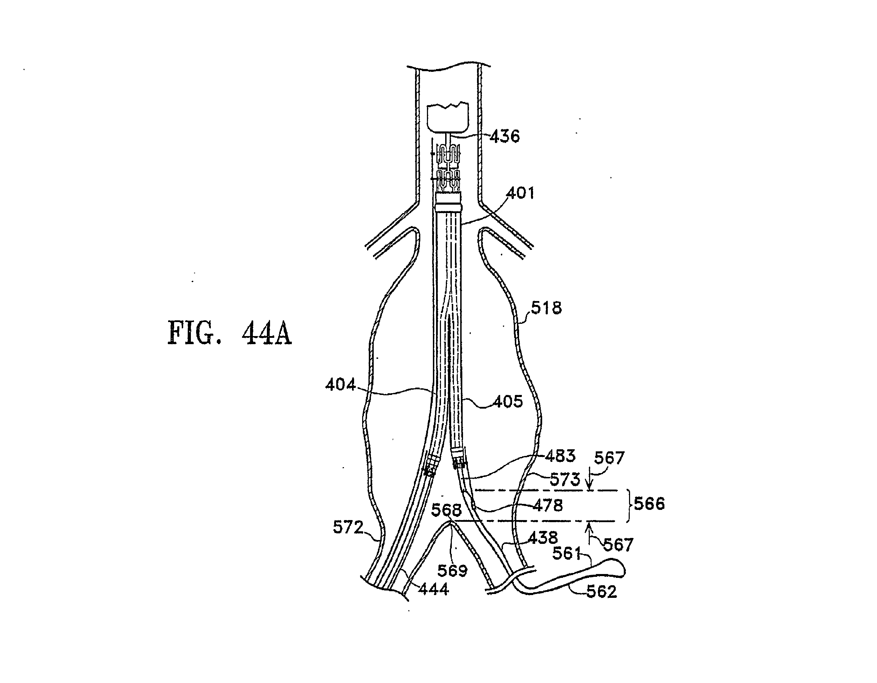

The delivery system 400 is positioned in a location suitable for initiating the deployment process, such as one in which the distal end 425 of the delivery system 400 is disposed beyond, or distal to the position in which the graft 401 will be placed, as shown in FIG. 40. This position allows the proximal end 483 of the secondary belt support member 454 to be laterally displaced without mechanical interference from the patient's vasculature. Such clearance for lateral displacement is shown in FIG. 44.

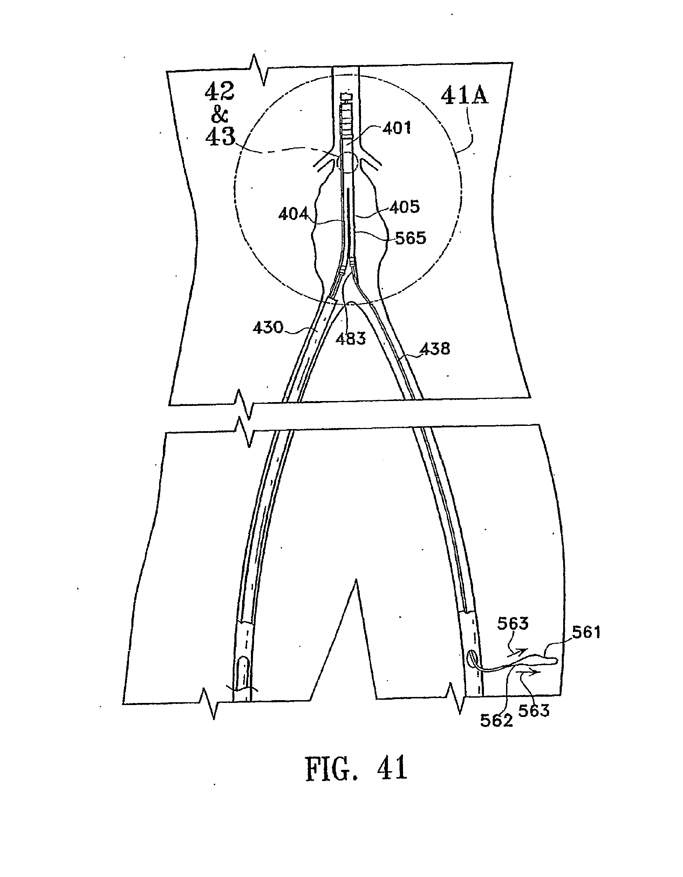

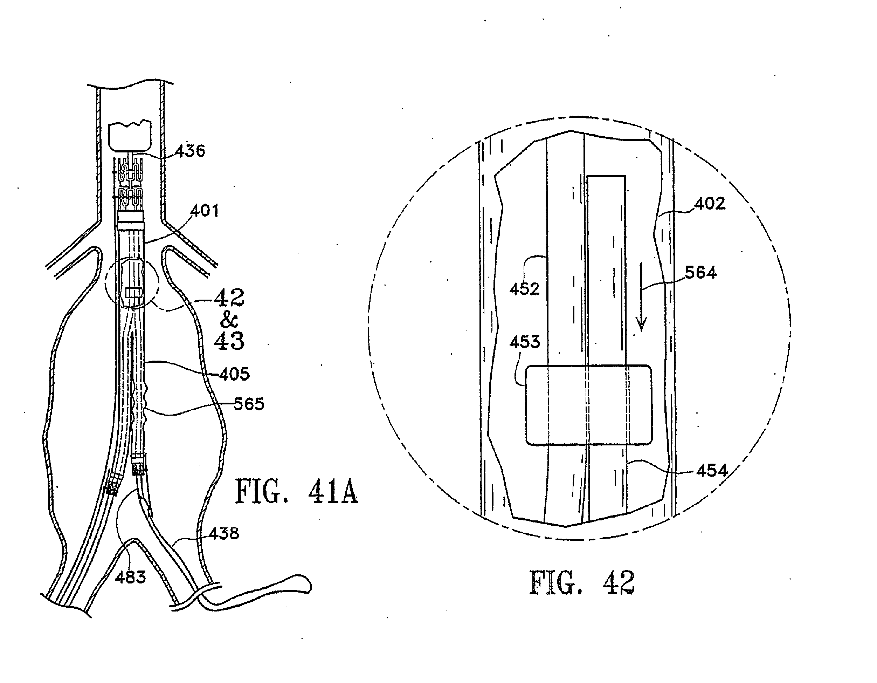

Once the distal section 426 of the elongate shaft 423 and the endovascular graft 401 are positioned, the deployment process is initiated. First, the outer tubular member 431 is proximally retracted by pulling on the proximal end 433 of the outer tubular member 431 relative to the inner tubular member 430. The inner tubular member 430 should be maintained in a stable axial position, as the position of the inner tubular member 430 determines the position of the constrained bifurcated graft 401 prior to deployment. Upon retraction of the outer tubular member 431, the constrained bifurcated graft 401 is exposed and additional slack is created in the secondary release cable 438 as shown in more detail in FIG. 41.

Alternatively, a variety of different components may be substituted for the outer tubular member 431 in some of the examples of the invention. For instance, a shroud, corset, mummy-wrap, or other cover may be released or actuated to expose the constrained graft 401 after the delivering system 400 is introduced into the vasculature.

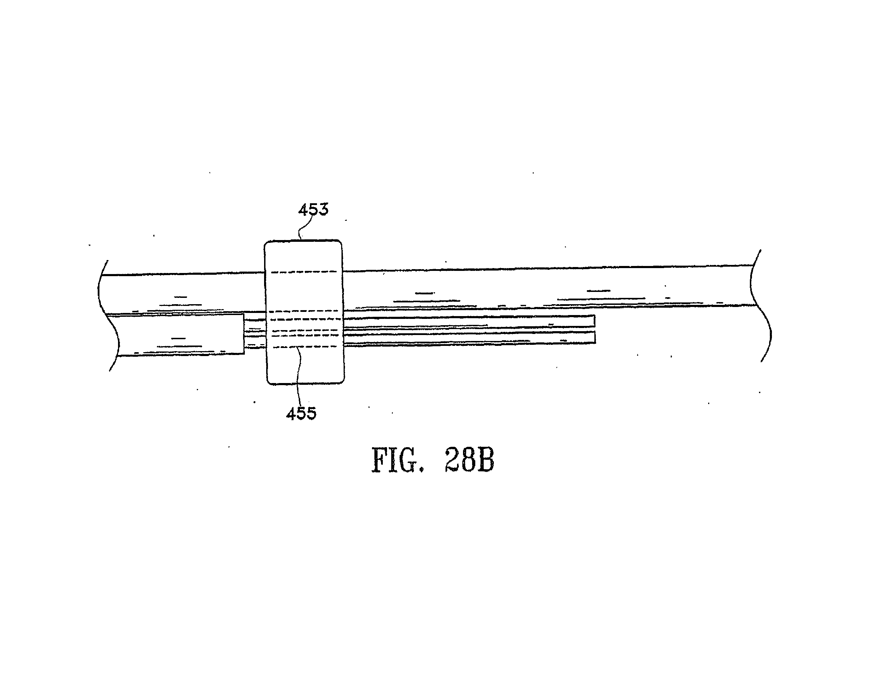

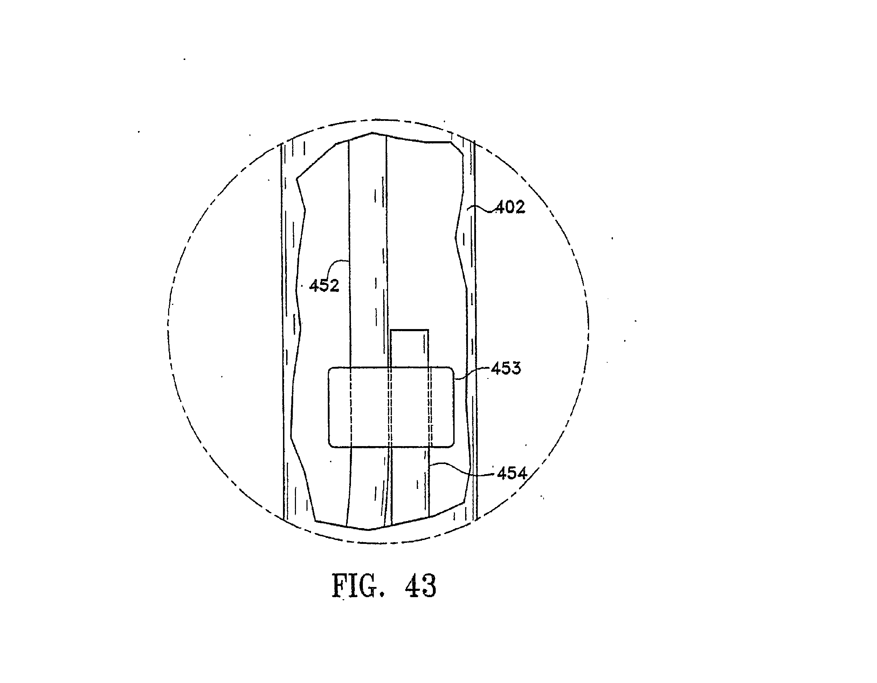

The slack in the secondary release cable 438 is taken up by applying tension to both lengths 561 and 562 of the release strand 481 as shown by the arrows 563 in FIG. 41.. In alternative examples, release strand is not continuous such that lengths 561 and 562 each has a free end, each of which may be manipulated by the operator. As tension continues to be applied to both lengths 561 and 562 of the release strand 481, the secondary belt support member 454 begins to slide within the secondary belt support member housing 453 in a proximal direction as shown by the arrow 564 in FIG. 42. The secondary belt support member 454 continues to slide proximally until all the slack is removed from an axially compressed or folded portion 565 of the contralateral leg 405 of the graft 401 shown in FIG. 41 and the primary and secondary belt support members 452 and 454 are oriented relative to the secondary belt support member housing 453 as generally shown in FIG. 43. Rotational movement of the secondary belt support member 454 relative to the secondary belt support member housing 453 is prevented by the non-circular or asymmetric cross section of the member 454 as shown in FIGS. 28-28B. This prevents the contralateral leg 405 from twisting or becoming entangled with other components of the graft 401 or delivery system 400 during deployment.

Axial compression of all or a portion of the contralateral leg 405 while the graft 401 is in a constrained state within the delivery system 400 prior to deployment allows the axial position of the two proximal self-expanding members 407 and 408 to be axially offset from each other. Alternatively, graft legs 404 and 405 having different lengths may be used to prevent overlap of the self-expanding members 407 and 408 within the delivery system 400. The cross sectional profile or area of the overlap self-expanding members 407 and 408 is generally greater than that of the adjacent polymer material portion of the legs 404 and 405 of the graft 401, so eliminating the overlap can be desirable. The self-expanding members 407 and 408 are typically made of a metal or metallic alloy and maintain a cylindrical configuration, even when in a constrained state. The polymer material of the legs 404 and 405 or main body portion 402 of the graft 401, by contrast, is relatively soft and malleable and can conform to the shape of whatever lumen in which it may be constrained. Placing both proximal self-expanding members 407 and 408 adjacent each other in a compressed state at a single axial position within the delivery system 400 would require a configuration in which two objects having an approximately circular cross section are being placed within another circular lumen. Such a configuration generates a significant amount of wasted or unused cross sectional area within that axial position of the delivery system 400 and would likely result in less flexibility and greater cross section than a delivery system 400 in which the proximal self-expanding members 407 and 408 are axially offset.

A gap 566 indicated by the arrows 567 in FIG. 44 allows the proximal end 483 of the secondary belt support member 454 and secondary release wire actuator hub 478 to move in a lateral direction without mechanical interference from the carina 568 of the iliac artery bifurcation 569. Gap 566 may vary depending on the patient's particular anatomy and the specific circumstances of the procedure.

The lateral movement of the contralateral leg 405 and secondary belt support member 454 is accomplished by application of tension on both lengths 561 and 562 of the release strand 481 as shown by the arrows 571 in FIG. 44. This movement away from the primary belt support member 452 allows the secondary belt support member 454 to transition from alignment with the right iliac artery 572 to alignment with the left iliac artery 573 as shown in FIG. 44.

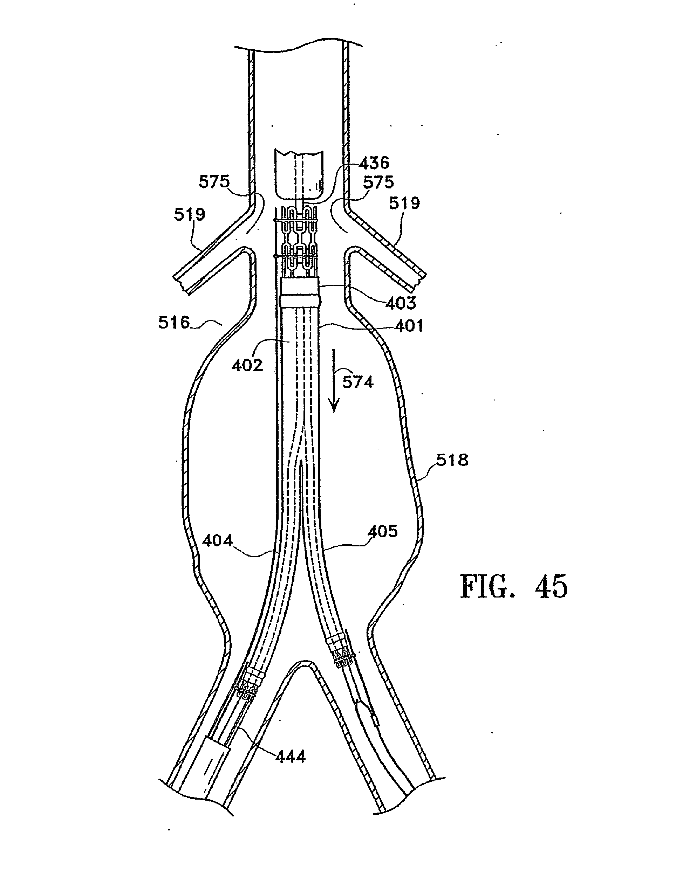

Once the ipsilateral leg 404 of the graft 401 and contralateral leg 405 of the graft 401 are aligned with the right and left iliac arteries 572 and 573, respectively, the delivery system 400 may then be retracted proximally, as shown by the arrow 574 in FIG. 45, so as to reposition the distal section 426 of the elongate shaft 423 and the bifurcated graft 401 into the desired position for deployment as shown in FIG. 45.

As discussed above with respect to placement of a tubular graft example 11 , when deploying the graft 401 in the abdominal aorta 516 it is generally desirable to ensure that the distal end 403 of the graft main body portion 402 is installed proximal to, or below, the renal arteries 519 in order to prevent their significant occlusion. However, the distal self-expanding members 411 and 422 of the graft 401 may, depending upon the anatomy of the patient and the location of the aneurysm 518, partially or completely span the ostia 575 of one or both renal arteries 519. It can be desirable, however, to ensure that ostia 575 of the renal arteries 519 are not blocked by the distal end 403 of the graft main body portion 402. As discussed previously, a variety of imaging markers 551 and 554 may be used on either or both the delivery system 400 and the graft 401 itself to help guide the operator during the graft positioning process.

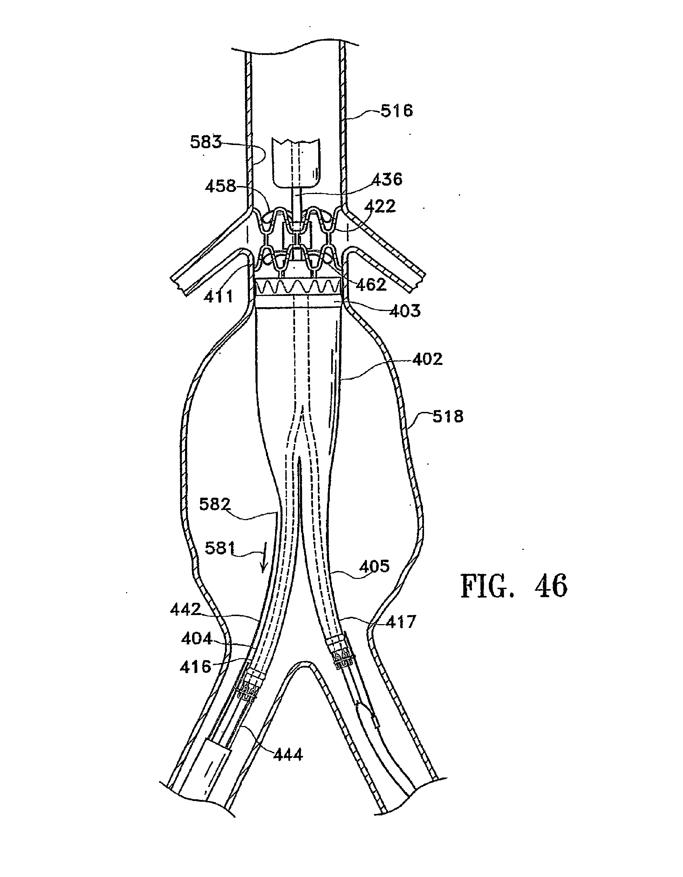

After proper positioning, the first and second distal self-expanding members 411 and 422 may then be deployed. The operator first unscrews or otherwise detaches a threaded portion 576 of the distal primary release wire handle 495 from an outer threaded portion 577 of a first side arm end cap 578 shown in FIG. 31. Next, the distal primary release wire handle 495 is proximally retracted, which in turn retracts the distal primary release wire 442 in a proximal direction, as shown by the arrow 581 in FIG. 46. As the distal end 582 of the distal primary release wire 442 passes through the end loops 472 and 473 of the first distal primary belt 458 and second distal primary belt 462, the end loops 472 and 473 are released, freeing the first distal self-expanding member 422 and second distal self-expanding member 411 to self-expand in an outward radial direction so to contact an inner surface 583 of the patient's aorta 516. The first and second distal primary belts 458 and 462 remain secured to the primary belt support member 452 and will eventually be retracted from the patient with the delivery system 400 after deployment is complete.

As the first and second distal self-expanding members 411 and 422 expand and contact the aorta 516, a distal end 403 of the graft main body portion 402 opens with the self-expanding members 411 and 422 and promotes opening of the graft polymer material portion from the flow of blood into the distal end 403 of the graft main body portion 402 with a "windsock" effect. As a result, once the first and second distal self-expanding members 411 and 422 are expanded to contact the aorta inner surface 583, the graft main body portion 402 and legs 404 and 405 balloon out or expand while the proximal ends 416 and 417 of the legs 404 and 405 of the graft 401 remain constricted due to the constrained configuration of the proximal self-expanding members 407 and 408 of the ipsilateral and contralateral legs 404 and 405, as shown in FIG. 46. At this point, there typically will be partial or restricted blood flow through and around the graft 401.

Bifurcated graft 401 may then be optionally be inflated with an inflation material via inflation tube 444 and inflation port 421 until the inflatable channels 418 and inflatable cuffs 413, 414 and 415 have been filled to a sufficient level to meet sealing and other structural requirements necessary for the bifurcated graft main body portion 402 and the ipsilateral and contralateral legs 404 and 405 to meet clinical performance criteria. As described in later conjunction with an alternative disclosure, inflating the graft 401 prior to deploying the proximal and distal self-expanding members 407 and 408, respectively, is useful in anatomies where the vasculature is tortuous or angled.

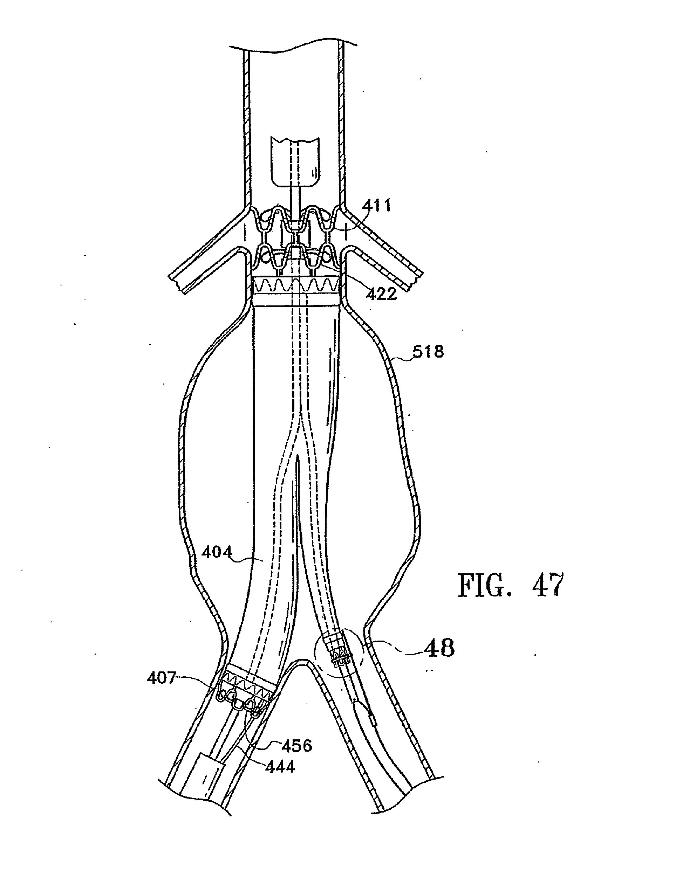

Next, the proximal self-expanding member 407 of the ipsilateral leg 404 is deployed. Deployment of the first and second distal self-expanding member 411 and 422 has exposed the proximal primary release wire handle 496, making it accessible to the operator. A threaded portion 584 of the proximal primary release wire handle 496 is unscrewed or otherwise detached from an inner threaded portion 585 of the first side arm end cap 578. The proximal primary release wire handle 496 may then be retracted proximally so as to deploy the proximal primary belt 456 and proximal self-expanding member 407 of the ipsilateral leg 404 as shown in FIG. 47.

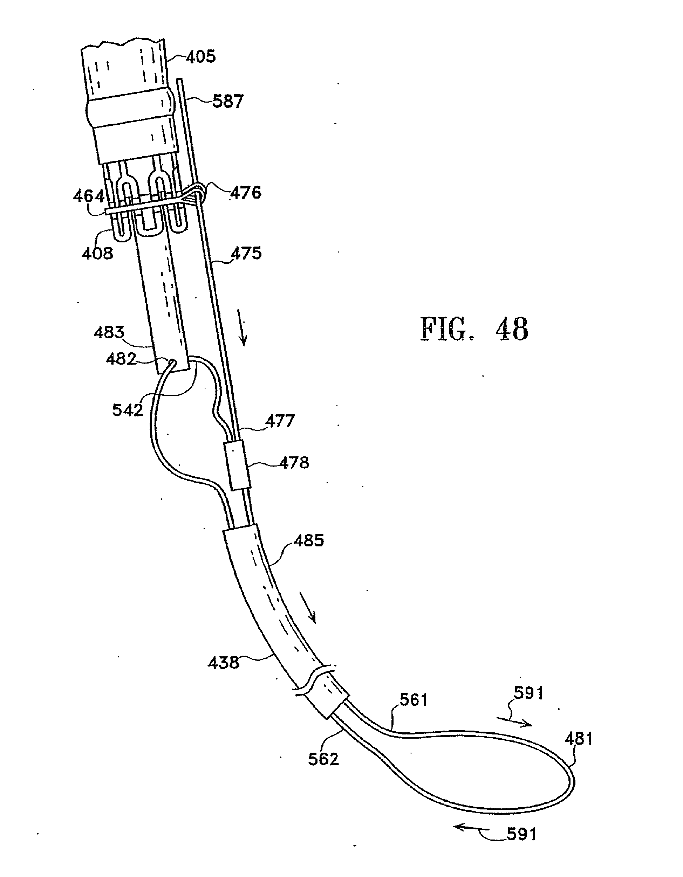

FIG. 48 depicts an enlarged view of the proximal end 483 of the secondary belt support member 454. The proximal self-expanding member 408 of the contralateral leg 405 is secured to the proximal end 417 of the contralateral leg 405. The proximal self-expanding member 408 is constrained in a radial direction by the secondary belt 464, which has end loops 476 releasably constrained by the distal end 587 of the secondary release wire 475. The proximal end 477 of the secondary release wire 475 terminates with and is secured to the actuator hub 478. The release strand is secured to the actuator hub 478 and loops through an aperture or hole 482 in the proximal end 483 of the secondary belt support member 454. As discussed above, a portion of the release strand 481 is disposed within the release strand tube 485 to form the secondary release cable 438.

When both a first length 561 and second length 562 of the release strand 481 are pulled together in a proximal direction from a proximal end 588 of the secondary release cable 438, the entire pulling force is exerted on the proximal end 483 of the secondary belt support member 454 because the looped distal end 542 of the release strand 481 pulls on the proximal end 483 of the secondary belt support member 454 without displacing the actuator hub 478.

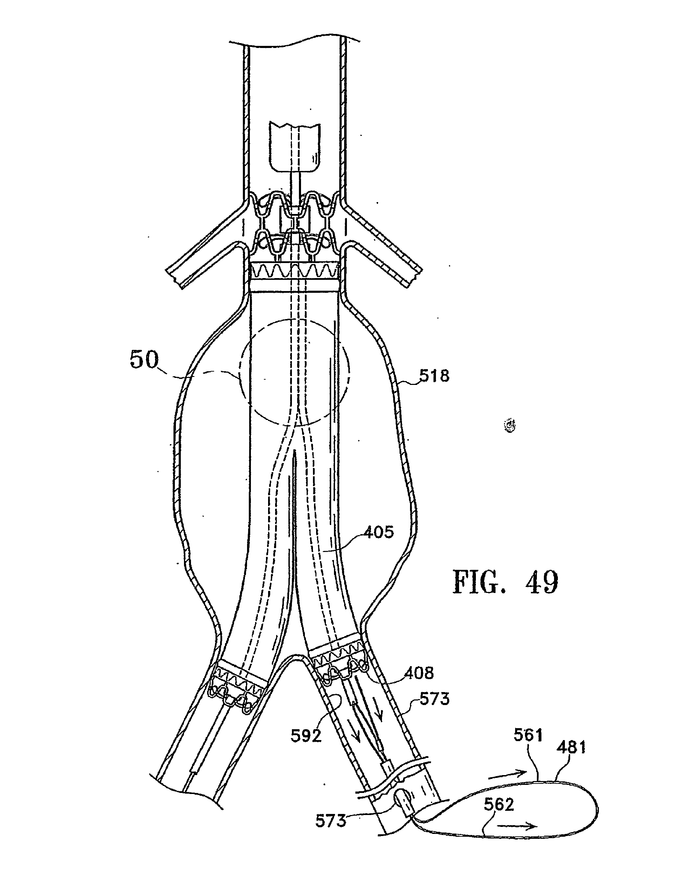

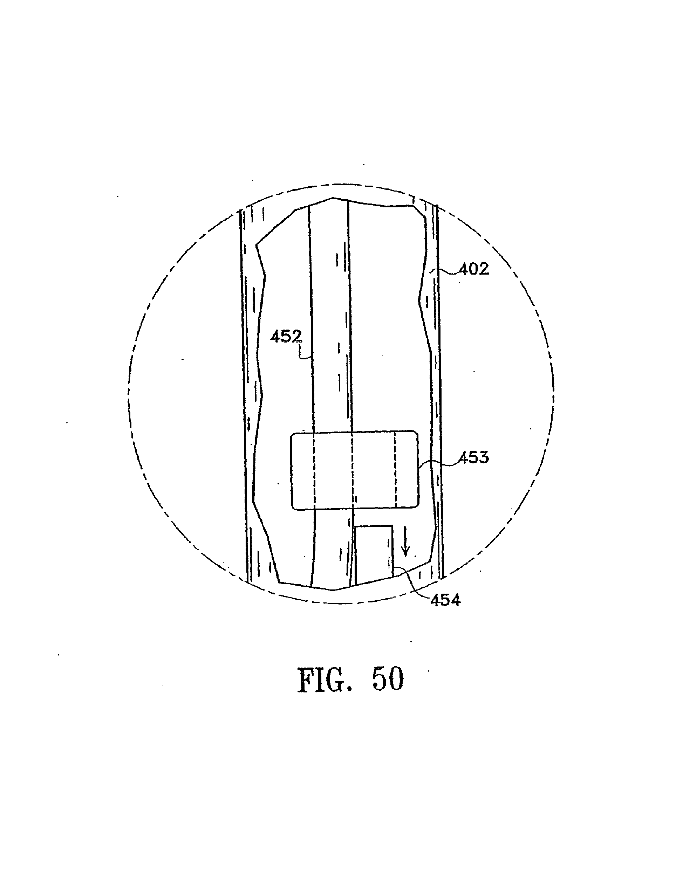

When deployment of the proximal self-expanding member 408 of the contralateral leg 405 is desired, the operator applies tension in a proximal direction only to the first length 561 of the release strand 481, which extends proximally from the actuator hub 478. The direction of such tension is indicated in FIG. 48 by the arrows 591. Upon the application of this proximal tension, the actuator hub 478 is moved proximally, as is the secondary release wire 475 that is secured to the actuator hub 478. The proximal self-expanding member 408 of the contralateral leg 405 deploys when the distal end 587 of the secondary release wire 475 passes through the end loops 468 of the secondary belt 464 so as to release the radial constraint on the proximal self expanding member 408 imposed by the secondary belt 464. Upon release of the radial constraint, the proximal self-expanding member 408 expands so as to contact an inside surface 592 of the left iliac artery 573 as shown in FIG. 49. Once the proximal self-expanding member 408 of the contralateral leg 405 is expanded, the operator may then apply tension to both lengths 561 and 562 of the release strand 481 to withdraw the secondary belt support member 454 from the housing 453 (as shown in FIG. 50) and remove it from the patient's vasculature through the left femoral artery access hole 537.

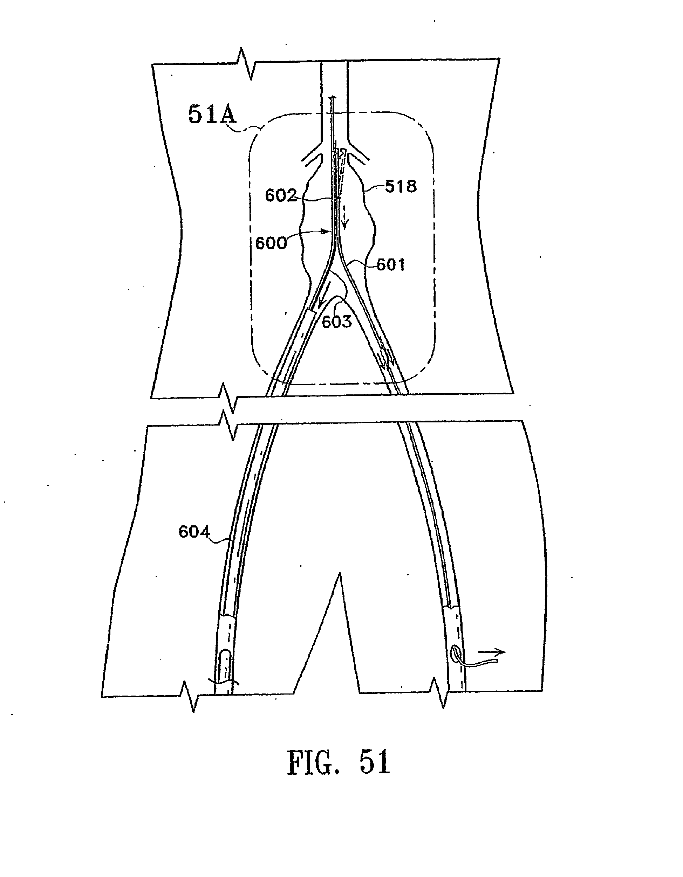

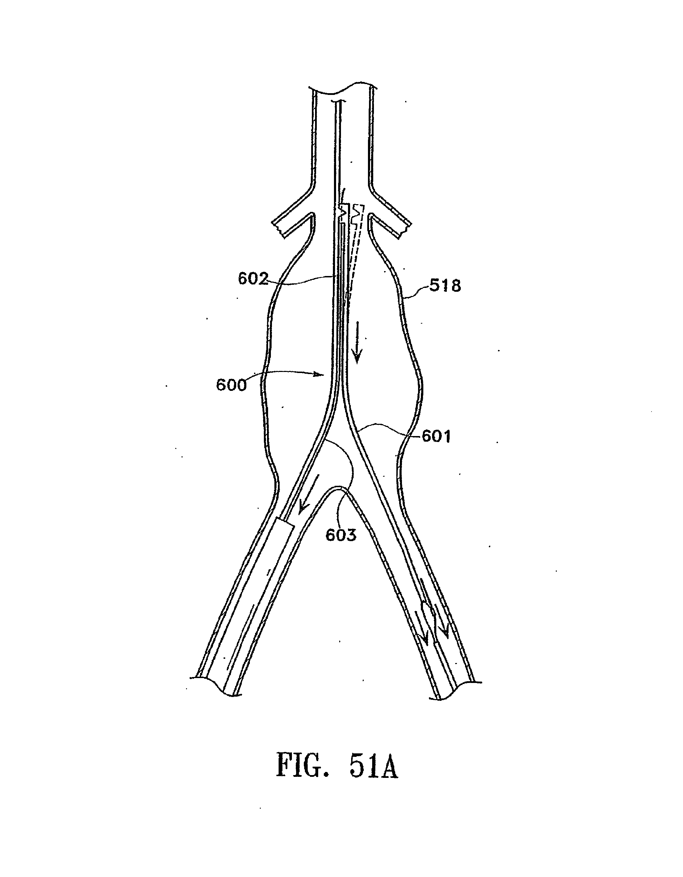

FIG. 51 depicts an alternative example of a belt support member assembly 600 in which the secondary belt support member 601 is detached from the primary belt support member 602 by withdrawal of a latch wire 603. Generally, all other features of the delivery system 604 of the example of FIG. 51 can be the same as the delivery systems discussed above. It should be noted, however, that the example shown in FIG. 51 does not allow the secondary belt support member 601 to slide in an axial direction relative to the primary belt support member 602. As such, it may be desirable to use this example to deliver and deploy a graft having legs that are not substantially equal in length. Otherwise, if proximal self-expanding members are to be axially offset, the secondary belt support member 601 would have to be detached from the primary belt support member 602 prior to deploying and releasing the secondary belt (not shown).

In another configuration (not shown), a similar retention or latch wire 603 passes through aligned apertures in the secondary belt support member 454 and a housing, such as secondary belt support member housing 453 of FIG. 43. Linear and rotational motion of secondary belt support member 454 relative to primary belt support member 452 is prevented until wire 603 is withdrawn, freing member 454 to be removed from housing 453. Typically the aperatures are disposed at an angle (such as about 45 degrees) relative to the surface of the members through which they reside so to minimize the angles through which retention wire 603 turn as is passes through the apertures. Retention wire may double as the primary proximal release wire for one or both of proximal self-expanding members 411 and 422.

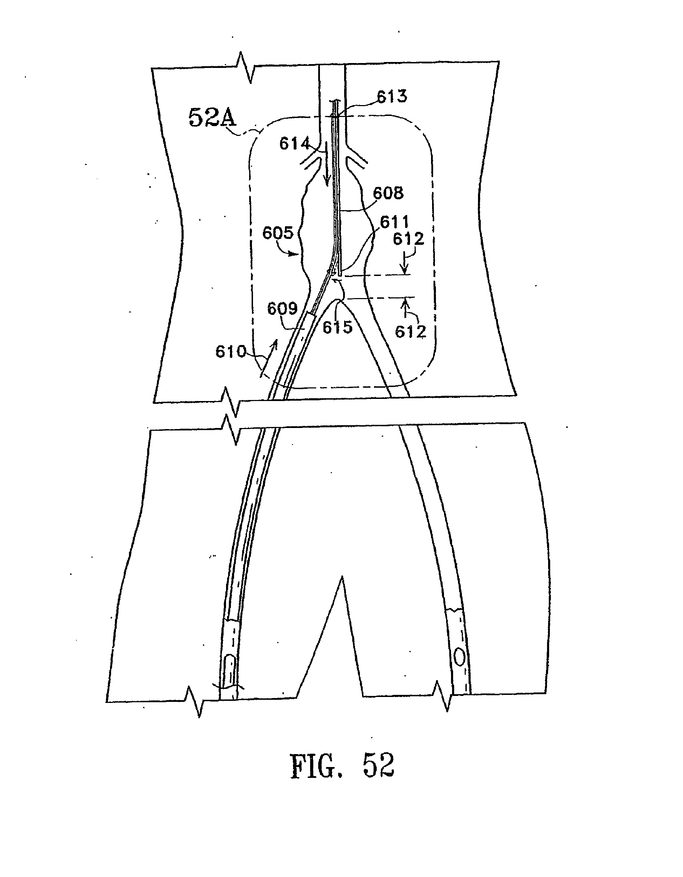

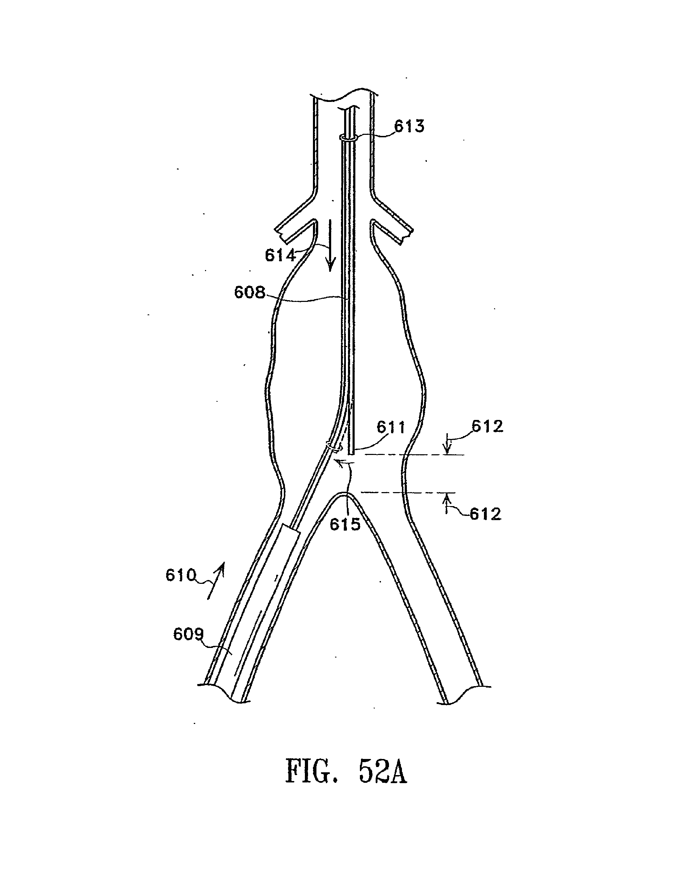

FIG. 52 shows an alternative belt support member assembly 606 wherein the secondary belt support member 607 is laterally displaced and locked into a position parallel with the primary belt support member 608 prior to removal of the delivery system 609 from the patient's vasculature. All other features of the delivery system 609 of the example of FIG. 52 can be the same as the delivery systems discussed above. In use, after all self-expanding members have been deployed, the delivery system 609 is advanced distally into the patient's vasculature, as shown by the arrow 610 in FIG. 52, in order to achieve a gap between a proximal end 611 of the secondary belt support member 607 and the patient's vasculature as shown by the arrows 612 in FIG. 52. A constraining ring 613 is then retracted proximally, as indicated by the arrow 614, so as to force the secondary belt support member 607 to be laterally displaced as shown by the arrow 615, also in FIG. 52. Once the secondary belt support member 607 has been fully retracted in a lateral direction so as to be substantially parallel to the primary belt support member 608, the delivery system 609 can then be retracted from the patient's vasculature.

If not previously filled, the bifurcated graft 401 may thereafter be inflated with an inflation material described with respect to the tubular graft embodiment 11.

For all the examples described, both tubular and bifurcated, inflation is generally accomplished by inserting or injecting, via one or more device such as a syringe or other suitable mechanism, the inflation material under a pressure- or volume-control environment.

For instance, in one example of a pressure-control technique, a volume of inflation material is first injected into the delivery system 400 (which at this point may include the graft, but may also include the inflation tube 444). The particular desired volume of inflation material will depend on several factors, including, e.g., the composition and nature of the inflation and polymer graft material, the size of the graft 401 to be deployed, the vessel or lumen diameter into which the graft 401 is deployed, the configuration of the graft 401 (tubular, bifurcated, etc.), the features of the graft main body 402 and (if present) legs 404 and 405, and the conditions during the procedure (such as temperature).

Thereafter, the operator may affix a pressure control device, such as an inflation syringe, to the injection port 621 of the proximal adapter 427 of the inflation tube and apply a pressure to the delivery system 400 and a graft 401 for a period of time. This serves to ensure that the fill material previously introduced enters the graft 401 and fills it to the desired pressure level.

We have found that a useful pressure-control approach involves a series of such constant pressure applications, each for a period of time. For instance, the graft 401 may first be pressurized at a level from about 0,34 bar to about 0,83 bar (5 psi to about 12 psi) or higher, preferably about 0,62 bar (9 psi), for between about 5 seconds and 5 minutes, preferably about 3 minutes or more. Optional monitoring of the fluid and the device during the fill procedure may be used to help ensure a proper fill. Such monitoring may be accomplished under fluoroscopy or other technique, for instance, if the fill material is radiopaque.

Thereafter, the fill protocol may be completed, or the pressure may be increased to between about 0,69 bar and about 1,03 bar (10 psi and about 15 psi) or higher, preferably about 0,83 bar (12 psi), for an additional period of time ranging from between about 5 seconds and 5 minutes or more, preferably about 1 minute. If the graft 401 so requires, the pressure may be increased one or more additional times in the same fashion to effect the proper fill. For instance, subsequent pressure may be applied between about 0,83 bar and 1,38 bar or more (12 and 20 psi or more), preferably about 1,10 bar to 1,24 bar (16 psi to 18 psi), for the time required to satisfy the operator that the graft 401 is sufficiently filled.

The details of particular pressure-time profiles, as well as whether a single pressure-time application or a series of such applications is used to fill embodiments of the graft 401 will depend on the factors described above with respect to the volume of fill material used; the properties and composition of the fill material tend to be of significance in optimizing the fill protocol. For example, a stepped series of pressure-time profiles as described above is useful when the fill material comprises a hardenable or curable material whose physical properties may be time-dependent and which change after being introduced into the graft 401 and its delivery system 400.

Alternatively, a volume-control method may be utilized to fill examples of the grafts 11 and 401, including both tubular and bifurcated. Here, a volume of fill material is again introduced into the delivery system 400 as described above. In this method, however, the volume of fill material used is precisely enough material to fill the graft 401, the inflation tube 444, and any other component in the delivery system 400 through which the fill fluid may travel on its way to the graft 401. The operator introduces the predetermined quantity of fill material, preferably with a syringe or similar mechanism, into the inflation tube 444 and graft 401. A precise amount of fill material may be measured into a syringe, for example, so that when the syringe is emptied into the delivery system 400 and graft 401, the exact desired amount of fill material has reached the graft 401. After a period of time (which period will depend on the factors previously discussed), the syringe or equivalent may be removed from the inflation tube 444 or injection port 621 of proximal adapter 427 and the procedure completed.

A pressurized cartridge of gas or other fluid may be used in lieu of a syringe to introduce the fill material into the delivery system and graft under this volume-control regime so to provide a consistent and reliable force for moving the fill material into the graft 401. This minimizes the chance that variations in the force and rate of fill material introduction via a syringe-based technique affect the fill protocol and possibly the clinical efficacy of the graft 401 itself.

For each of the pressure- and volume-control configurations, an optional pressure relief system may be included so to bleed any air or other fluid existing in the delivery system 400 prior to the introduction of the fill material (such as the inflation tube 444 or graft 401) so to avoid introducing such fluid into the patient. Such an optional system may, for example, comprise a pressure relief valve at the graft 401/inflation tube 444 interface and a pressure relief tube disposed through the delivery system 400 (e.g., adjacent the inflation tube 444) terminating at the proximal adapter 427 and vented to the atmosphere.

When graft 401 is deployed in certain anatomies, such as those where the iliac arteries are tortuous or otherwise angled, the lumen of one or more of graft inflatable cuffs 413, 414 and 415 and channels 418 of may become pinched or restricted in those portions of the graft 401 experiencing a moderate or high-angle bend due to the tortuosity of the vessel into which that portion of graft 401 is deployed. This reduction or even elimination of cuff/channel patency can hinder and sometimes prevent adequate cuff and channel inflation.

In addition, graft 401 main body 402 and/or legs 404, 405 may, upon initial retraction of outer tubular member 431 and deployment into the vasculature, resist the "windsock" effect that tends to open up the graft to its nominal diameter. Then in turn may lead to inadequate cuff 413, 414, and 415 and channel 418 patency prior to their injection with inflation material. The windsock effect has a higher likelihood of being hindered when graft 401 is deployed in relatively tortuous or angled anatomies; however, it may also be made more difficult when graft 401 (and even tubular graft examples such as graft 11) is deployed in relatively non-tortuous anatomies.

To address this issue, we have found it useful to incorporate an optional ripcord or monofilament into the inflatable channel 418. Pre-loading such a ripcord 510 into all or a portion of the channel 418 that runs along graft ipsilateral leg 404 and main body portion 402 promotes effective inflation of the graft cuffs and channels as will be described below in detail.

Ripcord 510 extends in one embodiment from distal cuff 413 through channel 418, proximal cuff 414 and inflation port 421, and continues through inflation tube 444 and through second side ann 499 of proximal adapter 427 as shown in FIG. 31A. A flexible fill catheter 523 may be affixed to end of second side arm 499 at injection port 509. Ripcord 510 extends through injection port 509 and catheter 523 where it is affixed to a removable Luer-type fitting or cap 521 at catheter 523 terminus 525 (which can serve as an injection port). Alternatively, in lieu of catheter 523, fitting 521 may be removably connected directly to injection port 509. Fill catheter may compromise an optional pressure relief valve (not shown).

In use, after graft 401 has been deployed into the vasculature but prior to injecting the inflation material through second side arm 499, the operator removes fitting 521 from catheter 523 and pulls ripcord 510 proximally out of the ipsilateral graft channel 418, second side arm 499 and out through the end of catheter 523. This leaves behind an unobstructed lumen in channel 418 through which inflation material may pass as it is injected into the device, despite any folds, wrinkles, or angles that may exist in graft 401 due to vessel tortuosity or angulation, lack of windsocking, or other phenomena. Inflation material may then be injected into channel 418 and cuffs 413, 414 and 415 through second side arm 499 as described elsewhere herein. Inflation material passes through the lumen in channel 418 left behind after ripcord 510 is removed and reaches distal cuff 413. As cuff 413 fills, a hemostatic seal is created at distal end of graft 401 which promotes the desired windsocking of the graft. This in turn promotes the effective filling of the rest of the cuffs 414, 415 and channels 418 and any other lumens in which the inflation material may be directed.

Suitable materials for ripcord 510 include polymeric monofilaments, such as PTFE, Polypropylene, nVion, etc. Metallic filaments such as stainless steel, nickel titanium, etc. may be used as well. The diameter of ripcord 510 should be small compared to the diameter of channel 418 lumen to minimize impact on delivery system profile, yet large enough to permit reasonable flow of inflation material into channel 418 lumen following its removal. We have found that a ripcord 510 diameter of between about 0,013 cm and 0,06 cm (0.005 inch and 0.025 inch) to be appropriate; in particular, a ripcord diameter of about 0,038 cm (0.015 inch) is suitable.

Alternatively, or in conjunction with ripcord 510, one or more permanent monofilament lumen patency members or beads may be incorporated into one or more of the cuffs and channels to facilitate the inflation process. We have found it useful to incorporate a single bead into graft contralateral leg 405 channel 418 along with ripcord 510 in the graft ipsilateral leg 404 channel 418.

FIG. 31B is a simplified cross sectional schematic view of contralateral leg 405 inflatable channel 418 having a bead 520 disposed in a lumen 522 of channel 418, taken along line 31B-31B in FIG. 19. Typically bead 520 extends from proximal cuff 414 to distal cuff 413, although it may be disposed in only a portion of channel 418 or in other cuffs or channels of graft 401.

Channel 418 is shown in FIG. 31B as bent or angled out of the plane of the page to simulate contralateral limb 405 placement in a highly angled iliac artery. Under such bending forces, the walls 524 of channel 418 tend to close on lumen patency member 520, reducing the size of lumen 522 to be confined to the areas indicated in FIG. 31B. As can be seen, bead 520 prevents the lumen 522 from collapsing to the point where lumen 522 loses patency sufficient for satisfactory passage of inflation material.

Bead 520 may have the same dimensions and comprise materials the same as or similar to ripcord 510. In particular, we have found a PTFE bead having a diameter of about 0.020 inch to be useful in the channel 418 embodiments of the present invention.

We have found that incorporating a ripcord 510 and/or one or more lumen patency members 520 in the system of the present invention enhances the likelihood that graft cuffs and channels will reliably and sufficiently fill with inflation material. In one extreme experiment designed to test the feasibility of this concept, a bifurcated graft contralateral leg 405 having a bead 520 disposed in the contralateral limb channel 418 was tied into a knot at the leg proximal end 417. Inflation material was injected through ipsilateral leg inflation port 421 under a pressure-control protocol. All cuffs and channels of graft 401, including contralateral leg channel 418 and proximal cuff 415, filled completely without having to increase the fill pressure beyond normal levels.

Although the benefits of ripcord 510 and one or more beads 520 (together or in combination) may be most readily gained when graft 401 is deployed in tortuous or highly angled anatomies, these components are also useful in grafts deployed in relatively straight and non-tortuous anatomies. They may also be used in tubular stent-grafts of the present invention.

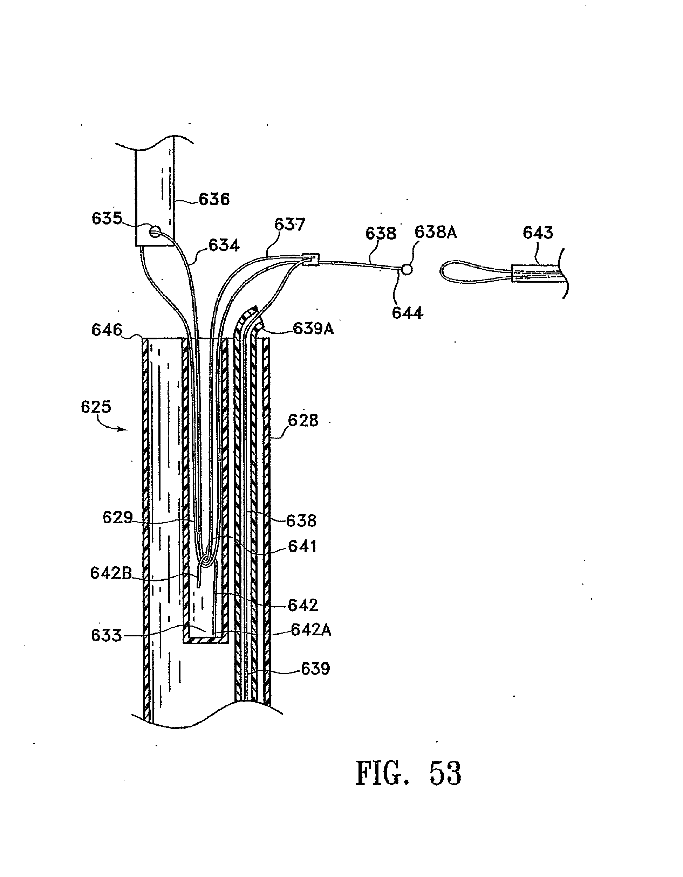

Turning now to FIG. 53, an examples of a bifurcated graft delivery system 625 and method is illustrated. This example is tailored to provide for a controlled withdrawal of a secondary release cable from a lumen of an inner tubular member 628 so to help eliminate the possibility that the release cable 626 becomes entangled or otherwise twisted during deployment.

Shown in FIG. 53 is a well 633 is disposed in the inner tubular member 628. Well 633 contains a release strand 629 that is looped at its proximal end 634 outside the well 633 through an aperture 635 in the secondary belt support member 636 and that is affixed or attached at its distal end 637 to a second guidewire 635. The second guidewire 638 is shown in the example of FIG. 53 as disposed in its own optional lumen 639 within the inner tubular member 628.

Within the well 633, the release strand 629 is arranged to form a "u-turn" in which it changes direction to double back on itself at juncture 641 as shown in FIG. 53. At juncture 641, a friction line 642 is looped around all or a portion of the release strand 629. This friction line 642 is fixed to the bottom of the well 633 on one end 642A and is free on another end 642B. The friction line 642 is preferably a polymeric monofilament such as polyimide, etc., but may be metallic and may be braided as necessary to achieve the desired friction characteristic needed to interact with release strand 629. Friction line 642 has a length sufficient to interact with the release strand 629 during the deployment process until the release strand 629 has been completely removed from the well 633 as will now be described in detail.

In use, the configuration of FIG. 53 works as follows. Once the left and right femoral access holes 531 and 537, discussed above, have been created, the delivery system 625 is introduced into and through the patient's vasculature. A snare catheter 643 is introduced into the left femoral artery access hole, such as the left femoral artery access hole 537 discussed above. The operator then captures the tip 644 of the second guidewire 638 with the snare 643. In the example of FIG. 53, the second guidewire 638 is shown as pre-attached to the release strand 629 at the distal end 637.

A ball capture tip 638A or similar member may optionally be disposed on the tip 644 of second guidewire 638 to facilitate its capture by snare catheter 643 and prevent possible injury to the vessel intima. In addition, tip 638A may be made radiopaque so that it may be readily located by the operator during the procedure. When in the form of a ball, tip 63 8A may have a diameter ranging from between about 0,05 cm to about 0,30 cm (0.020 inch to about 0.120 inch), specifically, between about 0,10 cm to about 0,15 cm (0.040 inch to about 0.060 inch). Although not shown in the figures, second guidewire 638 may also have one or more additional sections branching therefrom, each having a tip or member similar to tip 644, including tip 638A, so to provide the operator with one or more alternative sites for capture with snare 643 in case tip 638A is inaccessible.

An angled extension 639A may optionally be provided on one or both of the top of optional lumen 639 and/or the top of well 633. Angled extension 639A may be made of any suitable polymeric or metallic material such as stainless steel. As seen in FIGS. 53-54, extension 639A disposed on the top of lumen 639 is generally biased towards the artery in which snare 643 is disposed at an angle of between about 20 degrees and about 120 degrees, specifically, between about 40 degrees and about 95 degrees, so to guide the release strand 629 and 653 in the proper direction and thus facilitate ease of capture by snare 643.

As the second guidewire 638 is pulled out of the inner tubular member 628 from the left femoral artery access hole 537 in the direction shown by the arrow 544 in FIG. 37, the release strand 629 feeds out of the well 633 in an orderly and linear fashion in a direction from the release strand distal end 637 to its proximal end 634.

This is made possible by the forces created at the "u-turn" or juncture 641 by the physical interface with the friction line 642. The friction force (which can be tailored by the proper combination af release strand 629 and friction line 642 diameters and their materials and by properly dimensioning of the well 633, for example) provides enough resistance to counter the force applied by the operator so that the "u-tnrn" or juncture 641 moves in an orderly fashion in a direction from the well bottom 633 to the distal end 646 of the inner tubular member 628 until it exits out of the outer tubular member 628. At this point, any remaining friction line 642 at the juncture 641 is superfluous as it has served its purpose of facilitating an orderly withdrawal of the release strand 629. The operator continues to pull on the second guidewire 638 as previously described so that the release strand 629 extends through the left femoral artery access port 537. We have found the example of FIG. 53 to be useful in achieving an orderly and tangie-free deployment.

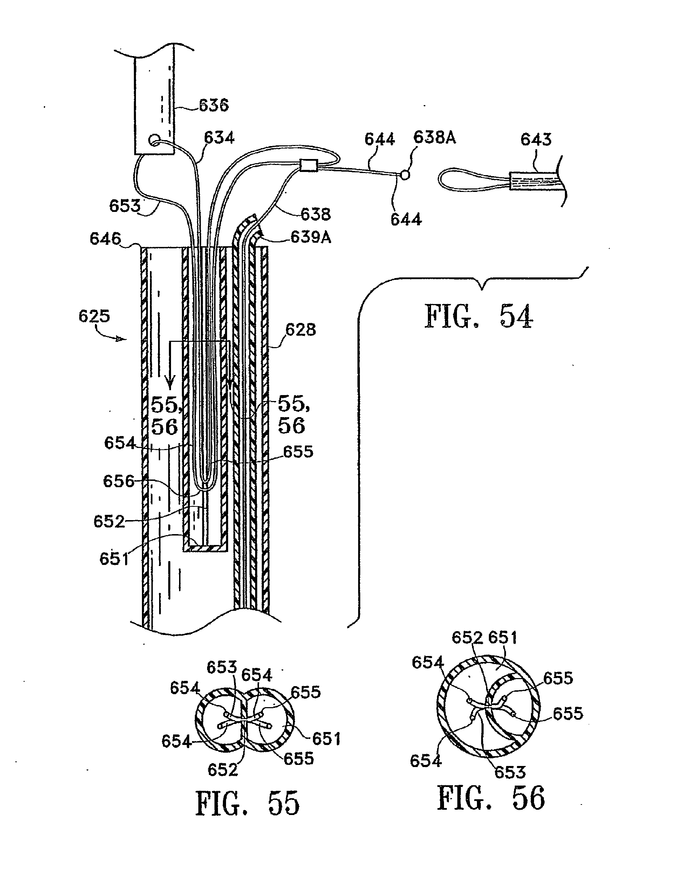

Alternatively, any number of other arrangements in which the release strand 629 may be fed out of the outer tubular member 628 in an orderly manner is disclosed herein. For instance, the well 651 shown in FIGS. 54-56 is, for instance, an extruded polymeric part having a unique cross-sectional configuration that eliminates the need for the friction line 642 in the embodiment shown in FIG. 53. Here, a narrowing constraint or gap 652 runs the length of the well interior 651, forming a physical barrier between first and second opposing portions 654 and 655 of the release strand 653, shown in FIGS. 54-56. The constraint or gap 652 is sized to allow the passage therethrough of the release strand juncture or "u-turn" 656. As the operator pulls the release strand 653 out of the well 651, the constraint or gap 652 prevents the opposing portions 654 and 655 of the release strand 653 from crossing into the other side of the well 651. Said another way, the constraint or gap 652 keeps the juncture or "u-turn" 656 within its vicinity to facilitate an orderly withdrawal of the release strand 653 from the well 651. In this example, the release strand 653 can have a diameter of between about 0,001 cm and 0,025 cm (0.004 and 0.010 inch); specifically between about 0,015 cm and 0,017 cm (0.006 and 0.007 inch). The gap or constraint 652 should be between about 0,007 cm and about 0,023 cm (0.003 and about 0.009 inch); preferably between about 0,013 cm and 0,015 cm (0.005 and about 0.006 inch).

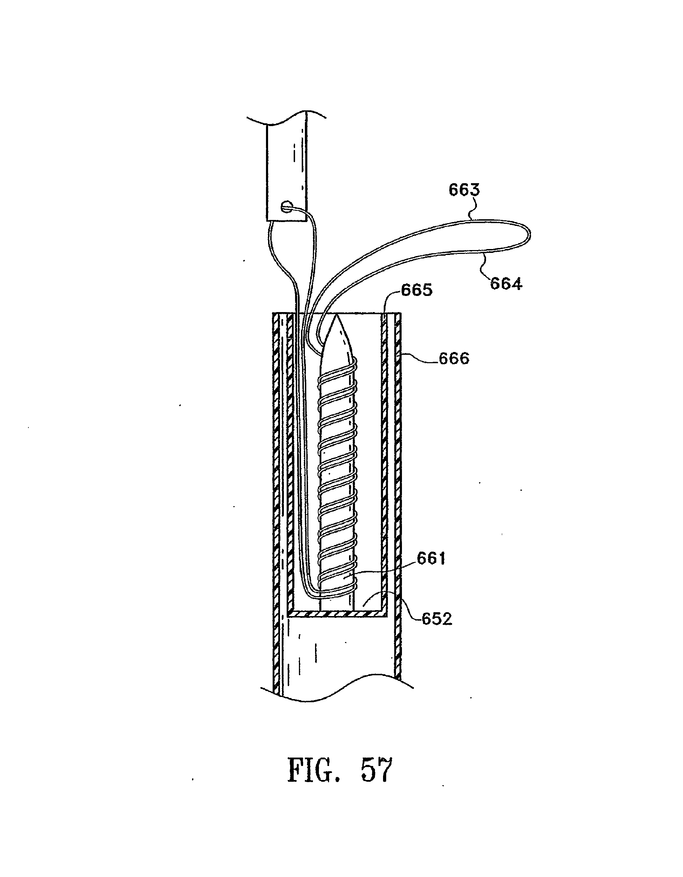

Yet another variation of this embodiment, shown in FIG. 57, includes a post 661 disposed in a well 652 around which the release strand 663 is wound such that as the operator pulls the distal portion 664 of the release strand 663 out of the distal end 665 of the well 652, the release strand 663 unwinds in an orderly fashion from the post 661. The post 661 may be optionally configured to spin on its longitudinal axis, similar to that of a fishing reel spinner, to facilitate the exit of the release strand 663.

Other variations, such as a block and tackle arrangement (not shown), are envisioned in which the release strand 663 is looped through a grommet or similar feature. The grommet provides the necessary friction to prevent the entire release strand 663 from pulling out of the well 652 in one mass as soon as the operator applies a force on a distal end thereof. Any arrangement in which a frictional or similar force is utilized to allow for the orderly dispensation of the release strand 663 from the shaft or post 661 is disclosed herein.

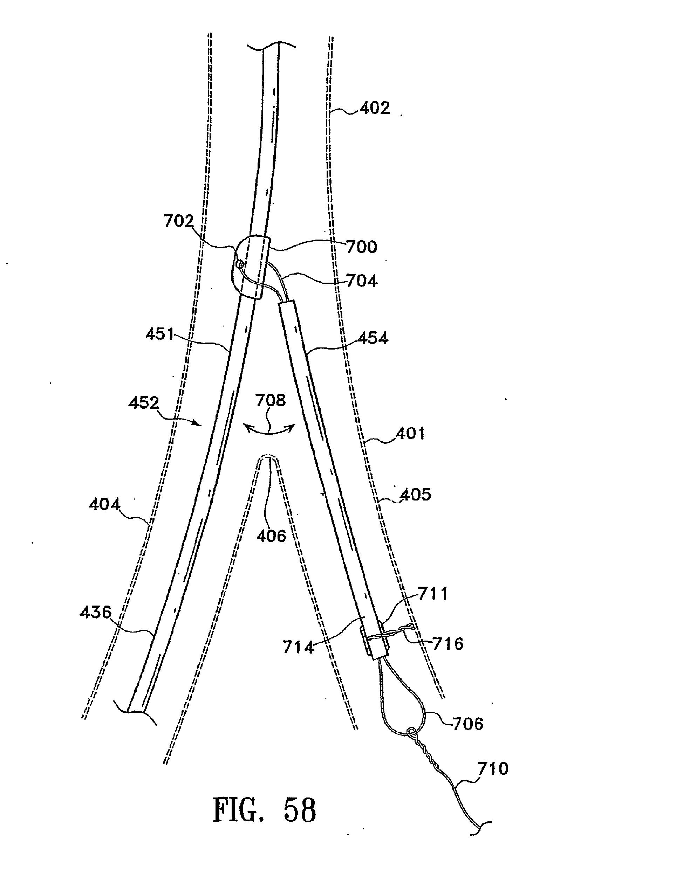

FIG. 58 depicts an optional hinged design for the belt support members that is particularly useful for deploying the bifurcated stent-graft in tortuous and/or angled anatomies, although it may be used in all anatomies. Bifurcated graft 401 is depicted in phantom for reference. A hinge body 700 is affixed to guide wire tube 436 or primary belt support member 452. Aperture 702 disposed on one side of primary belt support member 452 is configured to receive hinge attachment member 704, which in this example is a wire that is looped through aperture 702 and fixed to secondary belt support member 454. The hinge created at aperture 702 allows support member 454 to swing away from and towards primary belt support member 452 in the direction indicated by arrows 708 in FIG. 58.

As shown in FIG. 58, aperture 702 is disposed on the side of primary belt support member 452 opposite that on which secondary belt support member 454 resides to facilitate extraction of the belt support members from the graft and the patient's body after graft deployment. However, aperture 702 may also be disposed on the same side of primary belt support member 452 as that of secondary belt support member 454 or in any suitable orientation around member 452.

Release strand 710 is affixed to release strand attachment member 706 at secondary belt support member proximal end 714 and is preferably a stainless steel wire having a diameter of between about 0,01 cm and 0,025 cm (0.004 inch and 0.010 inch), although other materials and diameters may be used. Secondary belt 716 is shown disposed on support member 454 along with optional silicone tubing 711.

Chiefly in tortuous or angled anatomies, but also in straighter vessels, it is useful to allow for a degree of slack in the contralateral limb 405 to be loaded into the elongate shaft 423. Such slack helps the contralateral leg 405 negotiate various bends in the iliac and/or femoral arteries. The total amount of slack ΔI ideally necessary for a graft limb such as limb 405 to negotiate an angle ΔΘ is represented by the equation:

$$\Delta I = d \Delta \dot{\Theta}$$

where "ΔΘ" is the cumulative angle change (the sum of the absolute value of the angles through which the limb must negotiate) along its length, measured in radians, and where "d" is the diameter of the graft limb.

The hinge design of FIG. 58 allows the necessary amount of slack ∆I to be maintained in the contralateral leg 405 both during the step of loading graft 401 in shaft 423 and during graft deployment and placement. Note that in an example, a predetermined amount of slack may also be built into the ipsilateral leg 404 as it is assembled for delivery. By building a predetermined amount of slack in each of the legs of graft 401, the most prevalent patient anatomies may, for instance, be targeted so that the average graft delivery procedure will require the smallest amount of leg adjustment or manipulation by the operator.

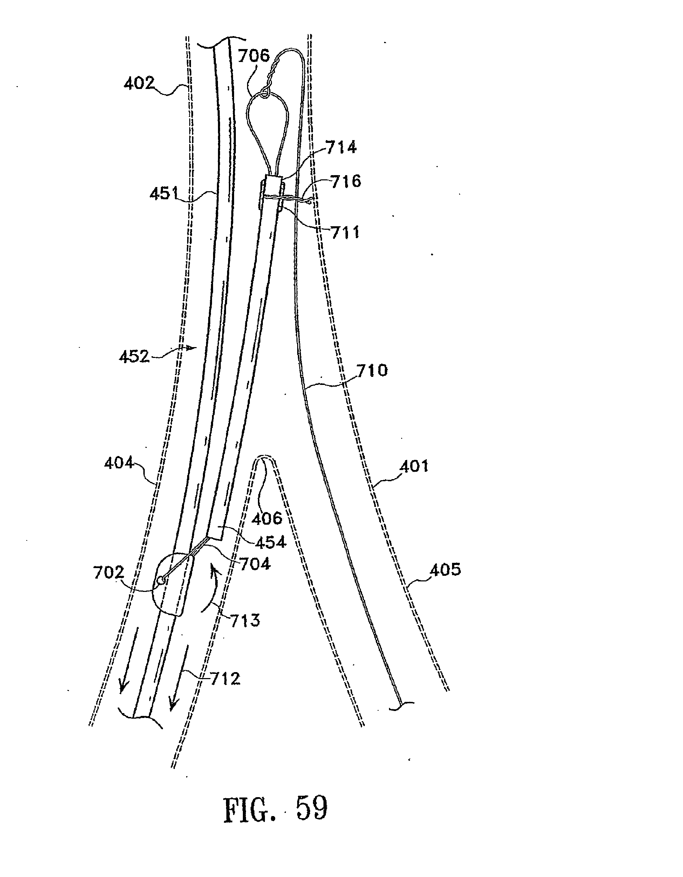

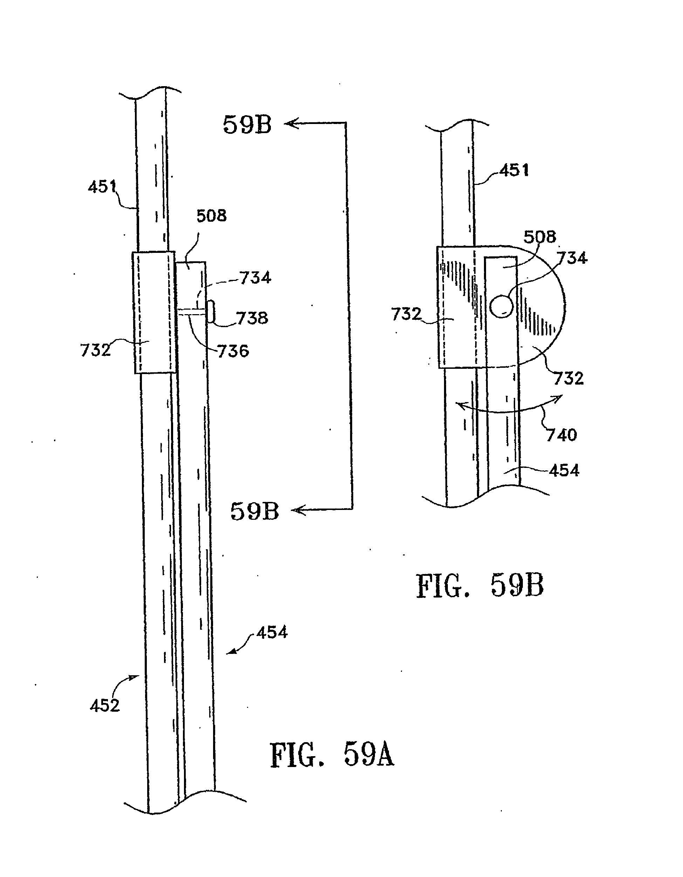

After graft 401 has been deployed, the apparatus of FIG. 58 is next withdrawn from the graft and the patient's vasculature in the direction of arrows 712 as shown in FIG. 59 over guide wire 530. During this withdrawal, secondary belt support member 454 rotates about aperture 702 and pivots towards primary belt support member 452 in the direction of arrow 713. An optional buttress may be employed as described later to facilitate the withdrawal process.

Both primary and secondary belt support members are ideally radiopaque to facilitate withdrawal from the vasculature. Secondary belt support member 454 and hinge attachment member 704 should be flexible enough to turn the corner around graft bifurcation 406 with little or no permanent deformation as the operator withdraws the primary belt support member 452 in the direction of arrows 712.

Withdrawal of member 452 causes secondary belt support member 454 to first retreat from contralateral limb 405 until the proximal end 714 of secondary belt support member 454 clears the graft walls in the vicinity of bifurcation 406, allowing the hinge to further act to align secondary belt support member in a generally parallel relationship with primary belt support member 452 as both are then withdrawn through the ipsilateral leg 404 and eventually out of the patient's body through right femoral access hole 531. Release strand 710 follows secondary belt support member 454 out of the body.

FIGS. 59A-B depict a variation of this hinge design that limits rotation of the secondary belt support member 454 to a single plane. Here, hinge body 732 is fixedly disposed on a distal portion 451 of primary belt support member 452 and comprises an offset flanged pin 734 or like element. Pin 734 is disposed in an aperture 736 that runs through the distal end 508 of secondary belt support member 454 and hinge body 732. In this configuration, secondary belt support member 454 is rotatably secured to pin 734 by optional flange 738 and is free to rotate about pin 734 in the direction indicated by arrows 740 to facilitate withdrawal of the delivery apparatus from the patient. The optional offset feature of pin 734 assists in the extraction of the belt support members from the graft and the Patient's body after graft deployment.

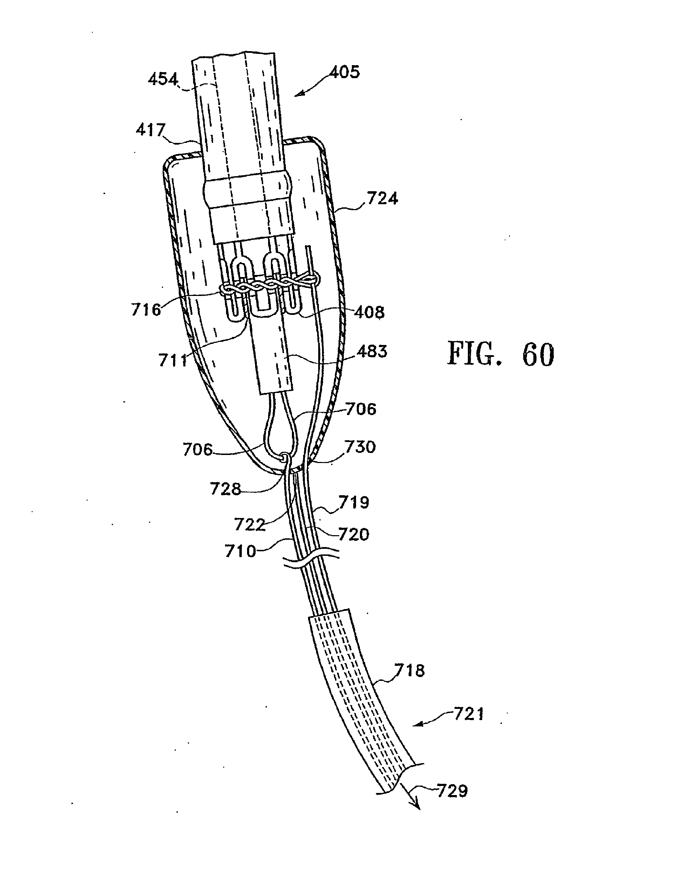

FIG. 60 shows a close up partial cross-sectional view of the proximal end 417 of graft contralateral leg 405 disposed on the FIGS. 58-59 (or alternatively FIG. 59A-B) secondary belt support member 454. Release strand tube 718, part of secondary release cable 721, houses release strand 710, a secondary release wire 719 (which holds secondary belt 716 around contralateral proximal self expanding member 408), and a shield line 720 that is fixedly attached at its distal end 722 to optional contralateral self-expanding member shield 724.

Optional expanding member shield 724 comprises PET or similar polymeric material. Shield 724 acts as a shroud to cover proximal self-expanding member 408, protecting ipsilateral leg 404 from being damaged by self-expanding member 408 during delivery system assembly and graft deployment. Further, shield 724 prevents direct contact between contralateral self-expanding member 408 and ipsilateral self-expanding member 407, keeping the various self expanding member components from snaring one another or otherwise getting entangled. The exact position of graft contralateral proximal self-expanding member 408 relative to graft ipsilateral leg 404 and self-expanding member 407 will depend on several factors, one of which is the degree of slack built into the graft legs 404, 405 on members 452 and 454.

Shield 724 may be removed prior to retraction of secondary release wire 719 by retracting shield line 720 in the direction indicated by arrow 729, typically after release strand tube 718 has been removed, and ultimately out of the patient's body through left femoral artery access hole 537. As shield 724 is retracted, release strand 710 and secondary release wire 719 pass through wire apertures 728 and 730, respectively. Alternatively, a single wire aperture may be disposed on shield 724 through which both release strand 710 and secondary release wire 719 pass.

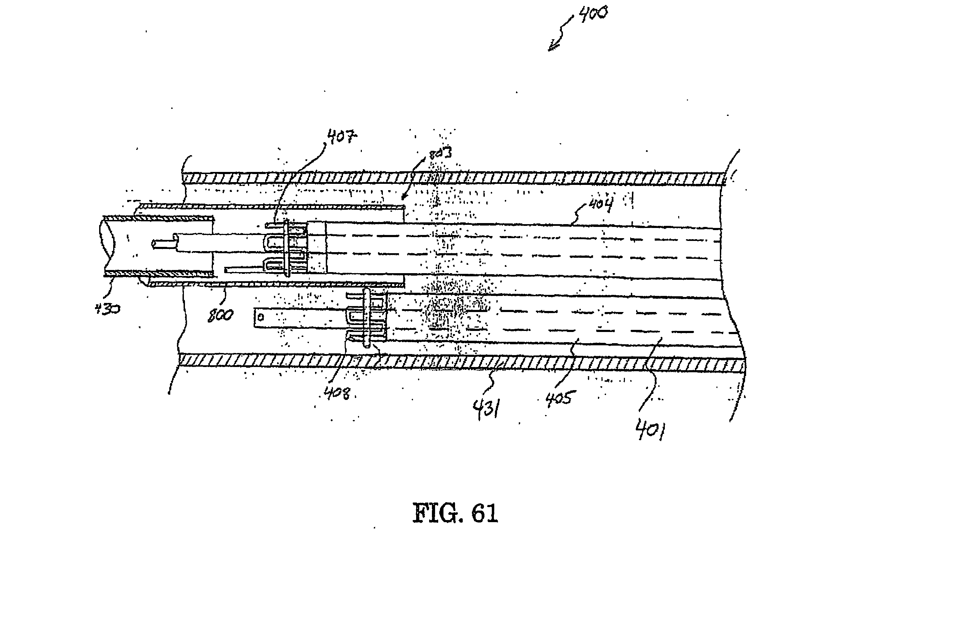

FIGS. 61-63B schematically depict an optional ipsilateral leg sleeve 800, an alternative component and method for achieving the purposes served by shield 724. Ipsilateral leg sleeve 800 may also protect ipsilateral leg graft material from being damaged by other graft components such as, e.g., a contralateral leg distal connector member disclosed in U.S. Patent Application Serial No. 10/327,711 to Chobotov et al, filed December 20, 2002 entitled "Advanced Endovascular Graft" . Sleeve 800 also may, in certain delivery system and graft configurations, further radially compress ipsilateral leg 404 and ipsilateral proximal self-expanding member 407 and any additional device components, helping to relieve ipsilateral leg 404 from radial forces exerted by contralateral leg self-expanding member 408 while bifurcated stent-graft 401 is disposed in the delivery system prior to deployment.

In this configuration of delivery system 400 and as shown generally in FIG. 61, at least a portion of graft 401 ipsilateral leg 404 is covered by an optional protective ipsilateral leg sleeve 800. Ipsilateral leg sleeve 800 may take on a wide variety of configurations. For instance, leg sleeve 800 may be a single covering such as a tube or catheter, a corset or wrapping of biologically compatible fabric or polymeric material, or it may be a composite structure of two or more components such as multiple tubes configured partially or fully coaxially with each other, in an abutting end-to-end relationship, etc. To this end, the example discussed below in conjunction with FIGS. 61-63B is but one of many examples of ipsilateral leg sleeve 800.

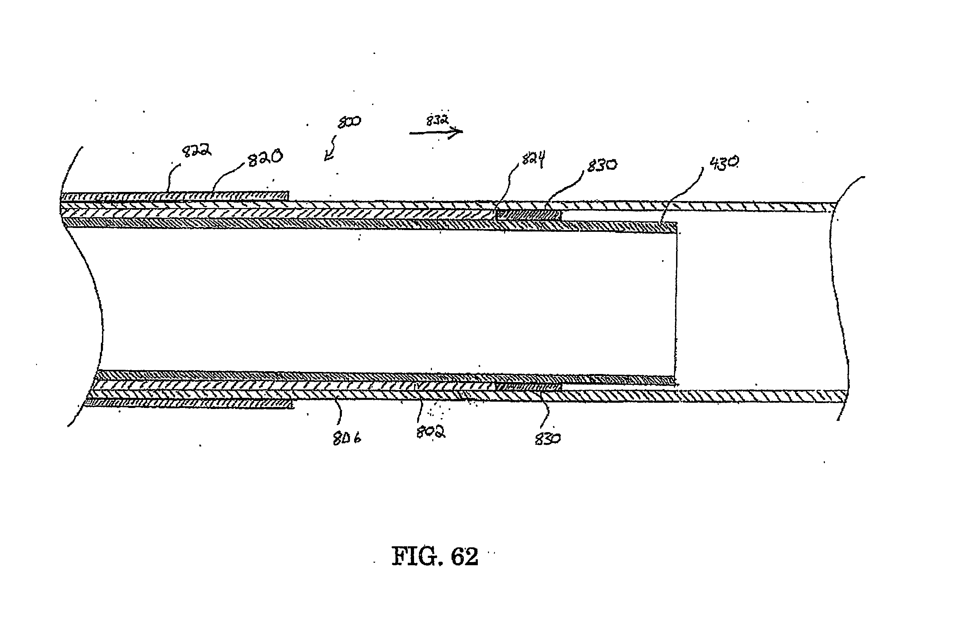

One variation of sleeve 800 comprises three coaxially arranged tubes 802,806, 820 as shown in FIG. 62. Here, ipsilateral leg sleeve 800 comprises an inner tube 802 covering at least a portion of the shaft inner tubular member 430 and a more compliant, coaxial protective tube 806 that may be slip fit or heat shrunk over the outer surface of inner tube 802 for covering the graft ipsilateral leg 404.

In this example, inner tube 802 may comprise PEEK or any comparable material, and ideally is relatively rigid so to impart column stiffness to sleeve 800 and to prevent sleeve 800 from buckling or bunching during system assembly and graft deployment. Although other configurations are possible, inner tube 802 may extend the length of leg sleeve 800 up to a distal end 824 so that only protective tube 806 covers graft ipsilateral leg 404.

Protective tube 806 may be made of FEP, HDPE, or any useful fluoropolymer. Tube 806 is ideally slip fit over the outer surface of inner tube 802. When protective tube 806 comprises FEP, for instance, it may be heat shrunk over inner tube 802 using known techniques.

Ipsilateral leg sleeve 800 extends distally over ipsilateral leg 404 such that when disposed adjacent contralateral leg 405, sleeve distal end is preferably distal to the distalmost extent of the graft contralateral leg 404 self-expanding member 408, including adjacent and distal to any additional components (such as, e.g., a contralateral leg distal connector member disclosed in U.S. Patent Application Serial No. 10/327,711).

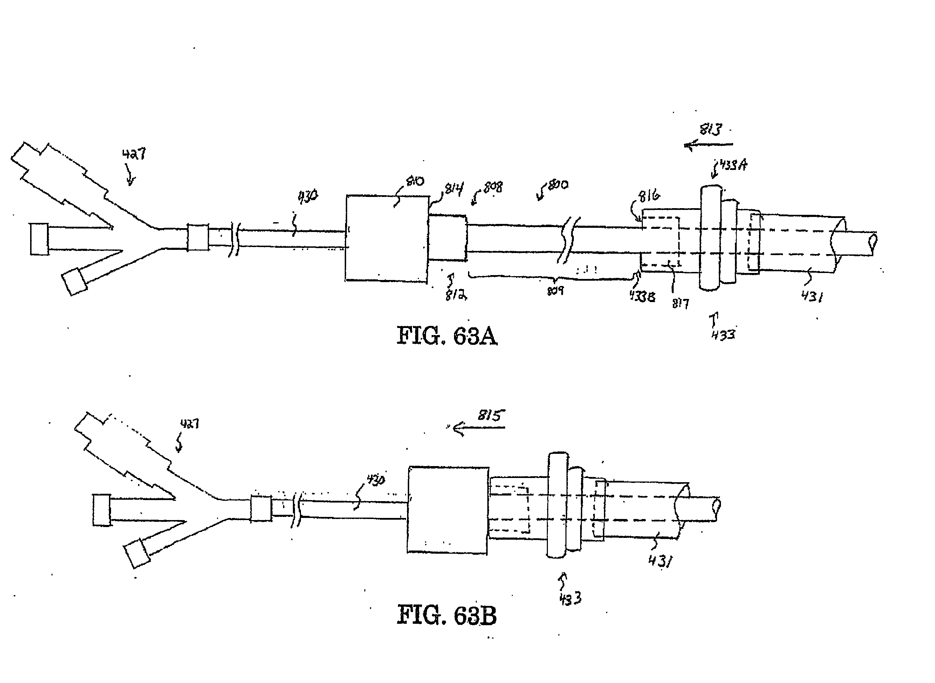

Ipsilateral leg sleeve 800 extends proximally beyond the proximal end 416 of ipsilateral leg 404 and is coaxially disposed over the outer surface of shaft inner tubular member 430 as shown in the simplified schematic depiction of FIGS. 61-62. Sleeve 800 preferably terminates at a sleeve proximal region 808 in a fitting such as nylon fitting 810 shown in FIGS. 63A-63B. The overall length of ipsilateral leg sleeve 800 generally will range from between about 40 cm to about 90 cm; more preferably between about 50 cm and about 75 cm.

As shown in FIGS. 63A and 63B, when included in delivery system 400, optional sleeve 800 may extend proximally beyond the proximal end 433 of outer tubular member 431, including any fittings such as shaft valve 43 3A, and preferably far enough proximally to provide a gripping region 809 for the physician as described below.

During the deployment of graft 401 in which outer tubular member 431 is proximally retracted, the physician typically will retract outer tubular member 431 with one hand and grip the portion of sleeve 800 in region 809 with the other hand. In such a case it is desirable to maximize the potential that the ipsilateral leg sleeve 800 does not slip in the physician's hand during the outer tubular member 431 retraction maneuver. To facilitate such slip-free gripping, the outer surface of protective tube 806 in this region 809 may be chemically etched to give the protective tube outer surface a relatively rough profile. Alternatively or in addition, protective tube 806 may comprise a material such as PET or the like that tends to be less "slippery" than other appropriate biological-grade polymers. Another option, as shown in FIG. 62, is to include a third tube 820 having an outer surface 822. Third tube 820 may comprise PET or like material as part of ipsilateral leg sleeve 800 over the outer surface protective tube 806. Third tube 820 may be heat shrunk or otherwise coaxially disposed over protective tube 806.

When gripping the ipsilateral sleeve leg 800 with one hand and simultaneously proximally moving outer tubular member 431 relative to inner tubular member 430 as described herein, the physician may also have an undesirable tendency to push sleeve 800 in a distal direction, risking damage to graft 401. To prevent sleeve 800 from extending distally beyond a given point on ipsilateral leg 404 in case sleeve 800 is pushed distally, a stop or shoulder may be affixed to the outer surface of inner tubular member 430 as shown in FIG. 62. If the sleeve 800 is inadvertently moved in the distal direction shown by arrow 832, stop 830 will abut the distal end 824 of inner tube 802 and prevent further distal motion of sleeve 800. Alternatively, sleeve 800 may be equipped with a tether (not shown) or the like attaching the proximal end of sleeve 800 to the proximal adapter of the delivery system.

In the example described herein and as shown in FIGS. 63A and 63B, the proximal region 808 of sleeve 800 comprises a fitting 810 whose distal end 812 is designed to abut and/or coaxially mate with a recess 817 in the proximal end 433 of shaft outer tubular member 431 or like fitting, such as valve 433A.

During the deployment of graft 401, the physician proximally retracts outer tubular member 431 by pulling on the proximal end 433 of the outer tubular member 431 relative to the ipsilateral leg sleeve 800 (and therefore also relative to the inner tubular member 430) and over the outer surface of sleeve 800 as shown by arrow 813 in FIG. 63A. In the example of FIGS. 61-63B, concurrently with or after the distal end 435 of outer tubular member 431 has proximally cleared the contralateral leg 405 as desired by the physician, valve 433A makes contact with sleeve fitting 810. In particular, a recess 817 formed in the proximal end of valve 433A encloses the distal end 812 of fitting 810 and stepped surface 814 of leg sleeve fitting 810 abuts the proximal surface 816 of valve 433A. As the physician continues to proximally retract outer tubular member 431, fitting 810 and sleeve 800 now proximally retract together with outer tubular member 431 and valve 433A as shown by arrow 815 in FIG. 63B until the sleeve 800 is removed from ipsilateral leg 404. At this point, deployment of graft 401 continues as described elsewhere herein.

The particular details described herein are but one way of accomplishing the objectives set forth for ipsilateral leg sleeve 800. A number of variations of this technique, including using different fitting configurations and different sleeve and tubular member retraction sequences are also possible. For instance, ipsilateral leg sleeve 800 may be configured for separate proximal retraction to expose ipsilateral leg 404 before, during or after retraction of outer tubular member 431.

A variation in the deployment sequence that may be used with any of the sequences and equipment described above may be appropriate in certain clinical settings when the patient's vasculature exhibits a degree of tortuosity and/or angulation.

Related to the cuff and channel lumen patency matter discussed above are at least two additional considerations when deploying a device such as bifurcated graft 401 in tortuous or angled anatomies. First, it can be more challenging to maintain the patency of either or both the blood flow passageways formed by the walls of graft contralateral leg 405 and/or ipsilateral leg 404. Such challenges may also be presented in the blood flow passageways defined by graft main body 402 of the bifurcated graft 401 and tubular graft 11 embodiments. This may in turn negatively affect the patency of the cuff and channel lumens such that the cuffs and channels cannot adequately be filled with inflation material. Second, the outer tubular member 431 can be more difficult to retract proximally relative to inner tubular member 430 when the delivery system 400 is disposed in such angled and/or tortuous anatomies.

The delivery method discussed with respect to FIGS. 34-50 teaches that the steps of deploying the distal and proximal self-expanding members are accomplished prior to the step of inflating the graft cuffs and channels. A variation in this deployment sequence that is useful for tortuous or angled patient anatomies is discussed below in conjunction with the delivery system components of FIGS. 31A, 31B and 58-60, although any of the delivery systems or their components described herein may employ this sequence variation.

During the delivery procedure, after the first and second distal self expanding members 411 and 412 have been released, the operator removes release strand tube 718 from the body through the left femoral access hole 537. This exposes release strand 710, secondary release wire 719, and shield line 720.

Next, the shield line 720 is pulled in a proximal direction 729 by the operator to remove shield 724 from the contralateral leg proximal end 417, exposing self-expanding member 408. A buttress, which can be a tubular member such as a catheter or the like, is threaded on the remaining secondary release wire 719 and release strand 710 and advanced distally until it physically abuts the proximal end 483 of the secondary belt support member 454. This provides a relatively stiff column that the operator may use to move the graft contralateral leg 405 in a distal direction as well as react the force necessary to deploy self-expanding member 408 by retracting release wire 719.

The operator next detaches Luer-type fitting or cap 521 from flexible fill catheter 523 and removes ripcord 510 from channel 418. Graft 401 cuffs and channels may then be filled with inflation material as previously described. When the inflation material is radiopaque or otherwise observable in *vivo,* the operator may interrogate the shape of the graft 401 and the various cuffs and channels under fluoroscopy or other suitable imaging technique to determine graft limb patency, the sufficiency of graft cuff and channel inflation, and whether any folds or other irregularities in the graft exist so that they may be corrected. When observed under fluoroscopy, the operator may adjust the C-arm of the fluoroscope to interrogate graft 401 from a number of angles.

If necessary, and after cuff and channel inflation but before proximal self-expanding member deployment, the operator may manipulate both the buttress catheter and/or release strand 710 to push or pull, respectively, the graft contralateral leg into. the proper position. By making fine adjustments in either direction, the operator may remove or add slack in the graft contralateral leg 405 and ensure optimal graft placement and patency. To minimize operator confusion, the release strand 710 and stent release wire 719 maybe different lengths, color coded, flagged or otherwise labeled, etc. We have found that making the stent release wire 719 shorter than release strand 710 helps in maintaining optimal operator orientation with respect to the various components of the graft delivery system.

When the operator is satisfied with the position, patency, and appearance of graft 401, contralateral self-expanding member 408 may be deployed by applying tension in the proximal direction 729 on secondary release wire 719 so that secondary belt 716 releases proximal self-expanding member 408 in the manner previously described.

Similarly, the operator next may adjust the position of the ipsilateral leg 404 of graft 401 by adjusting the position of primary belt support member 452 and then release proximal self-expanding member 407 of the ipsilateral leg 404 as described herein.

To withdraw the delivery apparatus, guide wire 530 is partially withdrawn in the proximal direction through nosepiece 434 into guide wire tube 436 to a point proximal of cuff 413. This prevents the guide wire 530 from possible interference with proper inflation of cuff 413. Next, the distal end 487 of the inflation tube 444 may be disengaged from the inflation port 421 by pulling on a proximal end 491 of retention wire 488 as previously discussed. Using the buttress to push on belt support member proximal portion 483 if necessary, the operator may then proximally withdraw the primary belt support member 452 over guide wire 530 with the secondary belt support member 454 following. Finally, guide wire 530 is removed through left and right femoral access holes 537, 531, which may then be repaired using conventional techniques.

It is clear to those of skill in the art that although particular techniques and steps are described herein that we have found to be useful, variations in the order and techniques in which the various deployment steps described herein are within the scope of the present invention.

While particular forms of the invention have been illustrated and described, it will be apparent that various modifications can be made without departing from the scope of the invention. Accordingly, it is not intended that the invention be so limited.

**Claims**

1. An endovascular graft delivery system (300) configured to deliver an endovascular graft (11) to a target location within a patient's vasculature and deploy the endovascular graft (11) at the target location, comprising:

   an endovascular graft (11) including a graft body section and a distal self-expanding member (32) which is disposed distally of the graft body section;
   an elongate shaft (12) having a proximal section and a distal section (14), the distal section (14) including a portion having the endovascular graft (11);
   a first handle (335) disposed on a proximal portion (15) of the delivery system (300) connected to a first belt (22) via a first release member (312), wherein the first release member releasably secures the first belt in a constrained configuration, and wherein the first belt constrains the distal self expanding member, such that the radial constraint on the distal self-expanding member (32) of the endovascular graft (11) is partially released upon actuation of the first handle (335), and
   a second handle (331) disposed on the proximal portion (15) of the delivery system (300) connected to a second belt (21) axially spaced from the first belt via a second release member (316), wherein the second release member releasably secures the second belt in a constrained configuration, and wherein the second belt constrains the distal self expanding member, such that the distal self-expanding member (32) of the endovascular graft (11) is deployed upon actuation of the second handle (331),
   wherein the self-expanding member (32) of the endovascular graft (11) comprises one or more barbs configured to engage tissue of the patient's vasculature in a deployed state to prevent axial migration of the graft (11).

2. (currently amended) The endovascular graft delivery system of claim 1, further comprising an outer tubular member (53) disposed over the endovascular graft (11) and configured to cover the endovascular graft (11) in a constrained state during delivery to a target location within the patient's vasculature and configured to be retracted proximally to expose the endovascular graft (11).

3. The endovascular graft delivery system of claim 2 wherein the elongate shaft (12) further comprises a guidewire lumen (17).

4. The endovasculpr graft delivery system of claim 2 or 3 further comprising a radiopaque marker imageable by fluoroscopy.

5. The endovascular graft delivery system of any one of claims 2 to 4 wherein the one or more barbs of the distal self-expanding member comprise barbs which are integrally formed with respective struts of the distal self-expanding member (32).

**Patentansprüche**

1. Endovaskuläres Implantat-Zuführungssystem (300), welches ausgebildet ist, ein endovaskuläres Implantat (11) einer Zielposition in den Blutgefäßen eines Patienten zuzuführen und das endovaskuläre Implantat (11) an der Zielposition bereitzustellen, umfassend:

   ein endovaskuläres Implantat (11), welches einen Implantatkörperabschnitt und ein distales selbst-expandierendes Element (32), welches distal gegenüber dem Implantatkörperabschnitt angeordnet ist, umfasst;
   einen länglichen Schaft (12), welcher einen proximalen Abschnitt und einen distalen Abschnitt (14) aufweist, wobei der distale Abschnitt (14) einen Teilbereich umfasst, welcher das endovaskuläre Implantat (11) aufweist;
   einen ersten Griff (335), welcher an einem proximalen Teilbereich (15) des Zuführungssystems (300) angeordnet ist und mit einem ersten Band (22) über ein erstes Freigabeelement (312) verbunden ist, wobei das erste Freigabeelement das erste Band lösbar in einer Zwangskonfiguration festhält, und wobei das erste Band das distale selbst-expandierende Element zwingt, sodass der radiale Zwang auf das distale selbst-expandierende Element (32) des endovaskulären Implantats (11) auf eine Betätigung des ersten Griffs (335) teilweise gelöst wird, und einen zweiten Griff (331), welcher an dem proximalen Teilbereich (15) des Zuführungssystems (300) angeordnet ist und mit einem zweiten Band (21) axial beabstandet von dem ersten Band über ein zweites Freigabeelement (316) verbunden ist, wobei das zweite Freigabeelement lösbar das zweite Band in einer Zwangskonfiguration festhält, und wobei das zweite Band das distale selbst-expandierende Element zwingt, sodass das distale selbst-expandierende Element (32) des endovaskulären Implantats (11) auf eine Betätigung des zweiten Griffs (331) bereitgestellt wird,

wobei das selbst-expandierende Element (32) des endovaskulären Implantats (11) eine oder mehrere Widerhaken umfasst, welche ausgebildet sind mit Gewebe der Blutgefäße des Patienten in einem bereitgestellten Zustand in Eingriff zu sein, um axiale Migration des Implantats (11) zu vermeiden.

2. Endovaskuläres Implantat-Zuführungssystem nach Anspruch 1, ferner umfassend ein äußeres röhrenförmiges Element (53) welches über dem endovaskulären Implantat (11) angeordnet ist und ausgebildet ist das endovaskuläre Implantat (11) in einem gezwungenen Zustand während der Zuführung zu der Zielposition in den Blutgefäßen des Patienten zu bedecken und ausgebildet ist proximal zurückgezogen zu werden, um das endovaskuläre Implantat (11) freizulegen.

3. Endovaskuläres Implantat-Zuführungssystem nach Anspruch 2, wobei der längliche Schaft (12) ferner ein Führungsdrahtlumen (17) umfasst.

4. Endovaskuläres Implantat-Zuführungssystem nach Anspruch 2 oder 3, ferner umfassend einen röntgenstrahlenundurchsichtigen Marker, welcher durch Fluoroskopie abbildbar ist.

5. Endovaskuläres Implantat-Zuführungssystem nach einem der Ansprüche 2 bis 4, wobei die einen oder mehreren Widerhaken des distalen selbst-expandierenden Elements Widerhaken umfassen, welche integral mit den entsprechenden Streben des distalen selbst-expandierenden Elements (32) ausgebildet sind.

**Revendications**

1. Système de pose de greffon endovasculaire (300) configuré pour poser un greffon endovasculaire (11) sur un emplacement cible dans le système vasculaire d'un patient et déployer le greffon endovasculaire (11) à l'emplacement cible, comprenant:

un greffon endovasculaire (11) comprenant une section de corps de greffon et au moins un élément auto-expansible distal (32) qui est disposé de manière distale par rapport à la section de corps de greffon;

une tige allongée (12) ayant une section proximale et une section distale (14), la section distale (14) comprenant une partie ayant le greffon endovasculaire (11);

une première poignée (335) disposée sur une partie proximale (15) du système de pose (300) raccordée à une première courroie (22) via un premier élément de libération (312), dans lequel le premier élément de libération fixe de manière amovible la première courroie dans une configuration contrainte, et dans lequel la première courroie contraint l'élément auto-expansible distal, de sorte que la contrainte radiale sur l'élément auto-expansible distal (32) du greffon endovasculaire (11) est partiellement libérée suite à l'actionnement de la première poignée (335), et

une seconde poignée (331) disposée sur la partie proximale (15) du système de pose (300) raccordée à une seconde courroie (21) axialement espacée de la première courroie (316), via un second élément de libération, dans lequel le second élément de libération fixe de manière amovible la seconde courroie dans une configuration contrainte, et dans lequel la seconde courroie contraint l'élément auto-expansible distal, de sorte que l'élément auto-expansible distal (32) du greffon endovasculaire (11) est déployé suite à l'actionnement de la seconde poignée (331), dans lequel l'élément auto-expansible (32) du greffon endovasculaire (11) comprend un ou plusieurs ardillons configurés pour mettre en prise le système vasculaire du patient dans un état déployé afin d'empêcher la migration axiale du greffon (11).

2. Système de pose de greffon endovasculaire selon la revendication 1, comprenant en outre:

un élément tubulaire externe (53) disposé sur le greffon endovasculaire (11) et configuré pour recouvrir le greffon endovasculaire (11) dans un état contraint pendant la pose sur un emplacement cible à l'intérieur du système vasculaire du patient et configuré pour être rétracté de manière proximale afin d'exposer le greffon endovasculaire (11).

3. Système de pose de greffon endovasculaire selon la revendication 2, dans lequel la tige allongée (12) comprend en outre une lumière de fil-guide (17).

4. Système de pose de greffon endovasculaire selon la revendication 2 ou 3, comprenant en outre un marqueur radio-opaque pouvant être imagé par radioscopie.

5. Système de pose de greffon endovasculaire selon l'une quelconque des revendications 2 à 4, dans lequel les un ou plusieurs ardillons de l'élément auto-expansible distal comprennent des ardillons qui sont formés de manière solidaire avec des entretoises respectives de l'élément auto-expansible distal (32).

FIG. 1

FIG. 3

FIG. 5

FIG. 2

FIG. 4

FIG. 6A

FIG. 6B

EP 2 145 607 B1

FIG. 7B

FIG. 7A

EP 2 145 607 B1

FIG. 7E

FIG. 7D

FIG. 7C

FIG. 7F

EP 2 145 607 B1

FIG. 7G

FIG. 7H

FIG. 7I

FIG. 7J

FIG. 7K

FIG. 7L

EP 2 145 607 B1

FIG. 7N

FIG. 7M

FIG. 8

FIG. 9

FIG. 10

FIG. 11

FIG. 12

FIG. 13

FIG. 14

FIG. 15

FIG. 16

FIG. 17

FIG. 18

EP 2 145 607 B1

FIG. 19

FIG. 20

FIG. 21

FIG. 22

FIG. 23

FIG. 24

FIG. 25

FIG. 26

Fig. 29

Fig. 28

Fig. 27

Fig. 28A

Fig. 30

FIG. 28B

FIG. 31

EP 2 145 607 B1

FIG. 31A

EP 2 145 607 B1

FIG. 31B

FIG. 32

FIG. 33

FIG. 34

FIG. 35

**FIG. 36**

FIG. 37

434

553

552

425

551

FIG. 37A

554A

554

556

FIG. 37B

556

557

555

FIG. 38

FIG. 39

FIG. 40

FIG. 41

FIG. 41A

FIG. 42

FIG. 43

FIG. 44

FIG. 44A

EP 2 145 607 B1

FIG. 45

FIG. 46

FIG. 47

405
587
464 476
408 475
483
482
542
477
478
485
438
561 591
481
562
591

FIG. 48

FIG. 49

FIG. 50

FIG. 51

FIG. 51A

FIG. 52

FIG. 52A

FIG. 53

636

634

644    638A    643

653

638

646    639A

625    FIG. 54

628

55,
56

55,
56

654    655

656

652

651

653 654
654    655

654    651
652    655

FIG. 55

652 651
654    655

655

654    653

FIG. 56

FIG. 57

FIG. 58

FIG. 59

59B ←

451

508

734

738

732    736

59B ←

59B ←

451

508    734

732

732

740

454

FIG. 59B

454

452

FIG. 59A

FIG. 60

FIG. 61

FIG. 62

FIG. 63A

FIG. 63B

# REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4580568 A
- US 13397898 A, M. Chobotov
- US 09133978 B
- US 327711 A
- US 32771102 A, Chobotov

**Non-patent literature cited in the description**

- **LAWRENCE, JR. et al.** Percutaneous endovascular graft: experimental evaluation. *Radiology,* May 1987
- **MIRICH et al.** Percutaneously placed endovascular grafts for aortic aneurysms: feasibility study. *Radiology,* March 1989